# EUROPEAN PATENT APPLICATION

(11) **EP 2 036 889 A2**
(43) Date of publication of application: **18.03.2009**
(21) Application number: 07747937.6
(22) Date of filing: 17.05.2007
(51) Int. Cl.: C07D 209/42, C07D 401/04, C07D 401/06, C07D 401/14, C07D 403/06, C07D 403/04, C07D 405/04, C07D 409/04, C07D 413/14, C07D 498/04, A61K 31/405, A61K 31/407, A61K 31/416, A61K 31/4439, A61K 31/4465, A61K 31/454, A61K 31/506, A61K 31/5365, A61K 31/5377, A61K 31/55

(54) **SUBSTITUTED INDOLES AND A METHOD FOR THE PRODUCTION AND USE THEREOF**

(30) Priority: 23.05.2006 RU 2006117557; 23.05.2006 RU 2006117558
(71) Applicant: Alla Chem, LLC., Karson City NV 89701 (US); Ivashchenko, Andrey, Alexandrovich, Moscow 127576 (RU)
(72) Inventor: IVASCHENKO, Andrey Alexandrovich, 127576 (RU); TKACHENKO, Sergey Yevgenievich, Moskovskaya obl., 142432 (RU); KHVAT, Alexander Viktorovich, San Diego, CA 92131 (US); MITKIN, Oleg Dmitrievich, Moscovskaya obl., 141400 (RU); OKUN, Ilya Matusovich, San Diego, CA 92121 (US); KYSELVEV, Alexandr Sergeevich, San Diego, CA 92130 (US); KYSIL, Volodymyr Mikhailovich, Kiev, 021140 (UA); SAVCHUK, Nikolay Filippovich, Moscow, 117420 (RU); IVASHCHENKO, Alexander Vasilievich, Encinitas, CA 92024 (RU)
(74) Representative: Henrion, Oliver
(86) International application number: PCT/RU2007/000244
(87) International publication number: WO 2007/136300

(57) **Abstract**

The invention relates to novel substituted indoles, to the use thereof in the form of pharmacological composition substances and to use of said compositions for the manufacture of medicinal preparations used for preventing and treating viral diseases, in particular caused by viruses of infectious hepatitis (HCV, HBV), human immune deficiency (HIV), atypical pneumonia (SARS) and bird flu. The invention proposes novel substituted indoles of general formula (I) or racemates, optical isomers, pharmaceutically acceptable salts and/or hydrates thereof, wherein R¹, R₁⁴ and R₂⁴ independently are an aminogroup substituent selected from hydrogen, optionally substituted alkyl, optionally substituted cycloalkyl, optionally substituted aryl or optionally substituted heterocyclyl, or R₁⁴ and R₂⁴ together with the nitrogen atom to which they are attached, form via R₁⁴ and R₂⁴ an optionally substituted azaheterocyclyl or guanidyl; R² is an alkyl substituent selected from hydrogen, an optionally substituted mercaptogroup, optionally substituted aminogroup and optionally substituted hydroxyl; R³ is lower alkyl, R⁵ is an hydrogen atom or R⁵ together with the oxygen atom to which it is attached and R₂⁴ together with the nitrogen atom to which it is attached close, via R⁵ and R₂⁴ an oxazine ring; R⁶ is a cyclic system substituent selected from hydrogen, halogen atom, cyanogroup, optionally substituted aryl or substituted heterocyclyl.

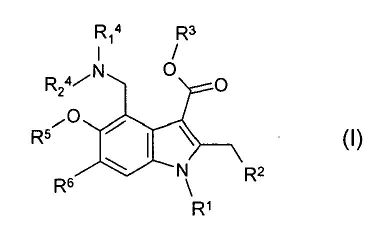

## Description

### Field of the invention

The present invention relates to new substituted indoles and their use as substances in pharmaceutical compositions, and also to the use of such pharmaceutical compositions for producing medicaments for prevention and treatment of viral diseases, particularly those caused by viruses of infectious hepatitis (HCV and HBV), human immunodeficiency virus (HIV), severe atypical respiratory syndrome (SARS), and bird flu.

### Background of the invention

Viral infections may cause many diseases, posing a serious threat to the health and life of humankind. In the past 20 years at least 30 absolutely new pathogens of infectious diseases have been discovered, including AIDS, viral hepatitis, acute and chronic diarrheas, hemorrhagic fevers (Ebola, Venezuelan, Brazilian, and Rift Valley fevers) [a) Lednicky J.A., Rayner J.O. Uncommon respiratory pathogens. Curr. Opin. Pulm. Med. 2006, 12(3), 235-239. b) Hayden F.G. Respiratory viral threats. Curr. Opin. Infect. Dis. 2006, 19(2), 169-178]. In particular, special concern is caused by the possibility of humans contracting the so-called bird (avian) flu. [a) Liu J.P. Avian influenza-a pandemic waiting to happen? J. Microbiol. Immunol. Infect. 2006, 39(1), 4-10. b) Henter J.I.; Chow C.B.; Leung C.W, Lau Y.L. Cytotoxic therapy for severe avian influenza A (H5N1) infection. Lancet. 2006 367(9513), 870-873. Review]. According to available statistics, between 60 and 65% of epidemic infections have a viral etiology. Because of the complexity of interactions in the triad of virus, host organism, and medicament, a majority of modem antiviral preparations have side effects in therapy and produce resistant virus strains [Jain R., Clark N.M., Diaz-Linares M., Grim S.A. Limitations of current antiretroviral agents and opportunities for development. Curr. Pharm. Des. 2006, 12(9), 1065-1074.]. In our day, the number of antiviral medicaments that can be used in clinical practice is very limited, a mere 43 low-molecular substances [http://integrity.prous.com/integrity], that are far from meeting the needs of viral disease prevention and treatment. Besides, there is a considerable number of viral infections causing diseases to treat which no chemotherapeutic medication have so far been developed. These are, for example, diseases caused by papilloma viruses, adenoviruses and viruses of Herpes-6, smallpox, SARS, hemorrhagic fevers, Western Nile fever, bird flu and so on [De Clercq E. Recent highlights in the development of new antiviral drugs. Curr Opin Microbiol. 2005, 8(5), 552-560].

Greatly important, therefore, are studies of new antiviral preparations, particularly those that have a novel antiviral mechanism, high activity, and low toxicity.

Antiviral pharmaceutical compositions known the art include an active substance such as ethyl ester hydrochloride of 6-bromo-4-(dimethylamino)-methyl-5-hydroxy-1-methyl-2-[(phenyl)thio]methyl-1H-indole-3-carboxylic acid (Arbidol) [Arbidol, PCT Int. Appl. WO 9008135, 1990]

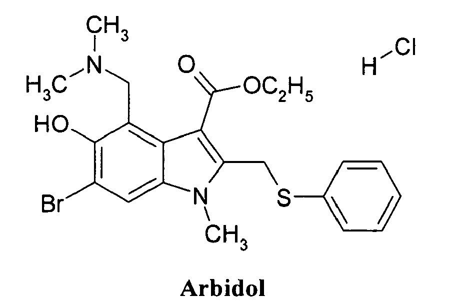

Arbidol is used to treat and prevent diseases caused by flu viruses. Arbidol is effective in inducing interferon and display an immunomodulation effect. [Arbidol. Drugs R. D. 1999, Sep; 2(3), 171-172. Glushkova, T.; Glushkov, R. Arbidol - Interferon inductor, immunomodulator, antioxidant. Rev. Esp. Quimioter. 2000, 13(Suppl. 2), Abstr. M182]. Recently, the antiviral activity of Arbidol has been demonstrated in relation to bird flu A/H5 [Fedyakina I.T., Leneva LA., Yamnikova S.S., Livov D.K., Glushkov R.G., Shuster A.M., Sensibility influenza viruses A/H5 recovered from wild birds on the territory of Russia to Arbidol in cell culture MDCK. Virology Problem, 2005, 50(6), 32-35], and suppressing activity of Arbidol against hepatitis C (HCV) has also been discovered [Y.S. Boriskin, E.I. Pécheur, S.J. Polyak. Arbidol: a broad-spectrum antiviral that inhibits acute and chronic HCV infection. Virology Journal 2006, 3:56 (http://www.virologyj.com/home)].

The principal drawback of Arbidol is, however, its high cell toxicity (CC₅₀ = 10-20 mM) and, for this reason, a small therapeutic window or a low selectivity index (SI₅₀). For the flu virus, for example, it is only 2.69 (on the MDCK cell line, TC₅₀ = 62.5 mkg/ml and IC₅₀ = 23.2 mkg/ml) [PCT Int Appl. WO 2005/087729 A1, 2005]. Its toxicity is even higher on some other cell lines (TC₅₀ = 15-25 µg/ml) [Brooks MJ: Studies with the antiviral drug arbidol [PhD thesis]. Melbourne, Australia: RMIT University; 2003].

Antiviral pharmaceutical compositions are known in the art that contain Arbidol analogs of the general formula **A** as the active substance: [PCT Int Appl. WO 2004060873, 2004**;** PCT Int Appl. WO 2005/087729 A1, 2005**.** Bioorg. Med. Chem. 2006, 14(4), 911-917]. 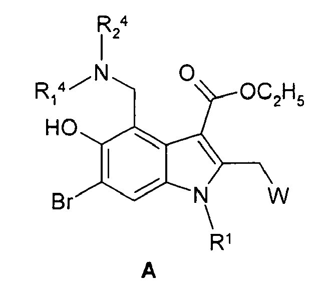 wherein: R¹ is alkyl or cycloalkyl; R₁⁴ and R₂⁴ independently from one another, an amino group substituent selected from hydrogen, optionally substituted alkyl, optionally substituted cycloalkyl, optionally substituted aryl or optionally substituted heterocyclyl, or R₁⁴ and R₂⁴, together with a nitrogen atom to which they are bound, form through R₁⁴ and R₂⁴ optionally substituted azaheterocyclyl; and W is a substituted mercapto group.

Arbidol analoges of the general formula A also display suppressing activity in relation to flu viruses A and B, and to viruses of hepatitis B (HBV) and human immunodeficiency virus (HIV) [Bioorg. Med. Chem. 2006, 14(4), 911-917. PCT Int Appl. WO 2005/087729 A1, 2005]. Like Arbidol itself, its analoges of the general formula A have a high cell toxicity and, for this reason, low selectivity index. For example, the selectivity index of this series of compounds in the case of hepatitis B is, as a rule, <10 (SI₅₀ = 1,81-10,8) [Bioorg. Med. Chem. 2006, 14(4), 911-917].

Substituted indoles are also known, which are 1,2,3,7-tetrahydro-pyrrolo[3,2-f][1,3]benzoxazines exhibiting properties of selective antagonists of M-4 muscarine receptors potentially suitable for treatment Parkinson's disease [Augelli-Szafran, C.E.; Jaen, J.C.; Moreland, D.W.; Nelson, C.B.; Penvose-Yi, J.R.; Schwarz, R.D. Identification and characterization of m4 selective antagonists. Bioorg. Med. Chem. Lett. 1998, 8(15), 1991].

Various types of biological activity are also displayed by 6-methyl-1,2,3,7-tetrahydro-pyrrolo[3,2-f][1,3]benzoxazine-5-carboxylic acids of the general formula **B** [DE 2408603, 1974**;** DE 2462471, 1977].

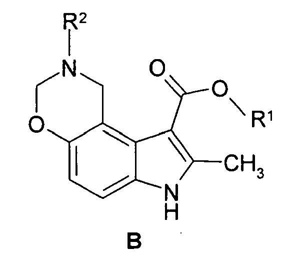

However, substituted 1,2,3,7-tetrahydropyrrolo[3,2-f][1,3]benzoxazines exhibiting antiviral activity have been unknown up to the present.

The search for highly effective antiviral preparations is today one of the key areas where new pharmacological medicaments are being developed for treating a wide and varied range of viral infections. Accordingly, a matter of urgency is the development of new compounds of this type, focused libraries, and pharmaceutical compositions containing these compounds, and also methods of producing and administering them.

### Disclosure of the Invention

Following below are definitions of terms that are used in describing this invention. **"Azaheterocycle"** means an aromatic or non-aromatic monocyclic or polycyclic system containing in the ring structure at least one nitrogen atom. An azaheterocyclic structure may contain one or more "substituents of the cyclic system".

**"Aliphatic ralical"** means a radical obtained by removing a hydrogen atom from the non-aromatic C-H bond. An aliphatic radical may further contain substituens, such as aliphatic or aromatic radicals defined in this section. Aliphatic radicals are represented by alkyl, alkenyl, alkynyl, cycloalkyl, cycloalkenyl, heterocyclyl, heterocyclenyl, aralkenyl, aralkyloxyalkyl, aralkyloxycarbonylalkyl, aralkyl, aralkynyl, aralkyloxyalkenyl, heteroaralkenyl, heteroaralkyl, heteroaralkyloxyalkenyl, heteroaralkyloxyalkyl, annelated arylcycloalkyl, annelated heteroarylcycloalkyl, annelated arylcycloalkenyl, annelated heteroarylcycloalkenyl, annelated arylheterocyclyl, annelated heteroarylheterocyclyl, annelated arylheterocyclenyl, annelated heteroarylheterocyclenyl.

**"Alkenyl"** means an aliphatic linear or branched hydrocarbon group containing from 2 to 7 carbon atoms and including double carbon-carbon bond. "Branched" means that one or more lower alkyl groups, such as methyl, ethyl or propyl, may be attached to a linear alkenyl chain. An alkyl group may have one or more substituents such as: halogen, alkenyloxy, cycloalkyl, cyano; hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkoxycarbonyl or Rₖ^{a}Rₖ₊₁^{a}N-, Rₖ^{a}Rₖ₊₁^{a}NC(=O)-, and Rₖ^{a}Rₖ₊₁^{a}NSO₂-, wherein Rₖ^{a} and Rₖ₊₁^{a} are, independently from one another, "substituents of the amino group", the value of which is defined elsewhere in this section, for example, a hydrogen atom, alkyl, aryl, aralkyl, heteroaralkyl, heterocyclyl or heteroaryl, or Rₖ^{a} and Rₖ₊₁^{a} together with an N-atom to they are attached, form through Rₖ^{a} and Rₖ₊₁^{a} a four - to seven-member heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, benzyloxycarbonylmethyl, and pyridylmethyloxycarbonylmethyl. The preferred alkenyl groups are ethenyl, propenyl, n-butenyl, iso-butenyl, 3-methylbuten-2-enyl, n-pentenyl and cyclohexylbutenyl.

**"Alkenyloxy"** means alkenyl-O-group, wherein alkenyl is defined in this section. Allyloxy and 3-butenyloxy are the preferred alkenyloxy groups.

**"Alkenyloxyalkyl"** means alkenyl-O-alkyl group, wherein alkyl and alkenyl are defined in this section.

**"Alkyl"** means an aliphatic hydrocarbon linear or branched group with 1-12 carbon atoms in the chain. Branched means that the alkyl chain has one or more "lower alkyl" substituents. Alkyl group may have one or more identical or different substituents ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NC(=S)-, Rₖ^{a}Rₖ₊₁^{a}NSO₂-, wherein Rₖ^{a} and Rₖ₋₁^{a} are, independently from one another, "amino group substituents", the value of which is defined elsewhere in this section, for example, a hydrogen atom, alkyl, aryl, aralkyl, heteroaralkyl, heterocyclyl or heteroaryl, or Rₖ^{a} and Rₖ₊₁^{a} together with an N-atom to which they are attached, form through Rₖ^{a} and Rₖ₊₁^{a}, a four- to seven-member heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl and pyridylmethyloxycarbonyl-methyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl

**"Alkyloxyalkyl"** means alkyl-O-alkyl group, wherein alkyl groups are, independently from one another, defined in this section. The preferred alkyloxyalkyl groups are methoxyethyl, ethoxymethyl, n-butoxymethyl, methoxypropyl and iso-propyloxyethyl.

**"Alkoxycarbonyl"** means alkyl-O-C(=O)-group, wherein alkyl is defined in this section. The preferred alkoxycarbonyl groups are methoxycarbonyl, ethoxycarbonyl, n-butoxycarbonyl, isopropyloxycarbonyl, benzyloxycarbonyl and phenethyloxycarbonyl.

**"Alkylthio"**means alkyl-S group,wherein alkyl group is defined in this section.

**"Alkoxy"** means alkyl-O-group, wherein alkyl is defined in this section. The preferred alkoxy groups are methoxy, ethoxy, n-propoxy, iso-propoxy and n-butoxy.

**"Alkoxycarbonylalkyl"** means alkyl-O-C(=O)-alkyl-group, wherein alkyl is defined in this section. The preferred alkoxycarbonylalkyl groups are methoxy-carbonylmethyl, ethoxycarbonylmethyl, methoxy-carbonylethyl and ethoxy-carbonylethyl.

**"Amino group"** means Rₖ^{a}Rₖ₊₁^{a}N-group substituted or not by "amino group substituent", the value of Rₖ^{a} and Rₖ₊₁^{a} are defined in this section, for example, amino (NH₂), methylamino, diethylamino, pyrrolidino, morpholino, benzylamino or phenethylamino.

**"Amino acid"** means a natural amino acid or non-natural amino acid, the value of which is defined in this section. The preferred amino acids are amino acids containing α- or β-amino group. Examples of natural amino acids are α-amino acids, and also alanine, valine, leucine, isoleucine, proline, phenylalanine, triptophane, methionine, glycine, serine, threonine, and cysteine.

**"Annelated cyclic structure"** (condensed cyclic structure) means bi- or polycyclic system in which the annelated cyclic structure and cyclic structure, or polycyclic structure to which it is "annelated" have at least two common atoms.

**"Annelated arylheterocycloalkenyl"** means an annelated aryl and heterocycloalkenyl, the value of which is defined in this section. Annelated arylheterocycloalkenyl may be bound through any possible atom of the cyclic system. The prefixes "aza", "oxa" or "thia" preceding the word "heterocycloalkenyl" indicate the presence of a nitrogen atom, an oxygen atom, or a sulfur atom, respectively, in the cyclic system. Annelated arylheterocycloalkenyl may have one or more "cyclic system substituens" that may be identical or different. Nitrogen and sulfur atoms in the heterocycloalkenyl part may be oxidized to an N-oxide, an S-oxide or an S-dioxide. Annelated arylheterocycloalkenyl are represented by indolinyl, 1H-2-oxoquinolinyl, 2H-1-oxoisoquinolinyl, 1,2-dihydroquinolinyl, and so on.

**"Annelated arylheterocycloalkyl"** means an annelated aryl and heterocycloalkyl the value of which is defined in this section. Annelated arylheterocycloalkyl may be bound through any possible atom of the cyclic system. The prefixes "aza", "oxa" or "thia" preceding the word "heterocycloalkyl" indicate the presence of a nitrogen atom, an oxygen atom, or a sulfur atom, respectively, in the cyclic system. Annelated arylheterocycloalkyl may have one or more "cyclic system substituens" that may be identical or different. Nitrogen and sulfur atoms in the heterocyclylalkyl part may be oxidized to an N-oxide, an S-oxide and an S-dioxide. Annelated arylheterocycloalkyls are represented by indolyl, 1,2,3,4-tetrahydroisoquinolinyl, 1,3-benzodiocolyl, and so on.

**"Annelated arylcycloalkenyl"**means an annelated aryl and cycloalkenyl, the value of which is defined in this section. Annelated arylcycloalkenyl may be bound through any possible atom of the cyclic system. Annelated arylcycloalkenyl may have one or more "cyclic system substituents" that may be identical or different. Annelated arylcycloalkenyls are represented by 1,2-dihydronaphthalenyl, indenyl and so on.

**"Annelated arylcycloalkyl"** means an annelated aryl and cycloalkyl, the value of which is defined in this section. Annelated arylcycloalkyl may be bound through any possible atom of the cyclic system. Annelated arylcycloalkyl may have one or more "cyclic system substituens" that may be identical or different. Annelated arylcycloalkyl are represented by indaninyl, 1,2,3,4-tetrahydranaphthyl, 5,6,7,8-tetrahydronapht-1-yl, and so on.

**"Annelated heteroarylcycloalkenyl"** means an annelated heteroaryl and cycloalkenyl, the value of which is defined in this section. Annelated heteroarylcycloalkenyl may be bound through any possible atom of the cyclic system. The prefixes "aza", "oxa" or "thia" preceding the word "heterocycloalkyl" indicate the presence of a nitrogen atom, an oxygen atom, or a sulfur atom, respectively, in the cyclic system. Annelated heteroarylcycloalkenyl may have one or more "cyclic system substituents" that may be identical or different. The nitrogen atom in the heteroaryl part may be oxidized to N-oxide. Annelated heteroarylcycloalkenyls are represented by 5,6-dihydroquinolinyl, 5,6-dihydroisoquinolinyl, 4,5-dihydro-1H-benzimidazolyl, and so on.

**"Annelated heteroarylcycloalkyl"** means an annelated heteroaryl and cycloalkyl, the value of which is defined in this section. Annelated heteroarylcycloalkyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system. Annelated heteroarylcycloalkyl may have one or more "cyclic system substituents" that may be identical or different. The nitrogen atom in the heteroaryl part may be oxidized to an N-oxide. Annelated heteroarylcycloalkyls are represented by 5,6,7,8-tetrahydroquinolinyl, 5,6,7,8-tetrahydroisoquinolinyl, 4,5,6,7-tetrahydro-1H-benzimidazolyl, and so on

**"Annelated heteroarylheterocyclenyl"** means an annelated heteroaryl and heterocyclenyl, the value of which is defined in this section. Annelated heteroarylheterocyclenyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system. Annelated heteroarylheterocyclenyl may have one or more "cyclic system substituents" that may be identical or different. The nitrogen atom in the heteroaryl part may be oxidized to an N-oxide. The nitrogen atom and the sulfur atom in the heterocyclenyl part may be oxidized to an N-oxide, an S-oxide and an S-dioxide. Annelated heteroarylheterocyclenyls are represented by 1,2-dihydro[2,7]naphthiridinyl, 7,8-dihydro[1,7]naphthiridinyl, 6,7-dihydro-3H-imidazo[4,5-c]pyridyl, and so on.

**"Annelated heteroarylheterocyclyl"** means an annelated heteroaryl and heterocyclyl, the value of which is defined in this section. Annelated heteroarylheterocyclyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system. Annelated heteroarylheterocyclyl may have one or more "cyclic system substituents" that may be identical or different. The nitrogen atom in the heteroaryl part may be oxidized to an N-oxide. The nitrogen atom and the sulfur atom in the heterocyclyl part may be oxidized to an N-oxide, an S-oxide and an S-dioxide. Annelated heteroarylheterocyclyls are represented by 2,3-dihydro-1H-pyrrolo[3,4-b]quinolin-2-yl, 2,3-dihydro-1H-pyrrolo[3,4-b]indol-2-yl, 1,2,3,4-tetrahydro[1,5]naphthiridinyl, and so on.

**"Aralkenyl"** means aryl-alkenyl group, wherein aryl and alkenyl are defined in this section. For example, 2-phenethenyl is aralkenyl group.

**"Aralkyl"** means alkyl group substituted by one or more aryl groups, wherein aryl and alkyl are defined in this section. For example, benzyl-, 2,2-diphenylethyl- or phenethyl- are aralkyl groups.

**"Aralkylamino"** means aryl-alkyl-NH-group, wherein aryl and alkyl are defined in this section.

**"Aralkylsulfinyl"** means aralkyl-SO-group, wherein aralkyl is defined in this section.

**"Aralkylsulfonyl"** means aralkyl-SO₂-group, wherein aralkyl is defined in this section.

**"Aralkylthio"** means aralkyl-S-group, wherein aralkyl is defined in this section.

"Aralkoxy" means aralkyl-O-group, wherein aralkyl is defined in this section. For example, benzyloxy or 1- or 2-naphthylenmethoxy are aralkyl groups.

**"Aralkoxyalkyl"** means aralkyl-O-alkyl-group, wherein aralkyl and alkyl are defined in this section. For example, benzyloxyethyl is aralkyl-O-alkyl group.

**"Aralkoxycarbonyl"** means aralkyl-O-C(=O)-group, wherein aralkyl is defined in this section. Benzyloxycarbonyl is an example of aralkoxycarbonyl group.

**"Aralkoxycarbonylalkyl"** means aralkyl-O-C(=O)-alkyl-group, wherein aralkyl and alkyl are defined in this section. Benzyloxycarbonylmethyl or benzyloxycarbonylethyl are examples of aralkoxycarbonylalkyl groups.

**"Aryl"** means an aromatic monocyclic or polycyclic system containing 6 to 14 carbon atoms, preferably from 6 to 10 carbon atoms. Aryl may contain one or more "cyclic system substituents" that may be identical or different. Aryl groups are represented by phenyl or naphthyl, substituted phenyl or substituted naphthyl. Aryl may be annelated to non-aromatic cyclic system or heterocyclic structure.

**"Arylcarbamoyl"** means aryl-NHC(=O)-group, wherein aryl is defined in this section.

**"Aryloxy"** means aryl-O-group, wherein aryl is defined in this section. Phenoxy- and 2-naphthyloxy- are the representatives of aryloxy groups.

**"Aryloxycarbonyl"** means aryl-O-C(=O)-group, wherein aryl is defined in this section. Phenoxycarbonyl and 2-naphthoxycarbonyl are the representatives of aryloxycarbonyl groups.

**"Arylsulfinyl"** means aryl-SO-group, wherein aryl is defined in this section.

**"Arylsulfonyl"** means aryl-SO₂-group, wherein aryl is defined in this section.

**"Arylthio"** means aryl-S-group, wherein aryl is defined in this section. Phenylthio- and 2-naphthylthio- are the representatives of arylthio groups.

**"Aroylamino"** means aroyl-NH-group, wherein aroyl is defined in this section.

**"Aroyl"** means aryl-C(=O)-group, wherein aryl is defined in this section. Benzoyl-, 1- and 2-naphthoyl- are the representatives of aroyl groups.

**"Aromatic radical"** means a radical obtained by dropping a hydrogen atom from the aromatic C-H bond. "Aromatic" radical contains aryl and heteroaryl cyclic structures defined in this section. Aryl and heteroaryl cyclic structures may further contain substituents, such as aliphatic or aromatic radicals defined in this section. Aromatic radicals are represented by aryl, annelated cycloalkenylaryl, annelated cycloalkylaryl, annelated heterocyclylaryl, annelated heterocyclenylaryl, heteroaryl, annelated cycloalkylheteroaryl, annelated cycloalkenylheteroaryl, annelated heterocyclenylheteroaryl and annelated heterocyclylheteroaryl.

**"Aromatic cyclic structure"** means a planar cyclic system in which all cyclic system atoms are involved in forming a common conjugation system containing, according to Hückel's rule, (4n + 2) π-electrons (n is a non-negative number). Examples of aromatic cyclic structures are benzene, naphthalene, anthracene, and so on. In the case of "heteroaromatic cyclic structures", the conjugation system involves π-electrons and p-electrons of heteroatoms, their total number being (4n + 2) as well. Examples of such cyclic structures are pyridine, thiophene, pyrrole, furan, thiazole, and so on. The aromatic cyclic structure may have one or more "cyclic system substituents" and may be annelated to non-aromatic cyclic structure, heteroaromatic or heterocyclic system.

**"Acyl"** means H-C(=O)-, alkyl-C(=O)-, cycloalkyl-C(=O), heterocyclyl-C(=O)-, heterocyclylalkyl-C(=O)-, aryl-C(=O)-, arylalkyl-C(=O)-, heteroaryl-C(=O)-, heteroarylalkyl-C(=O)-groups, wherein alkyl-, cycloalkyl-, heterocyclyl-, heterocyclylalkyl-, aryl-, arylalkyl-, heteroaryl-, heteroarylalkyl are defined in this section.

**"Acylamino"**means acyl-NH-group wherein acyl is defined in this section.

**"1,2-Vinyl radical"** means -CH=CH-group with one or more "alkyl substituents" that may be identical or different, the value of which are defined in this section.

**"Halogen"** means fluorine, chlorine, bromine and iodine. Preference is given to fluorine, chlorine and bromine.

**"Heteroannelated cyclic structure"** means that the cyclic structure that is attached (annelated or condenced) to another cyclic or polycyclic structure contains at least one heteroatom.

**"Heteroaralkenyl"** means heteroaryl-alkenyl-group, wherein heteroaryl and alkenyl are defined in this section. Preferably, heteroarylalkenyl contains the lower alkenyl group. Heteroarylalkenyls are represented by 4-pyridylvinyl, thienylethenyl, imidazolylethenyl, pyrazinylethenyl, and so on.

**"Heteroaralkyl"** means heteroaryl-alkyl-group, wherein heteroaryl and alkyl are defined in this section. Heteroaralkyls are represented by pyridylmethyl, thienylmethyl, furylmethyl, imidazolylmethyl, pyrazinylmethyl, and so on.

**"Heteroaralkyloxy group"** means heteroarylalkyl-O-group, wherein heteroarylalkyl is defined in this section. Heteroaralkyloxy group are represented by 4-pyridylmethyloxy, 2-thienylmethyloxy, and so on.

**"Heteroaryl"** means aromatic monocyclic or polycyclic system with 5 to 14 carbon atoms, preferably 5 - 10 C-atoms, wherein one or more carbon atoms are substituted by one or more heteroatoms, such as N, S or O. The prefixes "aza", "oxa" or "thia" preceding the word "heteroaryl" indicate the presence of a nitrogen atom, an oxygen atom, or a sulfur atom, respectively, in the cyclic system. Nitrogen atom in the heteroaryl part may be oxidized to an N-oxide. Heteroaryl may have one or more "cyclic system sustituents" that may be identical or different. Heteroaryl radicals are represented by pyrrolyl, furanyl, thienyl, pyridyl, pyrazinyl, pyrimidinyl, isooxazolyl, isothiazolyl, tetrazolyl, oxazolyl, thiazolyl, pyrazolyl, furazanyl, triazolyl, 1,2,4-thiadiazolyl, pyridazinyl, quinoxalinyl, phthalazinyl, imidazo[1,2-a]pyridinyl, imidazo[2,1-b]thiazolyl, benzofurazanyl, indolyl, azaindolyl, benzimidazolyl, benzothiazenyl, quinolinyl, imidazolyl, thienopyridyl, quinazolinyl, thienopyrimidinyl, pyrrolopyridinyl, imidazopyridinyl, isoquinolinyl, benzoazaindolyl, 1,2,4-triazinyl, thienopyrrolyl, furopyrrolyl, and so on.

**"Heteroarylsulfonylcarbamoyl"** means heteroaryl-SO₂-NH-C(=O)-group, wherein heteroaryl is defined in this section.

**"Heteroaroyl"-** means heteroaryl-C(=O)-group, wherein heteroaryl is defined in this section. Heteroaroyl groups are represented by nicotinoyl, thienoyl, pyrazoloyl, and so on.

**"Heterocyclenyl"** means non-aromatic monocyclic or polycyclic system containing 3 to 13 carbon atoms, preferably from 5 to 13 carbon atoms in which one or several carbon atoms are replaced with a heteroatom such as nitrogen, oxygen or sulfur, and which contains at least one double carbon-carbon bond or double carbon-nitrogen bond. The prefixes "aza", "oxa" or"thia" preceding the word "heterocyclenyl" indicate the presence of a nitrogen atom, an oxygen atom, or a sulfur atom, respectively, in the cyclic system. Heterocyclenyl may have one or more "cyclic system substituens" that may be identical or different. Nitrogen atom and sulfur atom in the heterocyclenyl part may be oxidized to an N-oxide, an S-oxide and an S-dioxide. Heterocyclenyl groups are represented by 1,2,3,4-tetrahydropyridinyl, 1,2-dihydropyridinyl, 1,4-dihydropyridinyl, 2-pyrrolinyl, 3-pyrrolinyl, 2-imidazolyl, 2-pyrazolinyl, dihydrofuranyl, dihydrothiophenyl, and so on.

**"Heterocyclyl"** means an aromatic or nonaromatic saturated monocyclic or polycyclic system containing 3 to 10 carbon atoms, preferably 5 to 6 carbon atoms, in which one or several carbon atoms are replaced with a heteroatom such as nitrogen, oxygen or sulfur. The prefixes "aza", "oxa" or "thia" preceding the word "heterocyclyl" indicate the presence of a nitrogen atom, an oxygen atom, or a sulfur atom, respectively, in the cyclic system. Heterocyclyl may have one or more "cyclic system substituents" that may be identical or different. Nitrogen atom and sulfur atom in heterocyclyl fragment may be oxidized to an N-oxide, an S-oxide and an S-dioxide. Heterocyclyls are represented by piperidinyl, pyrrolidinyl, piperazinyl, morpholinyl, thiomorpholinyl, thiazolidinyl, 1,4-dioxan-2-yl, tetrahydrofuranyl, tetrahydrothiophenyl, and so on.

**"Heterocyclyloxy"** means heterocyclyl-O-group, wherein heterocyclyl is defined in this section.

**"Hydrate"** means stoichiometric or nonstoichiometric compositions of the compounds or their salts with water.

**"Hydroxyalkyl"** means HO-alkyl-group, wherein alkyl is defined in this section.

**"Substituent"** means a chemical radical that is attached to a scaffold (fragment), for example, "alkyl substituent", "amino group substituent", "carbamoyl substituent", and "cyclic system substituent", the meanings of which are defined in this section.

**"Alkyl substituent"** means a chemical radical that is attached to alkyl or alkenyl group, the meanings of which are defined in this section. It may be selected from hydrogen, alkyl, halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NSO₂-, wherein Rₖ^{a} and Rₖ₊₁^{a} are, independently from one another, "amino group substituent", the meanings of which are defined in this section, for example, hydrogen, alkyl, aryl, aralkyl, heteroaralkyl, heterocyclyl or heteroaryl, or Rₖ^{a} and Rₖ₊₁^{a} together with a nitrogen atom to which they are bound, form through Rₖ^{a} and Rₖ₊₁^{a} a four- to seven-member heterocyclyl or heterocyclenyl. The preferred alkyl groups are represented by methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl, methoxycarbonylmethyl, and pyridylmethyloxycarbonylmethyl. The preferred "alkyl substituents" are represented by cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl. The meanings of "alkyl group substituents" are defined in this section.

**"Amino group substituent"** means a substituent attached to an amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl. The meanings of "amino group substituents" are defined in this section.

**"Carbamoyl substituent"** means a substituent attached to carbamoyl group, the value of which is defined in this section. Carbamoyl substituent may be selected from hydrogen, alkyl, cycloalkyl, aryl, heteroaryl, heterocyclyl, alkoxycarbonylalkyl, aralkoxycarbonylalkyl, heteroaralkyloxycarbonylalkyl or Rₖ^{a}Rₖ₊₁^{a}N-, Rₖ^{a}Rₖ₊₁^{a}NC(=O)-alkyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl. The preferred "carbamoyl substituents" are represented by alkyl, cycloalkyl, aryl, heteroaryl, heterocyclyl, alkoxycarbonylalkyl, aralkoxycarbonylalkyl, heteroaralkyloxycarbonylalkyl or Rₖ^{a}Rₖ₊₁^{a}N-, Rₖ^{a}Rₖ₊₁^{a}NC(=O)-alkyl, annelated arylheterocyclenyl, annelated arylheterocyclyl. The meanings of "carbamoyl substituents" are defined in this section.

**"Nucleophilic substituent"** is a chemical radical that is attached to the scaffold as a result of a reaction with a nucleophilic reagent, for example, one selected from the group of primary or secondary amines, alcohols, phenols, mercaptans and thiophenols.

**"Cyclic system substituent"** means a substituent attached to an aromatic or nonaromatic cyclic system selected from hydrogen, alkyl, alkenyl, alkynyl, aryl, heteroaryl, aralkyl, heteroaralkyl, hydroxy, hydroxyalkyl, amino, aminoalkyl, alkoxy, aryloxy, acyl, aroyl, halogen, nitro, cyano, carboxy, alkoxycarbonyl, aryloxycarbonyl, aralkoxycarbonyl, alkyloxyalkyl, aryloxyalkyl, heterocyclyloxyalkyl, arylalkyloxyalkyl, heterocyclylalkyloxyalkyl, alkylsulfonyl, arylsulfonyl, heterocyclylsulfonyl, alkylsulfinyl, arylsulfinyl, heterocyclylsulfinyl, alkylthio, arylthio, heterocyclylthio, alkylsulfonylalkyl, arylsulfonylalkyl, heterocyclylsulfonylalkyl, alkylsulfinylalkyl, arylsulfinylalkyl, heterocyclylsulfinylalkyl, alkylthioalkyl, arylthioalkyl, heterocyclylthioalkyl, arylalkylsulfonylalkyl, heterocyclylalkylsulfonylalkyl, arylalkylthioalkyl, heterocyclylalkylthioalkyl, cycloalkyl, cycloalkenyl, heterocyclyl, heterocyclenyl, amidino, Rₖ^{a}Rₖ₊₁^{a}N-, Rₖ^{a}N=, Rₖ^{a}Rₖ₊₁^{a}N-alkyl, Rₖ^{a}Rₖ₊₁^{a}NC(=O)- or Rₖ^{a}Rₖ₊₁^{a}NSO₂-, wherein Rₖ^{a} and Rₖ₊₁^{a} are, independently from one another, an "amino group substituent", the meanings of which are defined in this section, for example, hydrogen, optionally substituted alkyl, optionally substituted aryl, optionally substituted aralkyl, optionally substituted heteroaralkyl or Rₖ^{a}Rₖ₊₁^{a}N-substituent wherein Rₖ^{a} may be acyl or aroyl, the value of Rₖ₊₁^{a} is defined above, or "cyclic system substituents" are Rₖ^{a}Rₖ₊₁^{a}NC(=O)- or Rₖ^{a}Rₖ₊₁^{a}NSO₂- ,wherein Rₖ^{a} and Rₖ₊₁^{a}, together with a nitrogen atom to which they are bound, form through Rₖ^{a} and Rₖ₊₁^{a} a four to seven-member heterocyclyl or hetrocyclenyl.

**"Electrophilic substituent"** means a chemical radical attached to the scaffold as a result of a reaction with an electrophilic reagent, for example, one selected from a group of organic acids or their derivatives (anhydrides, imidazolides, acid chlorides), organic sulfonic acid esters or chlorides, organic haloformates, organic isocyanates and organic isothiocyanates.

**"Substituted amino group"** means Rₖ^{a}Rₖ₊₁^{a}N-group wherein Rₖ^{a} and Rₖ₊₁^{a}are the substituents of an amino group, the meanings of which are defined in this section.

**"Substituted carboxy group"** means C(O)OR-group. Substituted carboxyl has substituent R selected from alkenyl, alkyl, aryl, heteroaryl, heterocyclyl, the meanings of which are defined in this section.

**"Substituted mercapto group"** means SR, S(O)R or S(O₂)R group wherein substituent R represents alkenyl, alkyl, aryl, heteroaryl, heterocyclyl, the meanings of which are defined in this section.

**"Protective group" (PG)** means a chemical radical that is attached to the scaffold or intermediate product of synthesis to provide temporary protection for amino group in multifunctional compounds, including, but not limited to: an amide substituent, such as formyl, optionally substituted acetyl (for example, trichloroacetyl, trifluoroacetyl, 3-phenylpropionyl and so on), optionally substituted benzoyl and so on; a carbamate substituent, such as: optionally substituted C₁-C₇-alkoxycarbonyl, for example, methyloxycarbonyl, ethyloxycarbonyl, tert-butyloxycarbonyl, 9-fluorenylmethyloxycarbonyl (Fmoc) and others; optionally substituted C₁-C₇-alkyl substituent, for example tert-butyl, benzyl, 2,4-dimethoxybenzyl, 9-phenylfluorenyl and others; sulfonyl substituent, for example, benzenesulfonyl, p-toluenesulfonyl, etc.

**"Protected primary or secondary amine"** means a group of the formula Rₖ^{a}Rₖ₊₁^{a}N-, wherein Rₖ^{a} is a protective group PG, Rₖ₊₁^{a} is hydrogen, an "amino group substituent", the value of which is defined in this section, for example, selected from alkyl, alkenyl, aryl, aralkyl, annelated arylcycloalkenyl, annelated arylcycloalkyl, annelated arylheterocyclenyl, annelated arylheterocyclyl, cycloalkyl, cycloalkenyl, heteroaralkyl, heteroaryl, annelated heteroarylcycloalkenyl, annelated heteroarylcycloalkyl, annelated heteroarylheterocyclenyl, annelated heteroarylheterocyclyl, heterocyclenyl or heterocyclyl.

**"Imino group"** means Rₖ^{a}N= group substituted or not by an "amino group substituent" Rₖ^{a}, the value of which is defined in this section, for example, imino (HN=), methylimino (CH₃N=), ethylimino (C₂H₅N=), benzylimino (PhCH₂N=) or phenethylimino (PhCH₂CH₂N=).

**"Inert substituent"** ("non-interfering substituent") means a low- or non-reactive radical, including, but not limited to: C₁-C₇ alkyl, C₂-C₇ alkenyl, C₂-C₇ alkynyl, C₁-C₇ alkoxy, C₇-C₁₂ aralkyl, substituted by inert substituents aralkyl, C₇-C₁₂ heterocyclylalkyl, substituted by inert substituents heterocyclylalkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₂-C₁₀ alkylsulfinyl, C₂-C₁₀ alkylsulfonyl, (CH₂)ₘ-O-(C₁-C₇ alkyl), -(CH₂)ₘ-N(C₁-C₇ alkyl)", aryl; aryl substituted by halogen or inert substituent, alkoxy substituted by inert substituent, fluoroalkyl, aryloxyalkyl, heterocyclyl, heterocyclyl substituted by inert substituents and nitroalkyl; where m and n are ranged from 1 to 7. The preferred "non-interfering substituents" are represented by C₁-C₇ alkyl, C₂-C₇ alkenyl, C₂-C₇ alkynyl, C₁-C₇ alkoxy, C₇-C₁₂ aralkyl, C₇-C₁₂ alkaryl, C₃-C₁₀ cycloalkyl, C₃-C₁₀ cycloalkenyl, C₁-C₇ alkyl substituted by inert substituents, phenyl; phenyl substituted by inert substituents, (CH₂)ₘ-O-(C₁-C₇ alkyl), -(CH₂)ₘ-N(C₁-C₇ alkyl)ₙ, aryl; aryl substituted by inert substituents, heterocyclyl and heterocyclyl substituted by inert substituents.

**"Carbamoyl"** means C(=O)NRₖ^{a}Rₖ₊₁^{a}- group. Carbamoyl may have one or more "carbamoyl substituents" Rₖ^{a} and Rₖ₊₁^{a}, selected from hydrogen alkyl, alkenyl, aryl, heteroaryl, heterocyclyl, the meanings of which are defined in this section.

**"Carbamoylazaheterocycle"** means azaheterocycle with at least one carbamoyl group as a "cyclic system substituent". The meanings of "azaheterocycle", "cyclic system substituent", and "carbamoyl group" are defined in this section.

**"Carboxy"** means HOC(=O)- (carboxy) group.

**"Carboxyalkyl"** means HOC(=O)-alkyl group, wherein alkyl is defined in this section.

**"Carbocyclic structure"** means mono- or polycyclic system consisting of carbon atoms only. Carbocyclic rings can be either aromatic or alicyclic. Alicyclic polycyclic structures may have one or more common atoms. In the case of one common atom, spiro-carbocyclic compounds (for example, spiro[2,2]pentane) are formed; in the case of two common atoms, various condensed systems (for example, decaline) are produced; three common atoms result in bridged systems (for example, bicycle[3,3,1]nonane); a larger number of common atoms produce various polyhedral systems (for example, adamantane). Alicyclic structures may be "saturated", such as, for example, cyclohexane, or "partlially saturated", such as, for example, tetraline.

**"Combinatorial library"** means a collection of compounds produced by parallel synthesis and intended for searching for a hit or leader compound, and for optimization of physiological activity of the hit or leader as well, each compound of the library corresponds to the common scaffold, in this way the library is a collection of related homologues or analogues.

**"Methylene radical"** means -CH₂-group with one or two "alkyl substituents" that may be identical or different, the meanings of which are defined in this section.

**"Nonaromatic cyclic structure"** (saturated cyclic structure or partly saturated cyclic structure) means nonaromatic cyclic or polycyclic system formally derived as a result of complete or partial hydrogenization of unsaturated -C=C- or -C=N- bonds. A nonaromatic cyclic structure may have one or more "cyclic system substituents" and may be annelated to aromatic, heteroaromatic or heterocyclic systems. Examples of nonaromatic cyclic structures are cyclohexane or piperidine; examples of partly saturated cyclic structures are cyclohexene or piperideine.

**"Non-natural aminoacid"** means an aminoacid of non nucleinic origin. D-isomers of natural α-aminoacids, such as aminobutyric acid, 2-aminobutyric acid, γ-aminobutyric acid, N-α-alkylaminoacids, 2,2-dialkyl-α-aminoacids, 1-aminocycloalkylcarboxylic acids, β-alanine, 2-alkyl-β-alanines, 2-cycloalkyl-β-alanines, 2-aryl-β-alanines, 2-heteroaryl-β-alanines, 2-heterocyclyl-β-alanines and (1-aminocycloalkyl)-acetic acids are the representatives of non natural aminoacids in which the meanings of alkyl, cycloalkyl, aryl, heteroaryl and heterocyclyl are defined in this section.

**"Optionally aromatic cycle"** means a cycle which may be either aromatic or non-aromatic, the meanings of which are defined in this section.

**"Optionally substituted radical"** means a radical without or with one or more substituents.

**"Optionally annelated (condensed) cycle"** means a condensed or non-condensed cycle, the meanings of which are defined in this section.

**"Lower alkyl"** means a linear or branched alkyl radical containing from 1 to 4 carbon atoms.

**"Parallel synthesis"** means a method for carrying out a chemical synthesis of combinatorial library of individual compounds.

**"1,3-Propylene radical"** means -CH₂-CH₂-CH₂-group with one or more "alkyl substituents" that may be identical or different, the meanings of which are defined in this section.

**"Leader compound"** (leader) means a compound of outstanding (maximum) physiological activity associated with a concrete bio-target related to a definite (or several) pathology or disease.

**"Hit compound"** (hit) means a compound demonstrated the desired physiological activity during the primary screening process.

**"Sulfamoyl group"** means Rₖ^{a}Rₖ₊₁^{a}NSO₂-group substituted or not by "amino group substituents" Rₖ^{a} and Rₖ₊₁^{a}, the meanings of which are defined in this section.

**"Sulfonyl"** means R-SO₂-group wherein R may be selected from alkyl, cycloalkyl, aryl, heteroaryl, heterocyclyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, the meanings of which are defined in this section.

**"Template"** means the common structural formula of a group of compounds or compounds contained in the "combinatorial library".

**"Therapeutic kit"** is a combination of two or more medicaments administered simultaneously, which have different pharmacological mechanisms and are aimed at different bio-targets involved in the disease pathogenesis.

**"Thiocarbamoyl"** means Rₖ^{a}Rₖ₊₁^{a}NC(=S)-group. Thiocarbamoyl may have one or more "amino group substituents" Rₖ^{a} and Rₖ₊₁^{a}, the meanings of which are defined in this section, for example, alkyl, alkenyl, aryl, heteroaryl and heterocyclyl the meanings of which are defined in this section.

**"Cycloalkyl"** means non-aromatic monocyclic or polycyclic system containing from 3 to 10 carbon atoms. Cycloalkyl may have one or more "cyclic system substituents" that may be identical or different. The cyclic system groups are represented by cyclopropyl, cyclobutyl, cyclopentyl, cyclohexyl, decalinyl, norbornyl, adamant-1-yl, and so on. Cycloalkyl may be annelated to aromatic cycle or heterocycle. The preferred "cyclic system substituents" are represented by alkyl, aralkoxy, hydroxy or Rₖ^{a}Rₖ₊₁^{a}N-, the meanings of which are defined in this section.

**"Cycloalkylcarbonyl"** means cycloalkyl-C(=O)-group, wherein the value of cycloalkyl is defined in this section. Cyclopropylcarbonyl and cyclohexylcarbonyl are the representatives of cycloalkylcarbonyl groups.

**"Cycloalkoxy"** means cycloalkyl-O-group, wherein the value of cycloalkyl is defined in this section.

**"Pharmaceutical composition"** means a composition containing a compound of formula **I** and at least one of the components selected from a group consisting of pharmaceutically acceptable and pharmacologically compatible fillers, solvents, diluents, auxiliary, distributing and sensing agents, delivery agents, such as preservatives, stabilizers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and method of prescription and dosage. Examples of suspending agents are ethoxylated isostearyl alcohol, polyoxyethene, sorbitol and sorbitol ester, microcrystalline cellulose, aluminum metahydroxide, bentonite, agar-agar and tragacanth, and also mixtures of these substances. Protection against the effect of microorganisms can be provided by various antibacterial and antifungal agents, such as, for example, parabens, chlorobutanol, sorbic acid, and similar compounds. The composition may also contain isotonic agents, such as, for example, sugar, sodium chloride, and similar compounds. A prolonged effect of the composition may be achieved by agents slowing absorption of the active ingredient, for example, aluminum monostearate and gelatine. Examples of suitable carriers, solvents, diluents and delivery agents include water, ethanol, polyalcohols, and their mixtures, vegetable oils (such as olive oil) and for injection-grade organic esters (such as ethyl oleate). Examples of fillers are lactose, milk sugar, sodium citrate, calcium carbonate, calcium phosphate and the like. Examples of disintegrators and distributors are starch, alginic acid and its salts, and silicates. Examples of lubricants are magnesium stearate, sodium lauryl sulfate, talc and high molecular weight polyethylene glycol. A pharmaceutical composition for peroral, sublingual, transdermal, intramuscular, intravenous, subcutaneous, local or rectal administration of the active ingredient, alone or in combination with another active ingredient, may be administered to humans and animals in a standard administration form, or in a mixture with traditional pharmaceutical carriers. Suitable standard administration forms include peroral forms, such as tablets, gelatin capsules, pills, powders, granules, chewing-gums, and peroral solutions or suspensions; sublingual and transbuccal administration forms; aerosols; implants; local, transdermal, subcutaneous, intramuscular, intravenous, intranasal or intraocular forms, and rectal administration forms.

**"Pharmaceutically acceptable salt"** means relatively nontoxic organic and inorganic salts of acids and bases claimed in this invention. The salts may be produced in situ during synthesis, separation or purification of compounds, or to be prepared purposefully. In particular, base salts may be prepared purposefully, starting from a purified free base of a claimed compound and a suitable organic or inorganic acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, mesylates, malonates, salicilates, propionates, ethane sulphonates, benzene sulfonates, sulfamates and the like (Detailed description of the properties of such salts is given in: Berge S.M., et al., "Pharmaceutical Salts" J. Pharm. Sci., 1977, 66: 1-19). Salts of the claimed acids may be also produced by reacting a purified acids specifically with a suitable base; in which case salts of metals and amines may be synthesized. Metal salts are salts of sodium, potassium, calcium, barium, zinc, magnesium, lithium, and aluminum, sodium and potassium salts being preferred. Suitable inorganic bases from which metal salts can be produced are sodium hydroxide, carbonate, bicarbonate and hydride; potassium hydroxide, carbonate and bicarbonate, lithium hydroxide, calcium hydroxide, magnesium hydroxide, and zinc hydroxide. Organic bases from which salts of the claimed acids can be obtained are amines and amino acids of sufficient basicity to produce a stable salt and suitable for use for medical purposes (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane, and the like. Besides, salts can be formed with the use of tetraalkylammonium hydroxides, such as choline, tetramethylammonium, tetraethylammonium, and the like. Amino acids may be selected from the main amino acids - lysine, ornithine, and agrinine.

**"Fragment"** (scaffold) means the structural formula of a molecule part characteristic of a group of compounds or a molecular framework typical of a group of compounds, or compounds in the "combinatorial library".

**"1,2-Ethylene radical"** means -CH₂-CH₂-group containing one or more "alkyl substituents" that may be identical or different, the meanings of which are defined in this section.

Object of the present invention is to provide new substituted indoles and a method for the production thereof.

The object is achieved by substituted indoles of the general formula **1** or racemates, optical isomers, pharmaceutically acceptable salts and/or hydrates thereof: 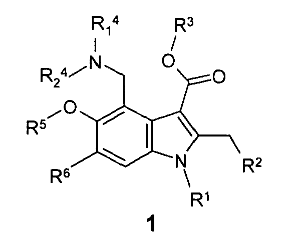 wherein:
R¹, R₁⁴ and R₂⁴ independently of each other represent an amino group substituent selected from hydrogen, optionally substituted alkyl, optionally substituted cycloalkyl, optionally substituted aryl or optionally substituted heterocyclyl, or R₁⁴ and R₂⁴ together with the nirogen atom to which they are attached via R₁⁴ and R₂⁴ form an optionally substituted azaheterocyclyl or guanidyl, R² is an alkyl substituent selected from the group consisting of hydrogen, optionally substituted mercapto group, optionally substituted amino group, optionally substituted hydroxyl group; R³ is a lower alkyl; R⁵ represents hydrogen, or R⁵ together with the oxygen atom to which is attached and R₂⁴ together with the nitrogen atom to which it is attached, via R⁵ and R₂⁴ form an oxazine ring; R⁶ is a cyclic system substituent selected from hydrogen, halogen, cyano group, optionally substituted aryl or optionally substituted heterocyclyl, with the exception of the compounds of the general formula **A.**

More preferred substituted indoles are esters of 5-hydroxy-1H-indole-3-carboxylic acids of general formula **1.1** and esters of 1,2,3,7-tetrahydro-pyrrolo[3,2-f][1,3]benzoxazine-5-carboxylic acids of general formula **1.2,** or racemates, optical isomers, pharmaceutically acceptable salts and/or hydrates thereof 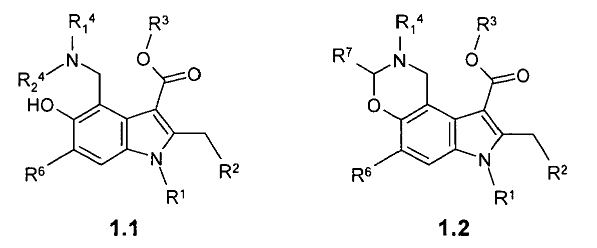 wherein:
R¹, R², R³, R₁⁴, R₂⁴, and R⁶ have the above meanings; R⁷ is a cyclic system substituent selected from hydrogen, optionally substituted alkyl, optionally substituted cycloalkyl, optionally substituted aryl or optionally substituted heterocyclyl.

More preferred substituted indoles are ethyl esters of 4-(aminomethyl)-6-aryl(or heterocyclyl)-5-hydroxy-1H-indole-3-carboxylic acids of general formula **1.1.1** or ethyl esters of 9-aryl(or heterocyclyl)-1,2,3,7-tetrahydropyrrolo[3,2-f][1,3]benzoxazine-5-carboxylic acids of general formula **1.2.1,** or racemates, optical isomers, pharmaceutically acceptable salts and/or hydrates thereof 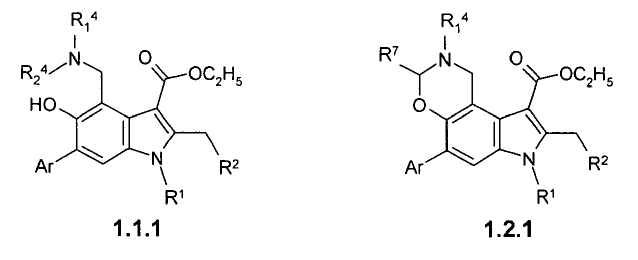 wherein:
R¹, R², R₁⁴, R₂⁴ and R⁷ have the above meanings; Ar is aryl or 5-6-membered heterocyclic structure containing at least one heteroatom selected from the group of N, O or S.

More preferred substituted indoles are ethyl esters of 4-(aminomethyl)-6-aryl(or pyridyl)-5-hydroxy-1-methylindole-3-carboxylic acids of general formula **1.1.2** or ethyl esters of 9-aryl(or pyridyl)-7-methyl-1,2,3,7-tetrahydropyrrolo[3,2-*f*][1,3]benzoxazine-5-carboxylic acids of general formula **1.2.2,** racemates, optical isomers, pharmaceutically acceptable salts and/or hydrates thereof 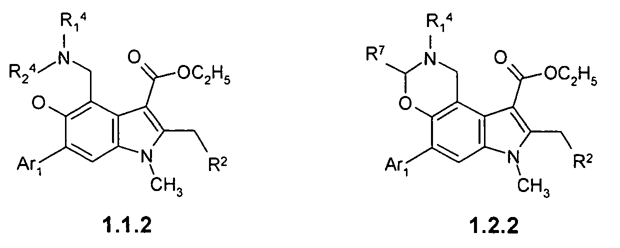 wherein:
R², R₁⁴, R₂⁴ and R⁷ have the above meanings; Ar₁ is aryl or pyridyl.

More preferred substituted indoles are ethyl esters of 6-aryl(or pyridyl)-2,4-bis(aminomethyl)-5-hydroxy-1-methylindole-3-carboxylic acids of general formula **1.1.3** and ethyl esters of 6-(aminomethyl)-9-aryl(or pyridyl)-7-methyl-1,2,3,7-tetrahydropyrrolo[3,2-*f*][1,3]benzoxazine-5-carboxylic acids of general formula **1.2.3** 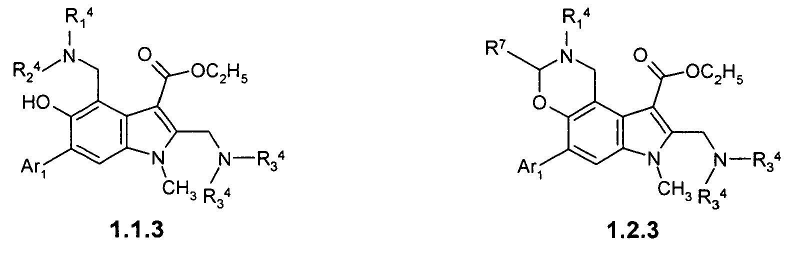 wherein:
R₁⁴, R₂⁴, R⁷ and Ar₁ have the above meanings; R₃⁴ and R₄⁴, independently from one another, are an amino group substituent selected from hydrogen, optionally substituted alkyl, optionally substituted cycloalkyl, optionally substituted aryl, or optionally substituted heterocyclyl, or R₃⁴ and R₄⁴, together with a nitrogen atom to which they are attached, form via R₃⁴ and R₄⁴ an optionally substituted aza-heterocyclyl or guanidyl.

More preferred substituted indoles are ethyl esters of 4-(aminomethyl)-6-aryl(or pyridyl)-2-(dimethylaminomethyl)-5-hydroxy-1-methylindole-3-carboxylic acids of general formula **1.1.4** and ethyl esters of 9-aryl(or pyridyl)- and 6-(dimethylaminomethyl)-7-methyl-1,2,3,7-tetrahydropyrrolo[3,2-*f*][1,3]benzoxazine-5-carboxylic acids of general formula **1.2.4** 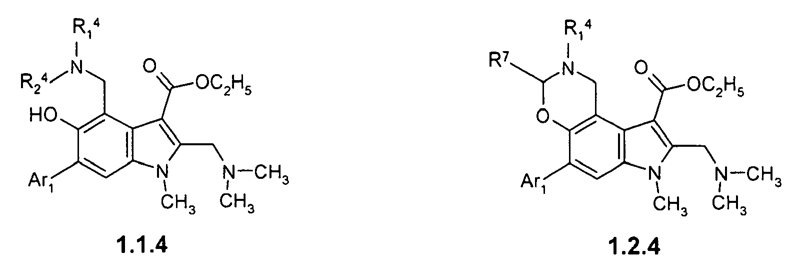 wherein:
R₁⁴, R₂⁴, R⁷ and Ar₁ have the above meanings.

More preferred substituted indoles are ethyl esters of 6-aryl(or pyridyl)-2,4-bis(dimethylaminomethyl)-5-hydroxy-1-methylindole-3-carboxylic acids of general formula 1.1.5 and ethyl esters of 9-aryl(or pyridyl)-3,7-dimethyl-6-(dimethylaminomethyl)-1,2,3,7-tetrahydropyrrolo[3,2-*f*][1,3]benzoxazine-5-carboxylates of general formula **1.2.5** 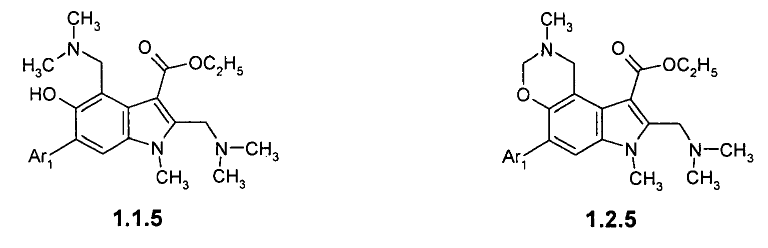 wherein:
R₁⁴, R₂⁴, and Ar₁ have the above meanings.

More preferred substituted indoles are ethyl ester of 2,4-bis(dimethylaminomethyl)-5-hydroxy-1-methyl-6-(pyridin-3-yl)-indole-3-carboxylic acid of formula **1.1.5(1)** and ethyl ester of 3,7-dimethyl-6-(dimethylaminomethyl)-9-(pyridin-3-yl)-1,2,3,7-tetrahydropyrrolo[3,2-*f*][1,3]benzoxazine-5-carboxylic acid of formula **1.2.5(1)**

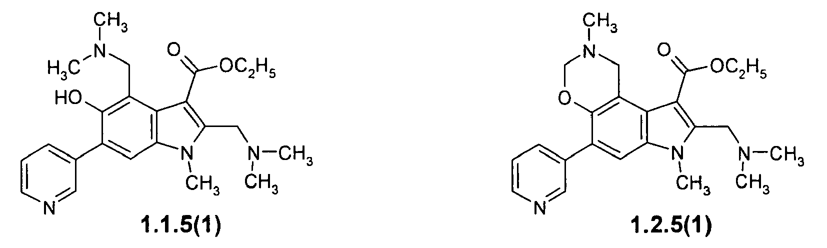

More preferred substituted indoles are ethyl ester of 2-(dimethylamino)methyl-5-hydroxy-1-methyl-6-(pyridin-3-yl)-4-(pyrrolidin-1-ylmethyl)-indole-3-carboxylic acid of the formula **1.1.5(2),** ethyl ester of 2-(dimethylamino)methyl-5-hydroxy-4-(1-imidazolylmethyl)-1-methyl-6-(pyridin-3-yl)-indole-3-carboxylic acid of the formula **1.1.5(3),** ethyl ester of 2-(dimethylamino)methyl-5-hydroxy-4-(guanidylmethyl)-1-methyl-6-(pyridin-3-yl)-indole-3-carboxylic acid of the formula **1.1.5(4),** ethyl ester of 4-(dimethylamino)methyl-5-hydroxy-1-methyl-6-(pyridin-3-yl)-2-(pyrrolidin-1-ylmethyl)-indole-3-carboxylic acid of the formula **1.1.5(5)**

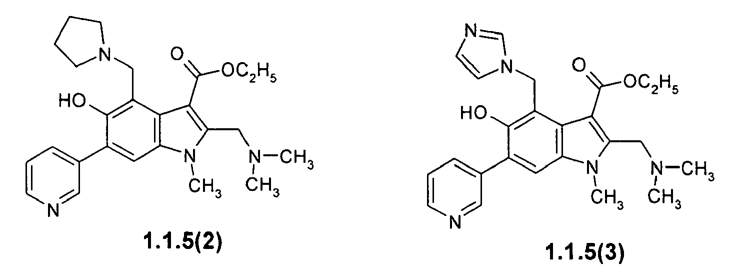

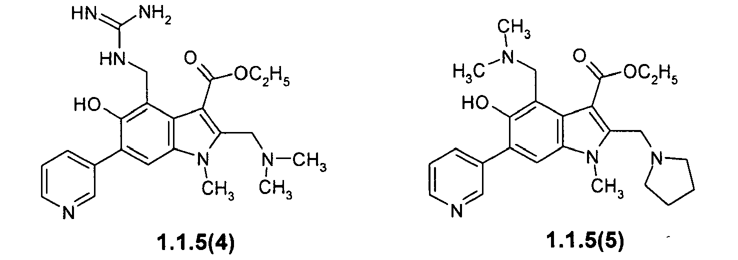

According to this invention, a method for producing of substituted 4-aminomethyl-5-hydroxy-1H-indole-3-carboxylic acids esters of general formula **1.1** comprises reacting substituted 5-hydroxy-1H-indole-3-carboxylic acids of general formula **2** with formaldehyde or paraform and amines of general formula **3** according to the following scheme 1. 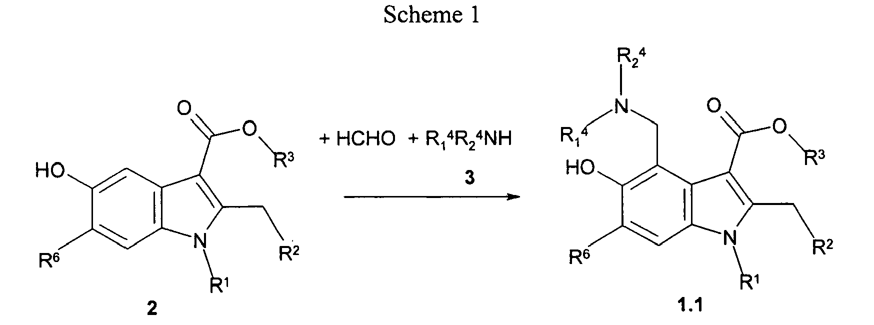 wherein:
R¹, R², R³, R₁⁴, R₂⁴ and R⁶ have the above meanings.

Subject matter of the present invention is also a method for producing esters of 1,2,3,7-tetrahydropyrrolo[3,2-f][1,3]benzoxazine-5-carboxylic acids of general formula **1.2** by reacting of esters of 5-hydroxy-1H-indole-3-carboxylic acids of general formula **2** with aldehydes of general formula **4** and primary amines of general formula **5** according to the following scheme 2. 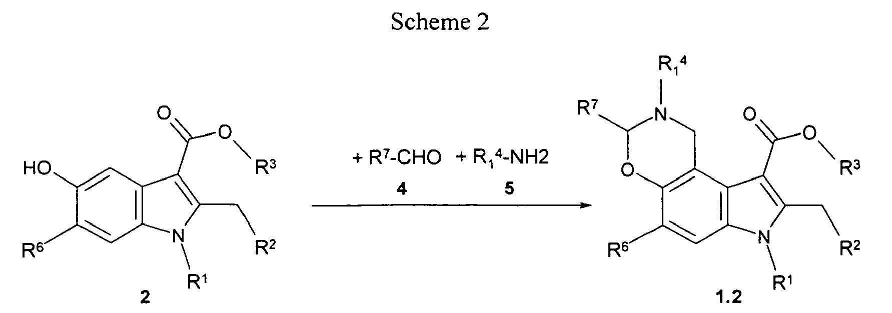 wherein: R¹, R², R³, R₁⁴, R⁶ and R⁷ have the above meanings.

Subject matter of the present invention is also a method for producing esters of 1,2,3,7-tetrahydropyrrolo[3,2-f][1,3]benzoxazine-5-carboxylic acids of general formula **1.2** by reacting esters of 4-(aminomethyl)-5-hydroxy-1H-indole-3-carboxylic acids of general formula **1.1,** where R₂⁴ = H, with aldehydes of general formula **4** according to the following scheme 3. 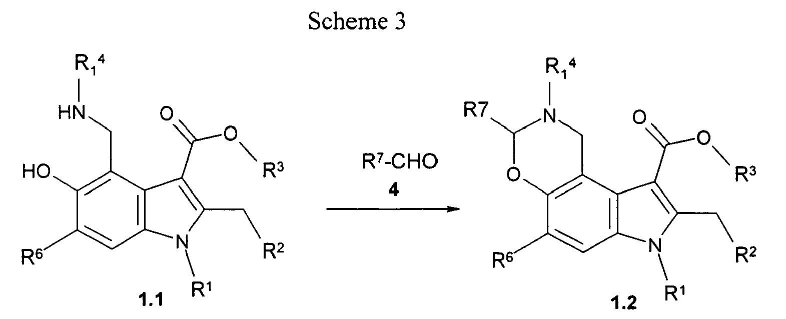 wherein: R¹, R², R³, R₁⁴, R⁶ and R⁷ have the above meanings.

Esters of substituted indoles of general formula **1** may form hydrates or pharmaceutically acceptable salts. Salts may be produced with the use of inorganic acids and organic acids, for example, hydrochloric acid, hydrobromic acid, hydroiodic acid, sulfuric acid, phosphoric acid, formic acid, acetic acid, propionic acid, trifluoro-acetic acid, maleic acid, tartaric acid, methanesulfonic acid, benzenesulfonic acid, and para-toluenesulfonic acid. Hydrates are commonly produced upon recrystallization of compounds of general formula **1** or their salts from water or water-containing solvents.

In accordance with this invention, substituted indoles of general formula **1** are used as active substances of pharmaceutical compositions exhibiting antiviral activity, including that in relation to infectious hepatitis (HCV and HBV), severe atypical respiratory syndrome (SARS), bird flue and human immunodeficiency virus (HIV).

This invention is further directed to pharmaceutical compositions having antiviral activity, particularly with respect to infectious hepatitis (HCV and HBV), severe atypical respiratory syndrome (SARS), bird flue and human immunodeficiency virus (HIV), and containing as an active substance at least one azaheterocyclic compound of general formula 1, or a racemate, optical isomer, pharmaceutically acceptable salt and/or hydrate thereof.

More preferred pharmaceutical compositions are compositions containing an active substance that is at least one ester of 5-hydroxy-1H-indole-3-carboxylic acid of general formula **1.1** or ester of 1,2,3,7-tetrahydro-pyrrolo[3,2-*f*][1,3]benzoxazine-5-carboxylic acid of general formula **1.2,** or a racemate, optical isomer, pharmaceutically acceptable salt and/or hydrate thereof.

More preferred pharmaceutical compositions are compositions containing an active substance that is at least one ethyl ester of 4-aminomethyl-6-aryl(or heterocyclyl)-5-hydroxy-1H-indole-3-carboxylic acid of general formula **1.1.1,** or ethyl ester of 9-aryl(or heterocyclyl)-1,2,3,7-tetrahydropyrrolo[3,2-*f*][1,3]benzoxazine-5-carboxylic acid of general formula **1.2.1** or a racemate, optical isomer, pharmaceutically acceptable salt and/or hydrate thereof.

More preferred pharmaceutical compositions are compositions containing an active substance that is at least one ethyl ester of 4-aminomethyl-6-aryl(or pyridyl)-5-hydroxy-1-methylindole-3-carboxylic acid of general formula **1.1.2** or ethyl ester of 9-aryl(or pyridyl)-7-methyl-1,2,3,7-tetrahydro-pyrrolo[3,2-*f*][1,3]benzoxazine-5-carboxylic acid of general formula **1.2.2,** or a racemate, optical isomer, pharmaceutically acceptable salt and/or hydrate thereof.

More preferred pharmaceutical compositions are compositions containing an active substance that is at least one ethyl ester of 6-aryl(or pyridyl)-2,4-bis(aminomethyl)-5-hydroxy-1-methylindole-3-carboxylic acid of general formula **1.1.3** and an ethyl ester 6-aminomethyl-9-aryl(or pyridyl)-7-methyl-1,2,3,7-tetrahydro-pyrrolo[3,2-*f*][1,3]benzoxazine-5-carboxylic acid of general formula **1.2.3,** or a racemate, optical isomer, pharmaceutically acceptable salt and/or hydrate thereof.

More preferred pharmaceutical compositions are compositions containing an active substance that is at least one ethyl ester of 4-aminomethyl-6-aryl(or pyridyl)-5-hydroxy-2-dimethylaminomethyl-1-methylindole-3-carboxylic acid of general formula **1.1.4** and an ethyl ester of 9-aryl(or pyridyl)-6-dimethylaminomethyl-7-methyl-1,2,3,7-tetrahydro-pyrrolo[3,2-*f*][1,3]benzoxazine-5-carboxylic acid of general formula **1.2.4,** or a racemate, optical isomer, pharmaceutically acceptable salt and/or hydrate thereof.

More preferred pharmaceutical compositions are compositions containing an active substance that is at least one ethyl ester of 6-aryl(or pyridyl)-2,4-bis(dimethylaminomethyl)-5-hydroxy-1-methylindole-3-carboxylic acid of general formula **1.1.5** and an ethyl ester of 9-aryl(or pyridyl)-3,7-dimethyl-6-dimethylaminomethyl-1,2,3,7-tetrahydro-pyrrolo[3,2-*f*][1,3]benzoxazine-5-carboxylic acid of general formula 1.2.5, or a racemate, optical isomer, pharmaceutically acceptable salt and/or hydrate thereof.

More preferred pharmaceutical compositions are compositions containing an active substance that is at least one of the ethyl esters of 2,4-bis(dimethylaminomethyl)-5-hydroxy-1-methyl-6-(pyridin-3-yl)-indole-3-carboxylic acid of formula **1.1.5(1);** 3,7-dimethyl-6-dimethylaminomethyl-9-(pyridin-3-yl)-1,2,3,7-tetrahydro-pyrrolo[3,2-*f*][1,3]benzoxazine-5-carboxylic acid of formula **1.2.5(1);** 2-dimethylaminomethyl-5-hydroxy-1-methyl-6-(pyridin-3-yl)-4-(pyrrolidin-1-ylmethyl)-indole-3-carboxylic acid of formula **1.1.5(2);** 2-dimethylaminomethyl-5-hydroxy-4-(imidazol-1-ylmethyl)-1-methyl-6-(pyridin-3-yl)-indole-3-carboxylic acid of formula **1.1.5(3);** 2-dimethylaminomethyl-5-hydroxy-4-guanidinylmethyl-1-methyl-6-(pyridin-3-yl)-indole-3-carboxylic acid of formula **1.1.5(4),** or 4-dimethylaminomethyl-5-hydroxy-1-methyl-6-(pyridin-3-yl)-2-(pyrrolidin-1-ylmethyl)-indole-3-carboxylic acid of general Formula **1.1.5(5),** or a racemate, optical isomer, pharmaceutically acceptable salt and/or hydrate thereof.

Pharmaceutical compositions may comprise pharmaceutically acceptable excipients. Pharmaceutically acceptable excipients are diluents, auxiliary agents, and/or carriers used in pharmaceutics. A pharmaceutical composition may, together with azaheterocyclic compounds of general formula **1** of the present invention, comprise other active substances, provided, however, that they do not cause undesirable effects, such as allergic reactions.

If necessary to use pharmaceutical compositions of the present invention in clinical practice, they may be mixed to prepare different forms, in which case they may comprise traditional pharmaceutical carriers; for example, peroral forms (such as tablets, gelatin capsules, pills, solutions or suspensions); injectable forms (such as injectable solutions or suspensions, a dry injectable powder that only requires injectable water to be added before use); and local forms (such as ointments or solutions).

The carriers used in pharmaceutical compositions of the present invention are those that are used in pharmaceutics to produce common forms, in particular, binding and wetting agents, disintegrators, solvents, diluents, stabilizers, suspending agents, colorless agents, and taste modifying agents are used in peroral forms; antiseptics, solubilizers, and stabilizers are used in injectable forms; and bases, diluents, lubricating agents, and antiseptics are used in local forms.

A further object of this invention is to provide a method for preparing pharmaceutical compositions.

This object is achieved by mixing an active substance with a neutral filler and/or solvent, the invention being distinguished in that the active substance is at least one azaheterocyclic compound of general formula **1,** or a racemate, optical isomer, pharmaceutically acceptable salt and/or hydrate thereof.

In accordance with this invention, pharmaceutical compositions containing as an active substance at least one azaheterocyclic compound of general formula **1,** or a racemate, optical isomer, pharmaceutically acceptable salt and/or hydrate thereof, are used to prepare medicaments in the form of tablets, capsules or injections placed in a pharmaceutically acceptable packing for preventing and treating various viral diseases, including diseases caused by viruses of infectious hepatitis (HCV and HBV), severe atypical respiratory syndrome (SARS), bird flu and human immunodeficiency virus (HIV).

The present invention is also directed to therapeutic kits for preventing and treating viral diseases, including diseases caused by viruses of infectious hepatitis (HCV and HBV), severe atypical respiratory syndrome (SARS), bird flu and human immunodeficiency virus (HIV), in which one of the components is a medicament prepared on the basis of a pharmaceutical composition containing as an active substance at least one azaheterocyclic compound of general formula **1,** or a racemate, optical isomer, pharmaceutically acceptable salt and/or hydrate thereof.

Therapeutic kits for preventing and treating hepatitis C (HCV) together with medicaments of this invention may comprise: inhibitors of dehydrogenase inosine-5-monophosphate, for example, Ribavirin (authorized) and Ribamidin; inhibitors of hepatitis C NS3 protease, for example, Telaprevir, Siluprevir, and SCH-503034; inhibitors of RNA polymerase NS5B, for example, XTL-2125; inhibitors of alpha-glucosidase, for example, amino-carbohydrate Selgosivir; and also antagonists of TLR receptors, hepatoprotectors, cyclosporines, various proteins (for example, interferons), antibodies, vaccines, etc.

Therapeutic kits for preventing and treating hepatitis B (HBV) together with medicaments of this invention may comprise: reverse transcriptase inhibitors, for example, Lamivudine and Tenofovir; inhibitors of DNA polymerase, for example, Telbivudine, Entecavir, and Adefovir dipivoxil; immuno-stimulants, for example, Serocion; and also angiogenesis inhibitors, hepatoprotectors, various proteins (for example, interferons), antibodies, vaccines, etc.

According to this invention, the method for preventing and treating viral diseases of animals and humans, including those caused by viruses of infectious hepatitis (HCV and HBV), human immunodeficiency virus (HIV), bird flu, and severe atypical respiratory syndrome (SARS), comprises administering to a warm-blooded animal or human medications in the form of tablets, capsules or injections containing an active substance that is at least one azaheterocyclic compound of general formula **1,** or a racemate, optical isomer, pharmaceutically acceptable salt and/or hydrate thereof, or therapeutic kits comprising said medicaments.

Medicaments may be administered perorally or parenterally (for example, intravenously, subcutaneously, intraperitoneally, or locally). Clinical doses of the active substance of an azaheterocyclic compound of general formula **1** administered to patients may be adjusted depending on: the therapeutic efficiency and bio-accessibility of active ingredients in the organism, the speed of their exchange and removal from the organism, and also the age, gender, and disease stage of the patient, the daily intake for adults normally being 10∼500 mg, preferably 50∼300 mg. Accordingly, the above effective doses are to be taken into consideration while preparing a medicament of the present invention from the pharmaceutical composition in the form of dose units, each dose unit of the preparation containing 10∼500 mg of the azaheterocyclic compound of general formula **1,** preferably 50∼300 mg. Following the instructions of a doctor or pharmacist, these preparations may be taken several times over specified periods of time (preferably, from one to six times).

### Best Embodiment of the invention

Following below are specific examples that illustrate this invention, but do not limit it thereto.

The invention is illustrated in the following drawings:
FIG. 1. Western analysis of the efficiency of Arbidol (prior art);
FIG. 2. Western analysis of the efficiency of substances **1.1(15)** and **1.2.5(1c);**
FIG. 3. Western analysis of the efficiency of substances **1.1.2(42)** and **1.1(20);**
FIG. 4. Western analysis of the efficiency of substances **1.2.2(14)** and **1.1.2(35);**
FIG. 5. Western analysis of the efficiency of substances **1.1.3(7)** and **1.2(29);**
FIG. 6. Western analysis of the efficiency of substances **1.1.5(5c)** and **1.1.5(2c);**
FIG. 7. Concentration ratios of inhibition of virus proteins (NS3+NS5a) by selected substances of general formula **1.**
FIG. 8. Concentration ratios of cytotoxicity of selected substances of general formula **1.**

### Example 1. Preparation of a combinatorial library of substituted 2-aminomethyl-5-hydroxy-1H-indole-3-carboxylic acids esters of general formula 1.1.

A mixture of 0.357 mmol of ester **2,** 0.43 mmol of a secondary amine **3,** and 0.43 mmol of formaldehyde in the form of formalin in 3 ml of dioxane is heated at 70-80°C at stirring for 12 to 48 hours. Progress of the reaction is monitored by chromato-mass-spectrometry. Upon completion of the reaction, the reaction mass is diluted with water, the residue is filtered and recrystallized from a suitable solvent, or purified by chromatography. Parallel synthesis results in a combinatorial library of substituted 1H-indole-3-carboxylic acids esters of general formula **1.1,** including:
ethyl 6-bromo-4-((dimethylamino)methyl)-5-hydroxy-2-(hydroxymethyl)-1-methyl-1H-indole-3-carboxylate hydrochloride **1.1(1),** LC-MS, *m*/*z* 386 (M+H), ¹H NMR (DMSO-D₆, 300 MHz): 9.37 (br, 1H), 9.10 (br, 1H), 8.03 (s, 1H), 8.66 (s, 1H), 5.48 (s, 1H), 4.94 (s, 2H), 4.90 (s, 2H), 4.33 (q, *J*=5.3Hz, 2H), 3.83 (s, 3H), 2.75 (s, 6H), 1.36 (t, *J*=5.3Hz, 3H); ethyl 4-((dimethylamino)methyl)-5-hydroxy-1-methyl-2-(phenylthiomethyl)-6-(pyrimidin-5-yl)-1H-indole-3-carboxylate **1.1.1(7),** LCMS, *m*/*z* 477 (M+H); ethyl 4-((dimethylamino)methyl)-5-hydroxy-1-methyl-2-(phenylthiomethyl)-6-(pyridin-3-yl)-1H-indole-3-carboxylate **1.1.2(5),** LCMS, *m*/*z* 476 (M+H); ethyl 4-((dimethylamino)methyl)-5-hydroxy-1-methyl-2-(phenylthiomethyl)-6-(pyridin-4-yl)-1H-indole-3-carboxylate hydrochloride **1.1.2(6),** LCMS, *m*/ 476 (M+H); ethyl 5-hydroxy-4-((3-(hydroxymethyl)piperidin-1-yl)methyl)-1-methyl-2-(phenylthiomethyl)-6-(pyridin-3-yl)-1H-indole-3-carboxylate **1.1.2(23),** LC-MS, *mlz* 546 (M+H); ethyl 5-hydroxy-4-((methylamino)methyl)-1-methyl-2-(phenylsulfonylmethyl)-6-(pyridin-3-yl)-1H-indole-3-carboxylate hydrochloride **1.1.2(29),** LC-MS, *m*/*z* 494 (M+H), NMR-¹H (DMSO): 9.53 (br.s, 1H), 9.11 (s, 1H); 8.88 (br.m, 2H); 8.85 (d, J=5.9 Hz, 1H), 8.77 (d, J=8.1 Hz, 1H), 8.09 (m, 1H), 7.95 (s, 1H), 7.72-7.79 (m, 3H), 7.611-7.65 (m, 2H), 5.41 (s, 2H), 4.61 (t, J=5.9 Hz, 2H), 4.11 (q, J=7.0 Hz, 2H), 3.73 (s, 3H); 2.59 (t, J=5.1 Hz, 3H), 1.28 (t, J=7.0 Hz, 3H); ethyl 5-hydroxy-4-((dimethylamino)methyl)-1-methyl-2-(phenylsulfonylmethyl)-6-(pyridin-3-yl)-1H-indole-3-carboxylate hydrochloride **1.1.2(31),** LC-MS, *m*/*z* 508 (M+H), ¹H NMR (DMSO-D₆): 9.57 (br, 1H), 9.30 (br, 1H), 9.17 (s, 1H), 8.88 (d, *J*=3.9 Hz, 1H), 8.76 (d, *J*=5.7Hz, 1H), 8.10 (m, 1H), 7.96 (s, 1H), 7.75-7.82 (m, 3H), 7.64-7.67 (m, 2H), 5.45 (s, 2H), 4.96 (s, 2H), 4.16 (q, *J*=5.1Hz, 2H), 3.78 (s, 3H), 2.79 (s, 6H), 1.32 (t, *J*=5.1Hz, 3H); ethyl 5-hydroxy-2-(hydroxymethyl)-1-methyl-4-((dimethylamino)methyl)-6-(pyridin-3-yl)-1H-indole-3-carboxylate hydrochloride 1.1.2(37), LCMS, *m*/*z* 370 (M+H), NMR-¹H (DMSO): 9.35 (br.s, 1H), 9.10 (s,1H); 8.84 (d, J=5.1 Hz, 1H), 8.78 (br.m, 2H); 8.73 (d, J=8.4 Hz, 1H), 8.08 (m, 1H), 7.90 (s, 1H), 4.97 (s, 2H), 4.63 (br.t, 2H), 4.33 (q, J=7.0 Hz, 2H), 3.88 (s, 3H); 2.61 (br.t, 3H), 1.36 (t, J=7.0 Hz, 3H); ethyl 5-hydroxy-1-methyl-4-((methylamino)methyl)-6-(pyridin-3-yl)-2-(pyrrolidin-1-ylmethyl)-1H-indole-3-carboxylate hydrochloride **1.1.3(16),** LCMS, *m*/*z* 423 (M+H), NMR-¹H (DMSO): 11.24 (br.m, 1H), 9.57 (br.s, 1H), 9.13 (d, J=1.8 Hz, 1H); 8.96 (br.m, 2H); 8.87 (d, J=5.5 Hz, 1H), 8.81 (m, 1H), 8.14 (m, 1H), 8.04 (s, 1H), 4.95 (d, J=4.8 Hz, 2H), 4.71 (t, J=5.1 Hz, 2H), 4.41 (q, J=7.0 Hz, 2H), 4/07 (s, 3H); 3.46 (m, 2H), 3.24 (m, 2H), 2.60 (t, J=5.1 Hz, 3H), 2.02 (m, 2H), 1.96 (m, 2H), 1.39 (t, J=7.0 Hz, 3H); ethyl 5-hydroxy-1-methyl-4-((methylamino)methyl)-2-(morpholin-4-ylmethyl)-6-(pyridin-3-yl)-1H-indole-3-carboxylate hydrochloride **1.1.3(18),** LC-MS, *m*/*z* 439 (M+H), NMR-¹H (DMSO): 11. 46 (br.m, 1H); 9.57 (br.s, 1H), 9.12 (d, J=1.5 Hz, 1H); 8.96 (br.m, 2H),8.87 (d, J=5.5 Hz, 1H), 8. 08 (m, 1H), 8.13 (m, 1H), 8.04 (s, 1H), 4.91 (br.s, 2H), 4.71 (t, J=5.5 Hz, 2H), 4.42 (q, J=7.0 Hz, 2H), 4.07 (s, 3H); 3.85-4.05 (m, 4H), 3.27-3.40 (m, 4H); 2.60 (t, J=5.1 Hz, 3H), 1.40 (t, J=7.0 Hz, 3H); ethyl 2-((dimethylamino)methyl)-5-hydroxy-1-methyl-4-((methylamino)methyl)-6-(pyridin-3-yl)-1H-indole-3-carboxylate hydrochloride **1.1.4(2),** LC-MS, *m*/*z* 397 (M+H), NMR-1H (DMSO): 10.84 (br.s, 1H), 9.57 (br.s, 1H), 9.12 (d, J=1.8 Hz, 1H); 8.96 (br.m, 2H); 8.87 (d, J=5.5 Hz, 1H), 8.78 (m, 1H), 8.13 (m, 1H), 8.04 (s, 1H), 4.89 (br.s, 2H), 4.72 (dr.t, J=5.1 Hz, 2H), 4.41 (q, J=7.0 Hz, 2H), 3.73 (s, 3H); 2.81 (br.s, 6H); 2.60 (t, J=5.5 Hz, 3H), 1.41 (t, J=7.0 Hz, 3H); ethyl 2-((dimethylamino)methyl)-5-hydroxy-1-methyl-6-(pyridin-3-yl)-4-(pyrrolidin-1-ylmethyl)-1H-indole-3-carboxylate hydrochloride **1.1.5(2c),** LC-MS, *m*/*z* 437 (M+H), NMR-¹H (DMSO): 10.72 (br.s (m?), 1H), 9.84 (br.m, 1H), 9.67 (br.s (m?), 1H); 9.18 (d, J=1.8 Hz, 1H); 8.88 (d, J=5.5 Hz, 1H), 8.82 (m, 1H), 8.14 (m, 1H), 8.03 (s, 1H), 5.13 (d, J=5.1 Hz, 2H), 4.89 (d, J=4.8 Hz, 2H), 4.41 (q, J=7.0 Hz, 2H), 4.04 (s, 3H); 3.39 (m, 2H), 3.21 (m, 2H), 2.82 (d, J=4.0 Hz, 6H), 2.0 (m, 2H), 1.88 (m, 2H), 1.41 (t, J=7.0 Hz, 3H); ethyl 4-((dimethylamino)methyl)-5-hydroxy-1-methyl-6-(pyridin-3-yl)-2-(pyrrolidin-1-ylmethyl)-1H-indole-3-carboxylate hydrochloride **1.1.5(5c),** LC-MS, *m*/*z* 437 (M+H), NMR-¹H (DMSO): 11.06 (br.m, 1H); 9.63 (br.m, 1H), 9.40 (br.s, 1H), 9.16 (s, 1H); 8.87 (d, J=5.5 Hz, 1H), 8.78 (d, J=8.1 Hz, 1H), 8.12 (m, 1H), 8.03 (s, 1H), 5.04 (d, J=4.0 Hz, 2H), 4.95 (d, J=4.4 Hz, 2H), 4.41 (q, J=7.0 Hz, 2H), 4.05 (s, 3H); 3.47 (m, 2H), 3.25 (m, 2H); 2.76 (d, J=4.0 Hz, 6H), 1.9-2.1 (m, 4H); 1.40 (t, J=7.0 Hz, 3H) and other 1H-indole-3-carboxylates of the general formula **1.1,** some of them are presented in Table 1.

### Example 2. Preparation of a combinatorial library of esters of 1,2,3,7-tetrahydro-pyrrolo[3,2-f][1,3]benzoxazine-5-carboxylic acids of general formula 1.2.

A. A mixture of 0.357 mmol of ester **2,** 0.89 mmol of aldehyde **4,** and 0.43 mmol of primary amine 5 in 3 ml of dioxane is heated at 70-80°C at stirring for 12 to 48 hours. Progress of the reaction is monitored by chromato-mass-spectrometry. Upon completion of the reaction, the reaction mass is diluted with water, the residue is filtered and recrystallized from a suitable solvent, or purified by chromatography. Parallel synthesis results in a combinatorial library of substituted pyrrolo[3,2-*f*][1,3]benzoxazines **1.2,** including:
ethyl 3,7-dimethyl-9-(pyridin-3-yl)-6-(phenylsulfonylmethyl)-1,2,3,7-tetrahydropyrrolo[3,2-f][1,3]benzoxazine-5-carboxylate methyl sulfonate **1.2.2(14),** LCMS, *m*/*z* 506 (M+H), ¹H NMR (DMSO-D₆): 9.16 (s, 1H), 8.89 (d, *J*=5.7Hz, 1H), 8.75 (d, *J*=8.1Hz, 1H), 8.08 (m, 1H), 7.94 (s, 1H), 7.62-7.83 (m, 5H), 5.45 (s, 2H), 5.26 (s, 2H), 4.83 (s, 2H), 4.10 (q, *J*=7.1Hz, 2H), 3.82 (s, 3H), 2.97 (s, 3H), 2.36 (s, 6H), 1.30 (t, *J*=7.1Hz, 3H), ethyl 3,7-dimethyl-9-(pyridin-3-yl)-6-(hydroxymethyl)-1,2,3,7-tetrahydropyrrolo[3,2-f][1,3]benzoxazine-5-carboxylate methyl sulfonate **1.2.2(16),** LCMS, *m*/*z* 382 (M+H), ¹H NMR (DMSO-D₆): 9.12 (s, 1H), 8.85 (d, *J*=4.5Hz, 1H), 8.66 (d, *J*=7.8Hz, 1H), 8.03 (dd, *J₁*=7.5Hz, *J₂*=6.0Hz, 1H), 7.89 (s, 1H), 5.25 (s, 2H), 4.98 (s, 2H), 4.87 (s, 2H), 4.34 (q, *J*=7.0Hz, 2H), 3.91 (s, 3H), 2.97 (s, 3H), 2.55 (t, *J*=5.4Hz, 1H), 2.34 (s, 6H), 1.38 (t, *J*=7.0Hz, 3H); ethyl 3,7-dimethyl-9-(pyridine-3-yl)-6-(pyrrolidin-1-ylmethyl)-1,2,3,7-tetrahydropyrrolo[3,2-f][1,3]benzoxazine-5-carboxylate hydrochloride **1.2.3(4),** LCMS, *m*/*z* 435 (M+H), NMR ¹H (DMSO): 8.72 (s, 1H), 8.46 (d, J=4.8 Hz, 1H), 7.92 (d, J=7.7 Hz, 1H), 7.40 (m, 1H), 7.38 (s, 1H), 4.71 (s, 2H), 4.23 (q, J=7.5 Hz, 2H), 4.14 (s, 2H), 3.97 (s, 2H), 3.76 (s, 3H), 2.44 (m, 4H), 2.42 (s, 3H), 1.62 (m, 4H), 1.29 (t, J=7.5 Hz, 3H); ethyl 3,7-dimethyl-6-((dimethylamino)methyl)-9-(pyridin-3-yl)-1,2,3,7-tetrahydropyrrolo[3,2-f][1,3]benzoxazine-5-carboxylate hydrochloride **1.2.5(1c),** LCMS, *m*/*z* 409 (M+H), NMR ¹H (DMSO): 10.41 (br. s, 1H), 9.06 (d, J=1.5 Hz, 1H), 8.80 (d, J=4.4 Hz, 1H), 8.56 (d, J=7.3 Hz, 1H), 7.96 (s, 1H), 7.95 (m, 1H), 5.21 (s, 2H), 4.82 (s, 4H), 4.39 (q, J=7.5 Hz, 2H), 4.02 (s, 3H), 2.88 (s, 3H), 2.82 (s, 6H), 1.41 (t, J=7.5 Hz, 3H); ethyl 7-benzyl-9-bromo-3-methyl-6-((1-methylpiperazin-4-yl)methyl)-1,2,3,7-tetrahydropyrrolo[3,2-f][1,3]benzoxazine-5-carboxylate **1.2(6),** LCMS, *m*/*z* 542 (M+H); ethyl 9-bromo-6-((dimethylamino)methyl)-3-methyl-7-phenyl-1,2,3,7-tetrahydropyrrolo[3,2-f][1,3]benzoxazine-5-carboxylate **1.2(21),** LC-MS, *m*/*z* 473 (M+H); ethyl 9-bromo-3,7-dimethyl-6-((pyridin-4-yl)methyl)-1,2,3,7-tetrahydropyrrolo[3,2-f][1,3]benzoxazine-5-carboxylate **1.2(18),** LCMS, *m*/*z* 460 (M+H); ethyl 3,7-dimethyl-6-(phenylsulfonylmethyl)-9-cyano-1,2,3,7-tetrahydropyrrolo[3,2-f][1,3]benzoxazine-5-carboxylate **1.2(27),** LCMS, *m*/*z* 422 (M+H); ethyl 9-bromo-3,7-dimethyl-6-((phenylamino)methyl)-1,2,3,7-tetrahydropyrrolo[3,2-f][1,3]benzoxazine-5-carboxylate **1.2(23),** LCMS, *m*/*z* 459 (M+H); ethyl 3,7-dimethyl-9-(pyridin-4-yl)-6-(phenylsulfonylmethyl)-1,2,3,7-tetrahydropyrrolo[3,2-f][1,3]benzoxazine-5-carboxylate **1.2(5),** LCMS, *m*/*z* 513 (M+H) and other pyrrolo[3,2-f][1,3]benzoxazines **1.2,** some of them are presented in Table 1.

**B.** A mixture of 0.357 mmol of the corresponding ester **1.1** and 0.43 mmol of aldehyde **4** in 3 ml of dioxane is heated at 70-80°C at stirring for 3 to 48 hours. Progress of the reaction is monitored by chromato-mass-spectrometry. Upon completion of the reaction, the reaction mass is diluted with water, the residue is filtered and recrystallized from a suitable solvent, or purified by chromatography. Parallel synthesis results in a combinatorial library of substituted pyrrolo[3,2-*f*][1,3]benzoxazines **1.2,** including: ethyl 9-bromo-2-butyl-3,7-dimethyl-6-((phenylamino)methyl)-1,2,3,7-tetrahydropyrrolo[3,2-f][1,3]benzoxazine-5-carboxylate **1.2(24),** LCMS, *m*/*z* 515 (M+H); ethyl 9-bromo-3,7-dimethyl-2-phenyl-6-((phenylamino)methyl)-1,2,3,7-tetrahydropyrrolo[3,2-f][1,3]benzoxazine-5-carboxylate **1.2(25),** LCMS, *m*/ 535 (M+H).

**Table 1. Substituted indoles of general formula 1**

| **Nº** | **Formula** | **MW** | **LCMS (M+1)** |
|---|---|---|---|
| **1.1(1)** | 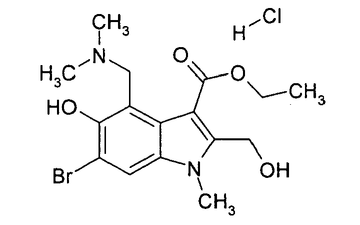 | 421,72 | 386 |
| **1.1(2)** | 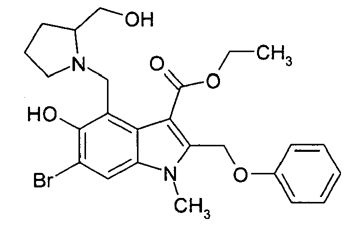 | 517,42 | 518 |
| **1.1(3)** | 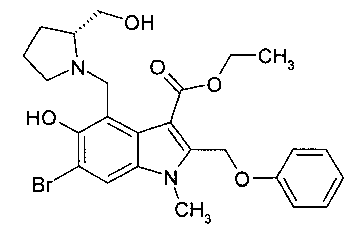 | 517,42 | 518 |
| Table 1. | | | |
| **1.1(4)** | 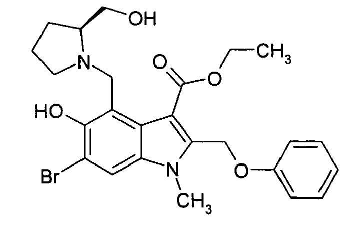 | 517,42 | 518 |
| **1.1(5)** | 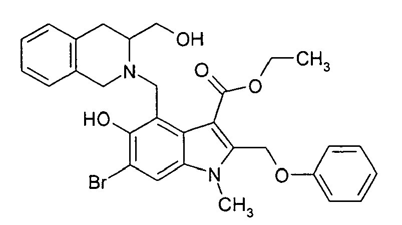 | 579,50 | 580 |
| **1.1(6)** | 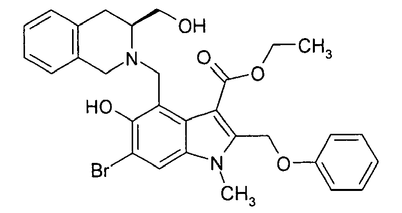 | 579,50 | 580 |
| **1.1(7)** | 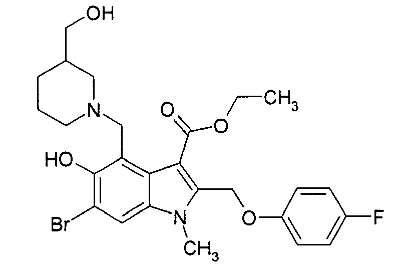 | 549,44 | 550 |
| **1.1(8)** | 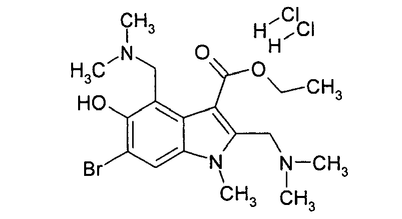 | 485,25 | 413 |
| **1.1(9)** | 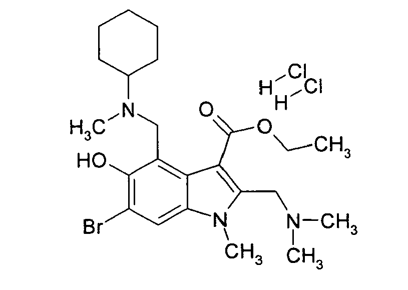 | 553,37 | 481 |
| Table 1. | | | |
| **1.1(10)** | 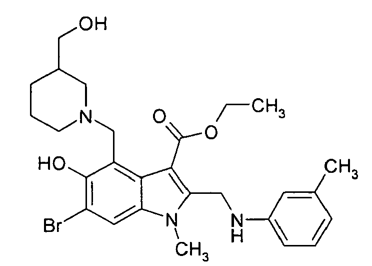 | 544,49 | 545 |
| **1.1(11)** | 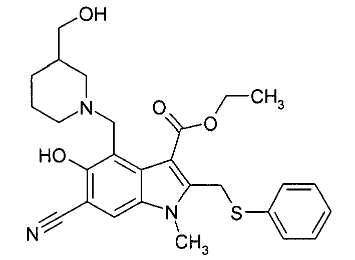 | 493,63 | 494 |
| **1.1(12)** | 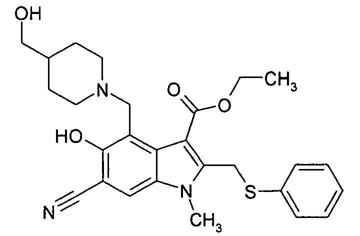 | 493,63 | 494 |
| **1.1(13)** | 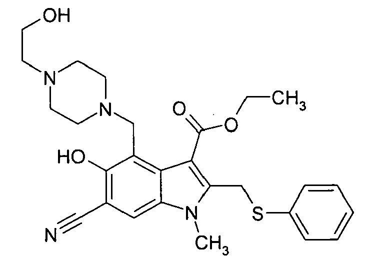 | 508,64 | 509 |
| **1.1(14)** | 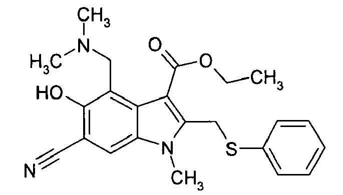 | 423,54 | 424 |
| **1.1(15)** | 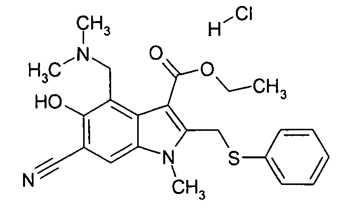 | 460,00 | 424 |
| Table 1. | | | |
| **1.1(16)** | 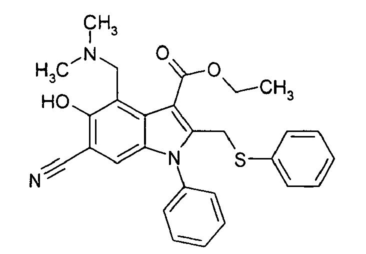 | 485,61 | 486 |
| **1.1(17)** | 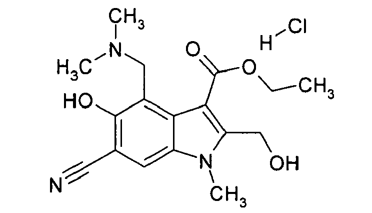 | 367,84 | 3321 |
| **1.1(18)** | 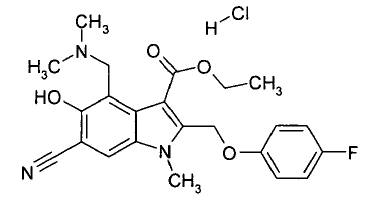 | 461,9 | 426 |
| **1.1(19)** | 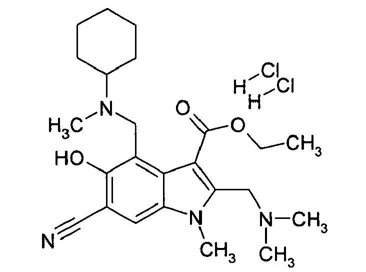 | 499.48 | 427 |
| **1.1(20)** | 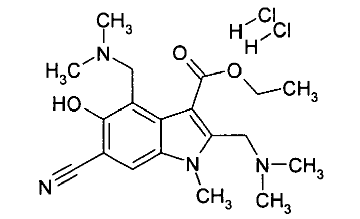 | 431,37 | 359 |
| **1.2(1)** | 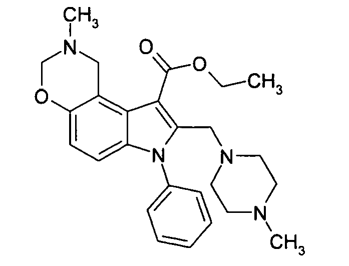 | 448,57 | 449 |
| Table 1. | | | |
| **1.2(2)** | 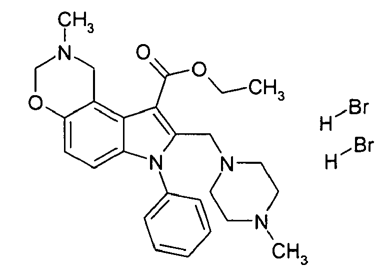 | 610,39 | 449 |
| **1.2(3)** | 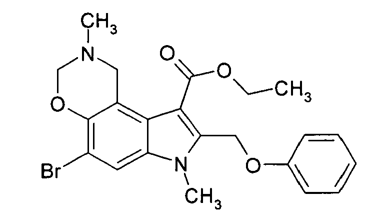 | 459.34 | 460 |
| **1.2(4)** | 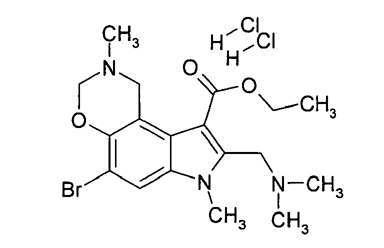 | 483.23 | 411 |
| **1.2(5)** | 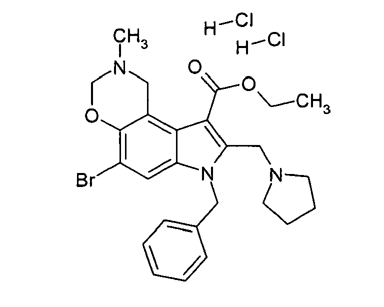 | 585,37 | 513 |
| **1.2(6)** | 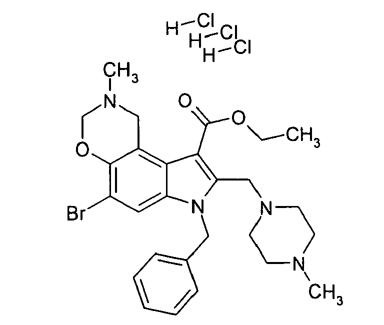 | 650.87 | 542 |
| Table 1. | | | |
| **1.2(7)** | 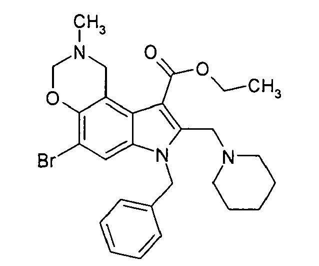 | 526,48 | 527 |
| **1.2(8)** | 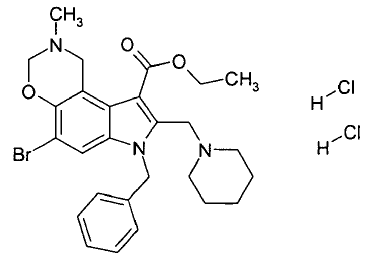 | 599,40 | 527 |
| **1.2(9)** | 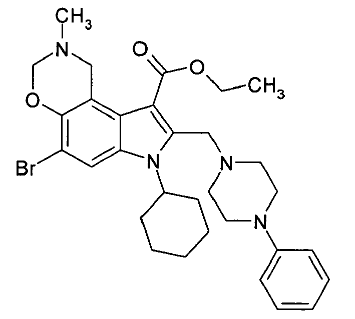 | 595,59 | 596 |
| **1.2(10)** | 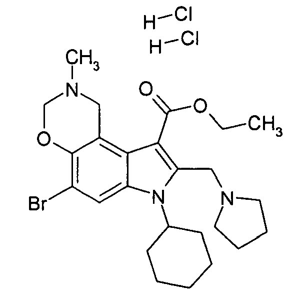 | 577.39 | 505 |
| **1.2(11)** | 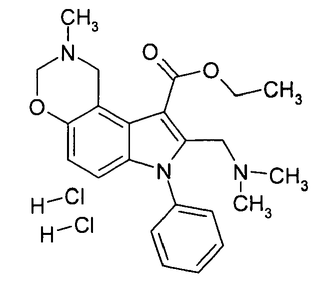 | 466,41 | 394 |
| Table 1. | | | |
| **1.2(12)** | 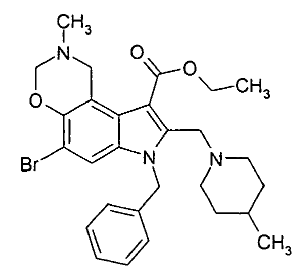 | 540,51 | 541 |
| **1.2(13)** | 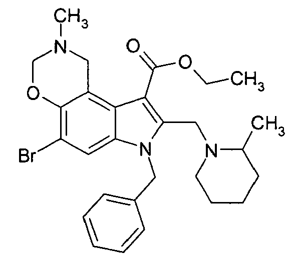 | 540,51 | 541 |
| **1.2(14)** | 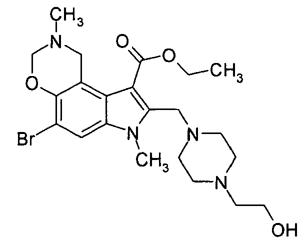 | 495,42 | 496 |
| **1.2(15)** | 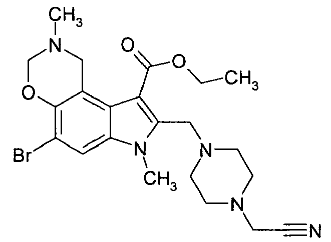 | 490,40 | 491 |
| **1.2(16)** | 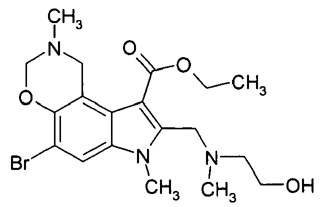 | 440,34 | 441 |
| **1.2(17)** | 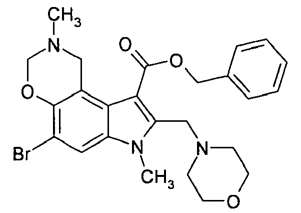 | 514,42 | 515 |
| Table 1. | | | |
| **1.2(18)** | 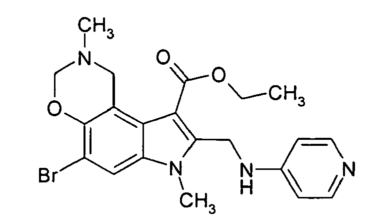 | 459,35 | 460 |
| **1.2(19)** | 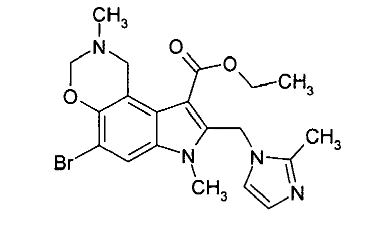 | 447,34 | 448 |
| **1.2(20)** | 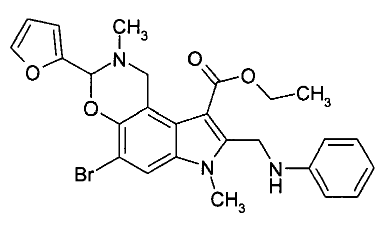 | 524,42 | 525 |
| **1.2(21)** | 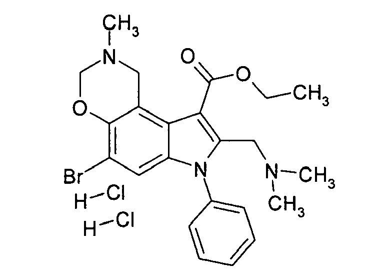 | 545.31 | 473 |
| **1.2(22)** | 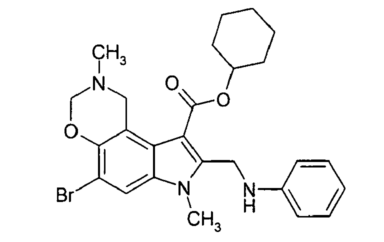 | 512,45 | 513 |
| **1.2(23)** | 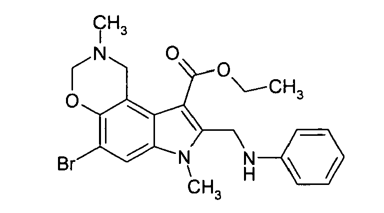 | 458,36 | 459 |
| Table 1. | | | |
| **1.2(24)** | 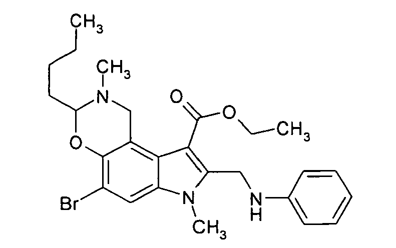 | 514,47 | 515 |
| **1.2(25)** | 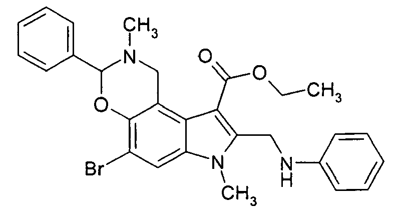 | 534,46 | 535 |
| **1.2(26)** | 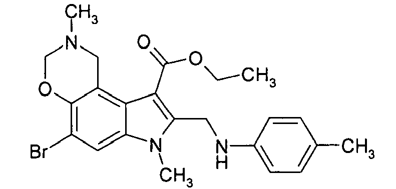 | 472,39 | 473 |
| **1.2(27)** | 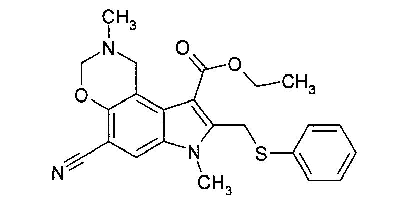 | 421,52 | 422 |
| **1.2(28)** | 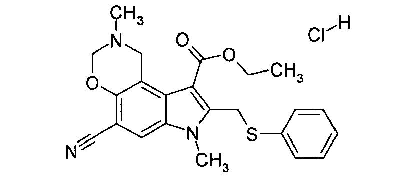 | 457,98 | 422 |
| **1.2(29)** | 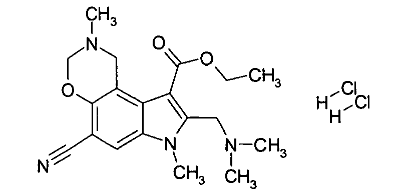 | 429.35 | 357 |
| **1.2(30)** | 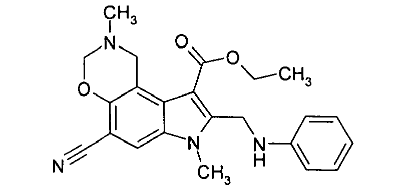 | 404,47 | 405 |
| Table 1. | | | |
| **1.2(31)** | 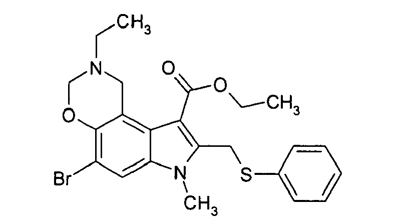 | 489,44 | 490 |
| **1.2(32)** | 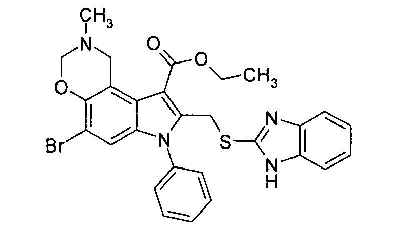 | 577,50 | 578 |
| **1.2(33)** | 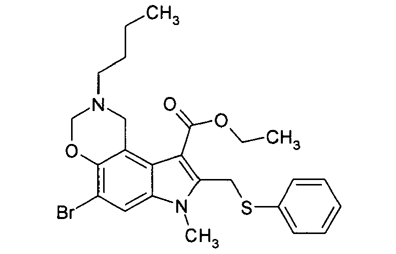 | 517,49 | 518 |
| **1.2(34)** | 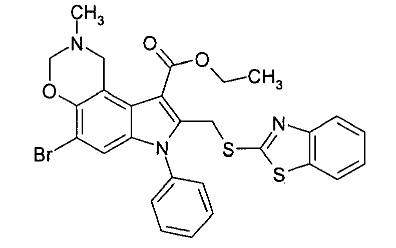 | 594,55 | 595 |
| **1.2(35)** | 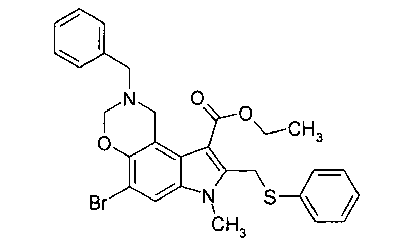 | 551,51 | 552 |
| **1.2(36)** | 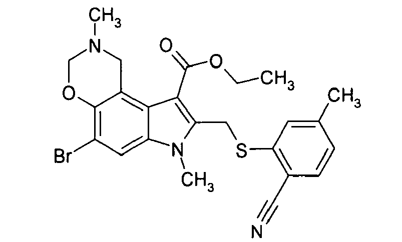 | 514,45 | 515 |
| Table 1. | | | |
| **1.2(37)** | 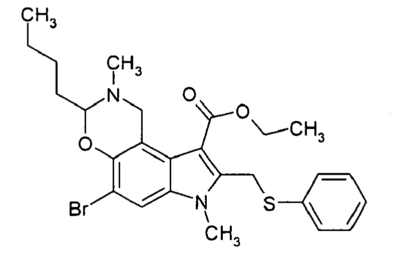 | 531,52 | 532 |
| **1.2(38)** | 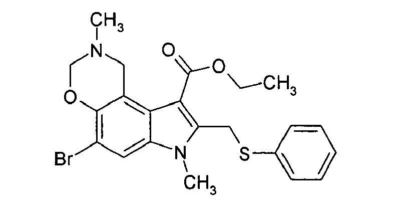 | 475,41 | 476 |
| **1.2(39)** | 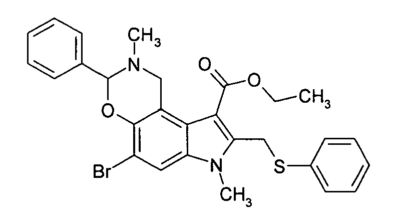 | 551,51 | 552 |
| **1.2(40)** | 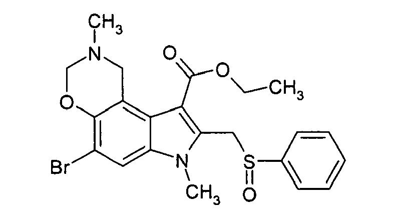 | 491,41 | 492 |
| **1.2(41)** | 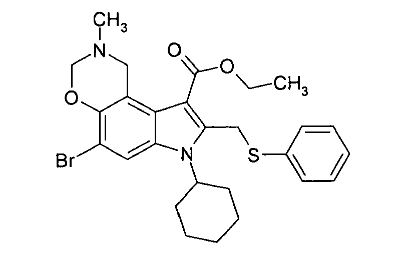 | 543,53 | 544 |
| **1.2(42)** | 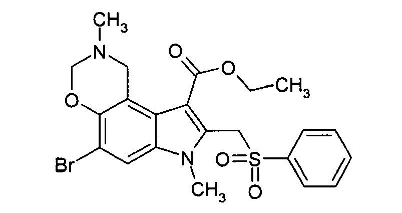 | 507,41 | 508 |
| Table 1. | | | |
| **1.2(43)** | 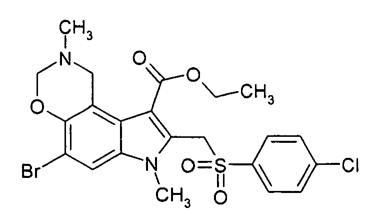 | 541,85 | 542 |
| **1.2(44)** | 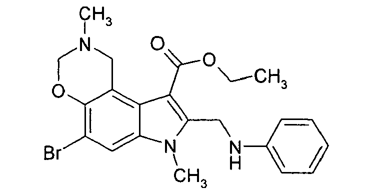 | 458,36 | 459 |
| **1.2(45)** | 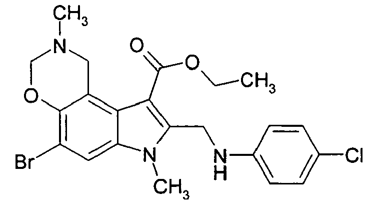 | 492,80 | 493 |
| **1.2(46)** | 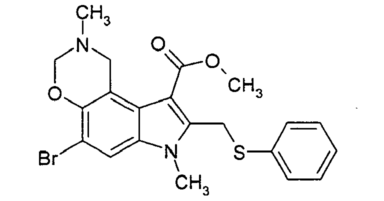 | 461,38 | 462 |
| **1.2(47)** | 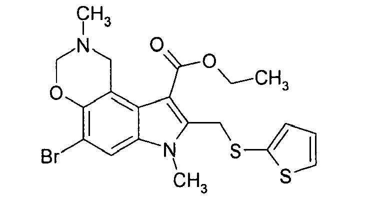 | 481,43 | 482 |
| **1.2(48)** | 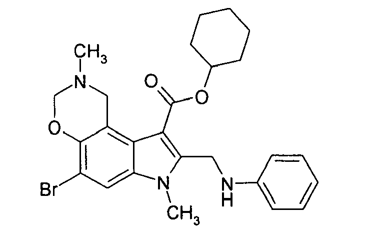 | 512,45 | 513 |
| **1.2(49)** | 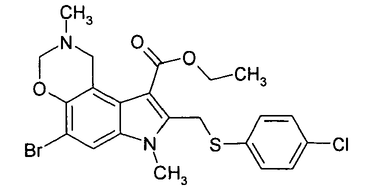 | 509,85 | 510 |
| Table 1. | | | |
| **1.2(50)** | 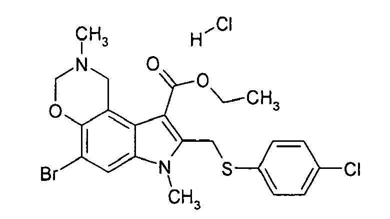 | 546,31 | 510 |
| **1.1.1(1)** | 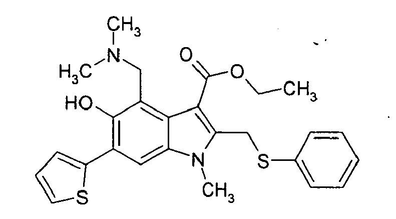 | 480,65 | 481 |
| **1.1.1(2)** | 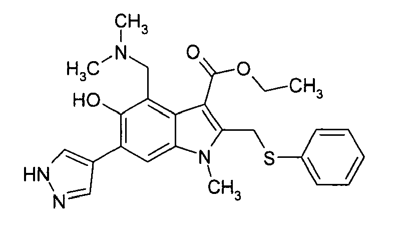 | 464,59 | 465 |
| **1.1.1(3)** | 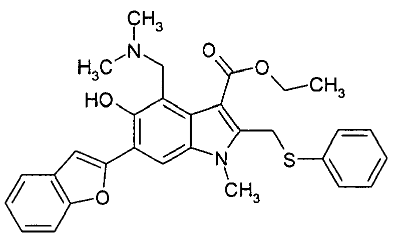 | 514,65 | 515 |
| **1.1.1(4)** | 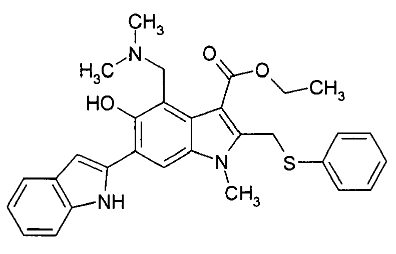 | 513,66 | 514 |
| **1.1.1(5)** | 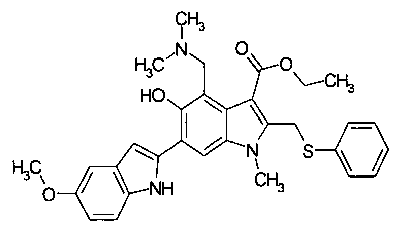 | 543,69 | 544 |
| Table 1. | | | |
| **1.1.1(6)** | 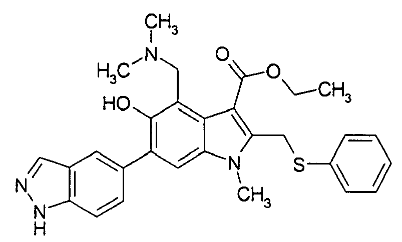 | 514,65 | 515 |
| **1.1.1(7)** | 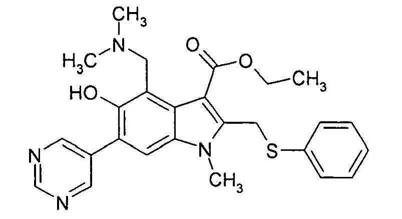 | 476,60 | 477 |
| **1.1.1(8)** | 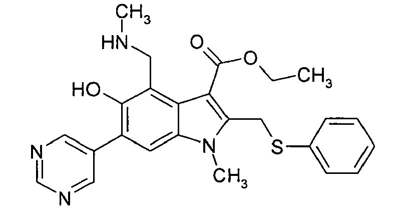 | 462,57 | 463 |
| **1.1.1(9)** | 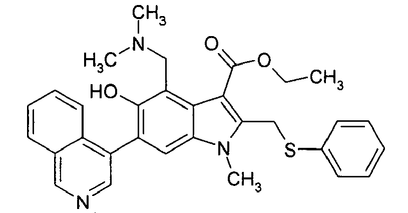 | 525,68 | 526 |
| **1.1.1(10)** | 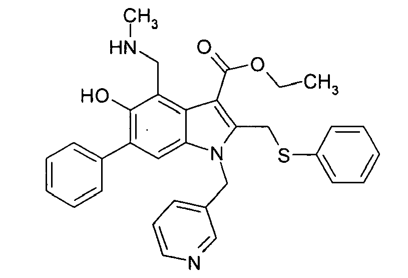 | 537,69 | 538 |
| **1.1.1(11)** | 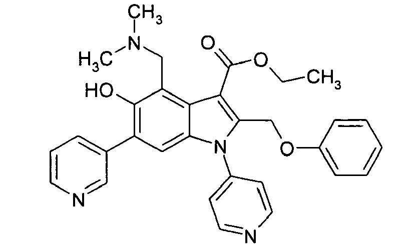 | 522,61 | 523 |
| Table 1. | | | |
| **1.1.1(12)** | 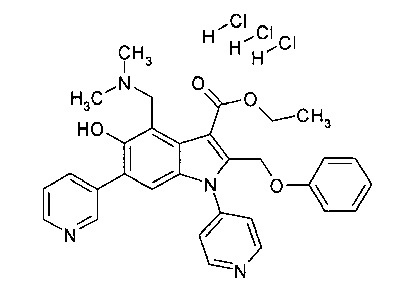 | 631,99 | 523 |
| **1.1.2(1)** | 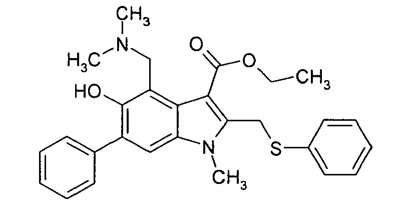 | 474,63 | 475 |
| **1.1.2(2)** | 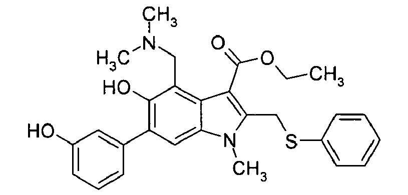 | 490,63 | 491 |
| **1.1.2(3)** | 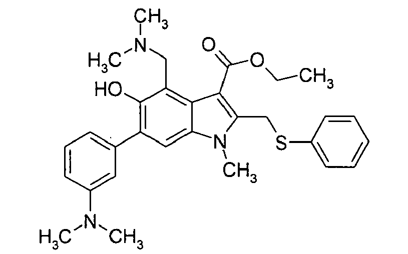 | 517,70 | 518 |
| **1.1.2(4)** | 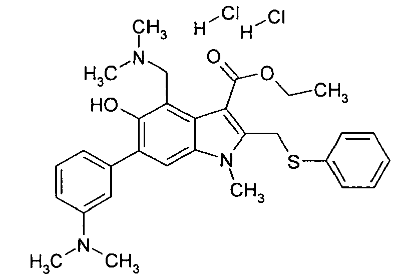 | 690,62 | 518 |
| **1.1.2(5)** | 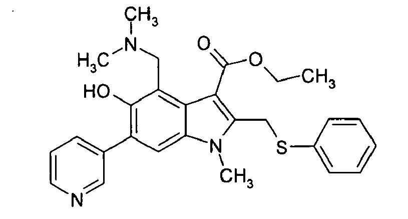 | 475.61 | 476 |
| Table 1. | | | |
| **1.1.2(6)** | 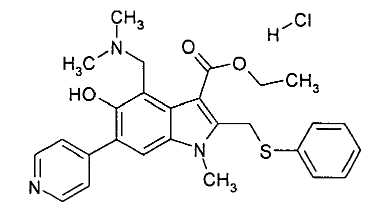 | 512,07 | 476 |
| **1.1.2(7)** | 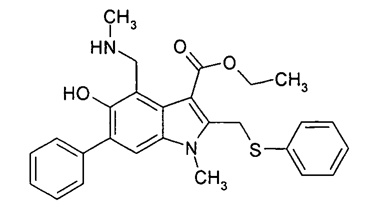 | 460,60 | 461 |
| **1.1.2(8)** | 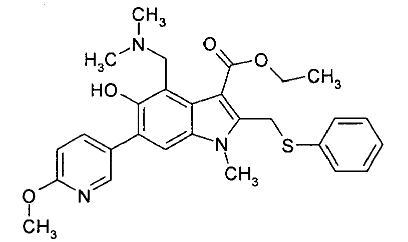 | 505,64 | 506 |
| **1.1.2(9)** | 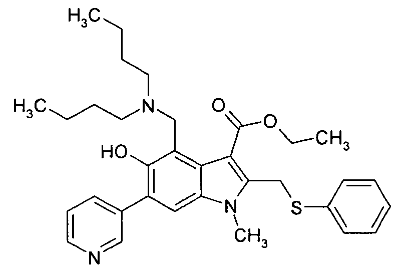 | 559,78 | 560 |
| **1.1.2(10)** | 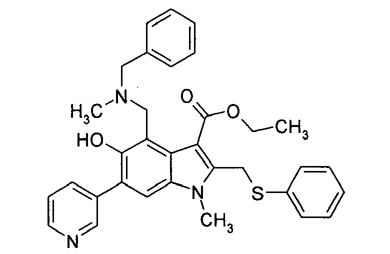 | 551,71 | 552 |
| **1.1.2(11)** | 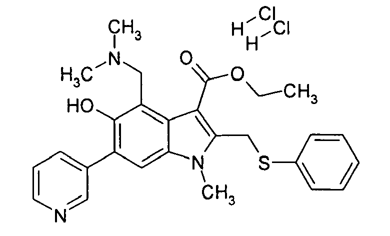 | 667,83 | 476 |
| Table 1. | | | |
| **1.1.2(12)** | 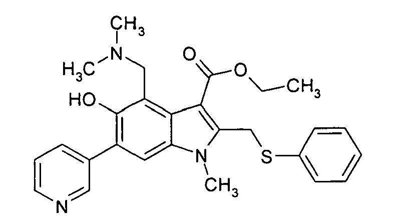 | 475,61 | 476 |
| **1.1.2(13)** | 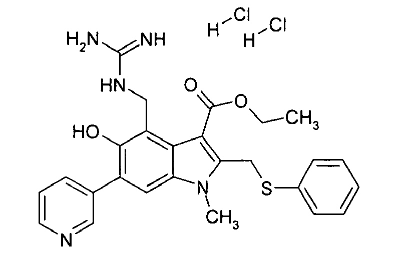 | 489,60 | 490 |
| **1.1.2(14)** | 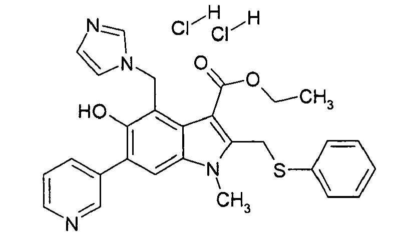 | 571,53 | 499 |
| **1.1.2(15)** | 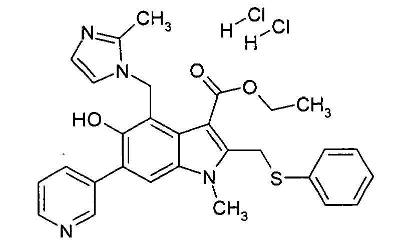 | 585.56 | 513 |
| **1.1.2(16)** | 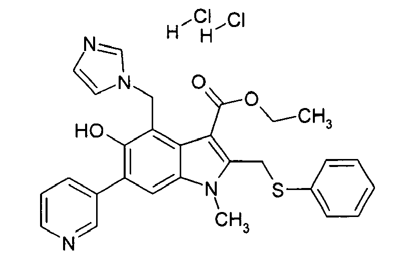 | 571,53 | 499 |
| **1.1.2(17)** | 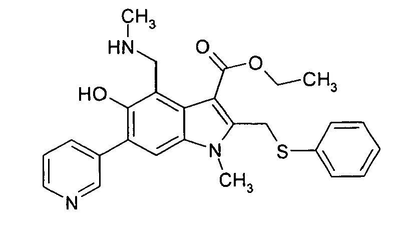 | 461,59 | 462 |
| Table 1. | | | |
| **1.1.2(18)** | 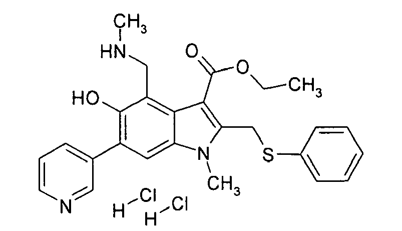 | 534,51 | 462 |
| **1.1.2(19)** | 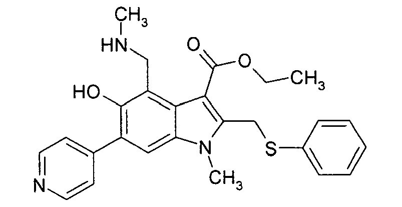 | 461,59 | 462 |
| **1.1.2(20)** | 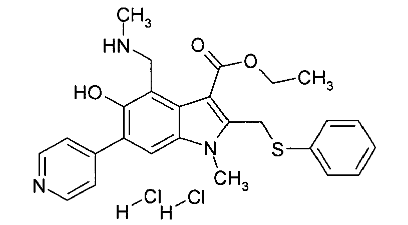 | 534,51 | 462 |
| **1.1.2(21)** | 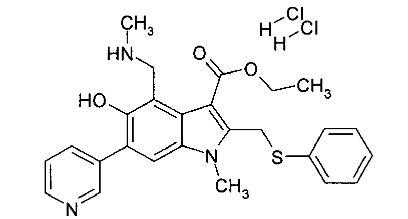 | 534,51 | 462 |
| **1.1.2(22)** | 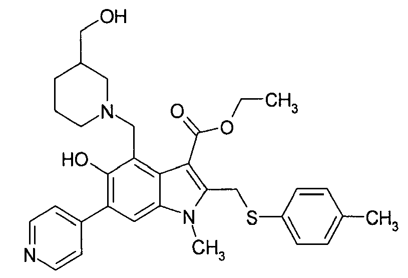 | 559,73 | 560 |
| **1.1.2(23)** | 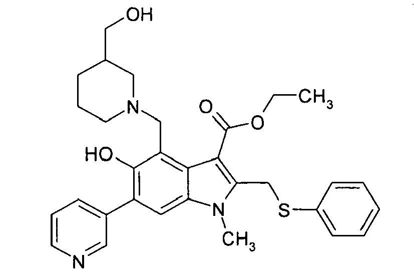 | 545,71 | 546 |
| Table 1. | | | |
| **1.1.2(24)** | 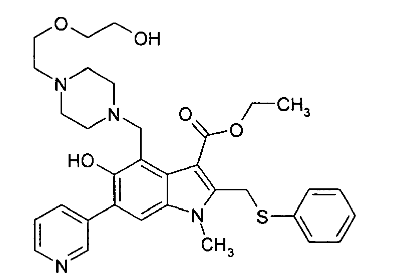 | 604,77 | 605 |
| **1.1.2(25)** | 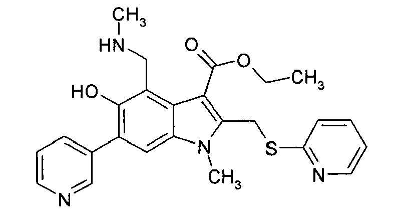 | 462,57 | 463 |
| **1.1.2(26)** | 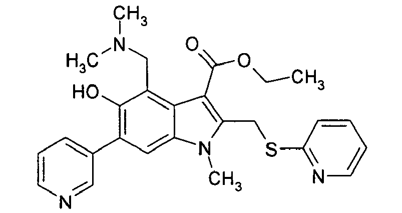 | 476,60 | 477 |
| **1.1.2(27)** | 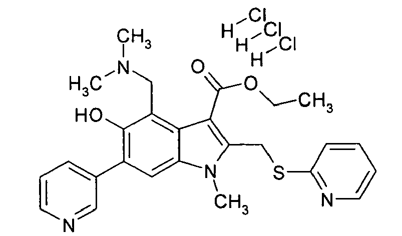 | 585,98 | 477 |
| **1.1.2(28)** | 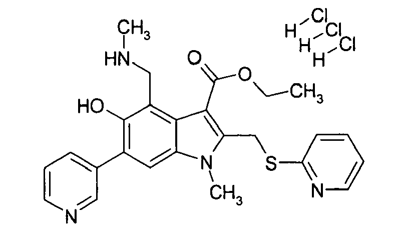 | 571.95 | 464 |
| **1.1.2(29)** | 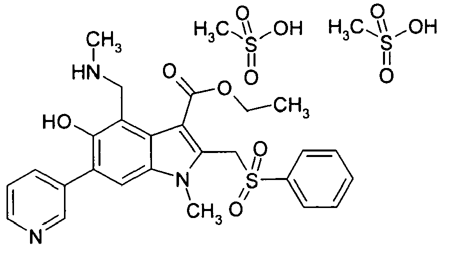 | 685.81 | 494 |
| Table 1. | | | |
| **1.1.2(30)** | 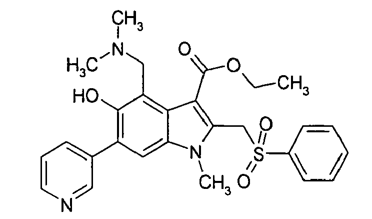 | 507.61 | 508 |
| **1.1.2(31)** | 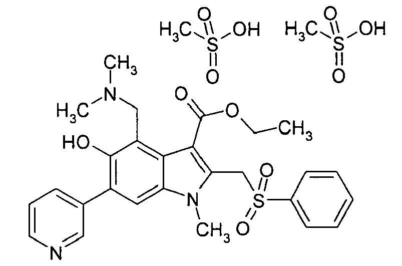 | 699,83 | 508 |
| **1.1.2(32)** | 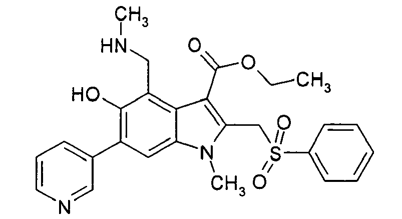 | 493,59 | 494 |
| **1.1.2(33)** | 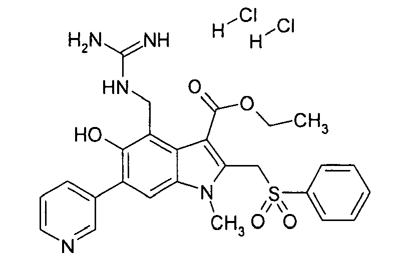 | 594.52 | 522 |
| **1.1.2(34)** | 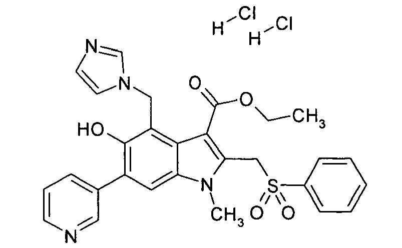 | 603.53 | 531 |
| **1.1.2(35)** | 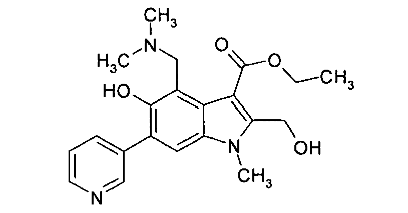 | 383,45 | 384 |
| Table 1. | | | |
| **1.1.2(36)** | 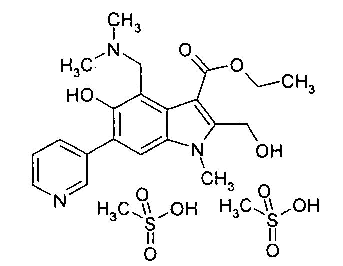 | 575,67 | 384 |
| **1.1.2(37)** | 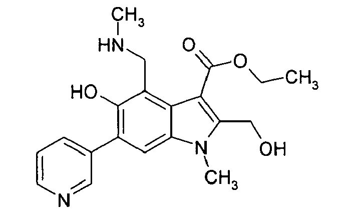 | 369,42 | 370 |
| **1.1.2(38)** | 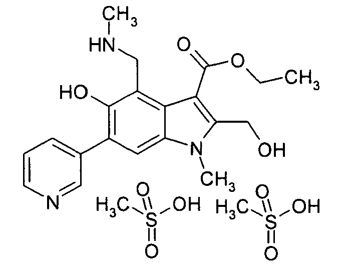 | 561,64 | 370 |
| **1.1.2(39)** | 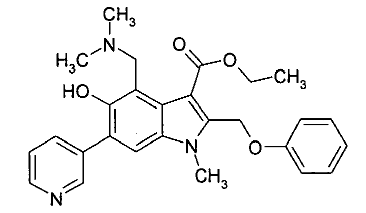 | 459,55 | 460 |
| **1.1.2(40)** | 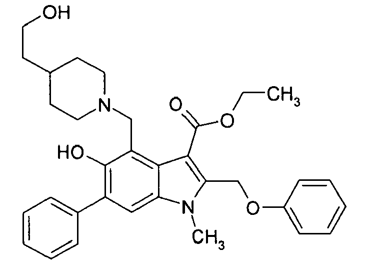 | 543,67 | 544 |
| **1.1.2(41)** | 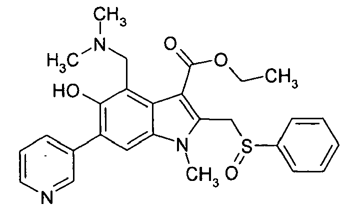 | 491,61 | 492 |
| Table 1. | | | |
| **1.1.2(42)** | 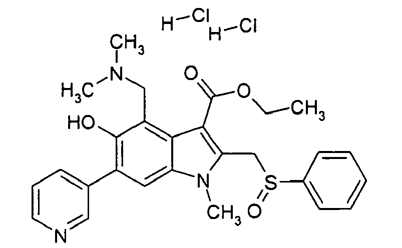 | 564,53 | 492 |
| **1.2.2(1)** | 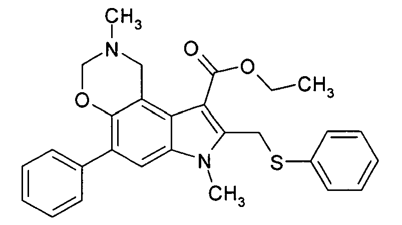 | 472,61 | 473 |
| **1.2.2(2)** | 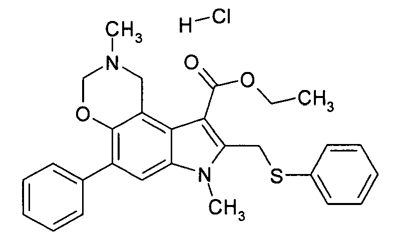 | 509,07 | 473 |
| **1.2.2(3)** | 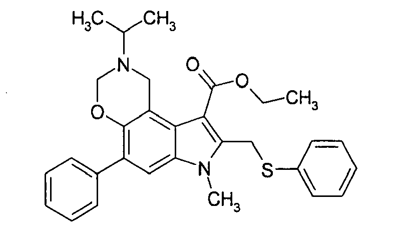 | 500,67 | 501 |
| **1.2.2(4)** | 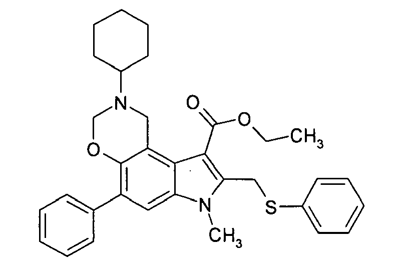 | 540,73 | 541 |
| **1.2.2(5)** | 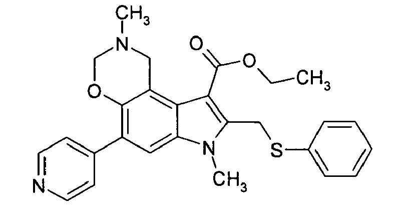 | 473,60 | 474 |
| Table 1. | | | |
| **1.2.2(6)** | 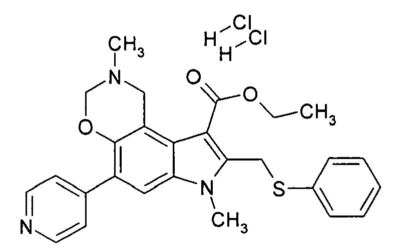 | 546,52 | 474 |
| **1.2.2(7)** | 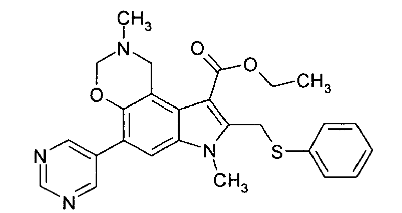 | 474,59 | 475 |
| **1.2.2(8)** | 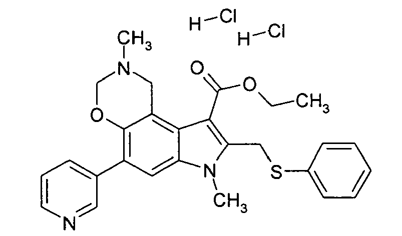 | 546.52 | 474 |
| **1.2.2(9)** | 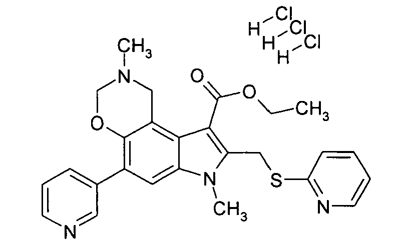 | 593,97 | 475 |
| **1.2.2(10)** | 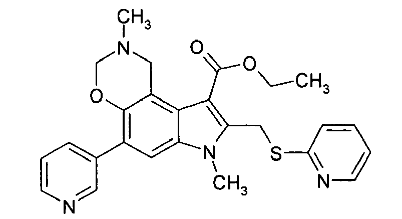 | 474,59 | 475 |
| **1.2.2(1 1)** | 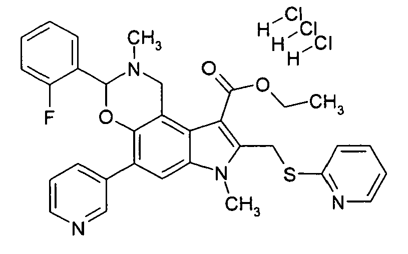 | 678,06 | 569 |
| Table 1. | | | |
| **1.2.2(12)** | 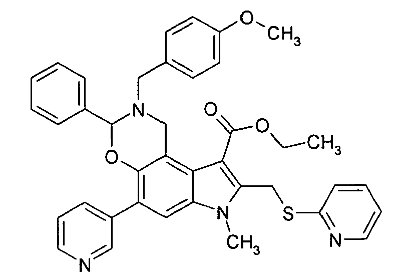 | 656,81 | 657 |
| **1.2.2(13)** | 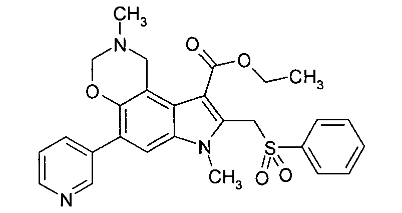 | 505.60 | 506 |
| **1.2.2(14)** | 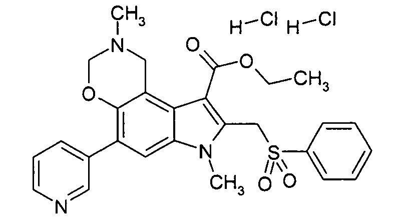 | 578,52 | 506 |
| **1.2.2(15)** | 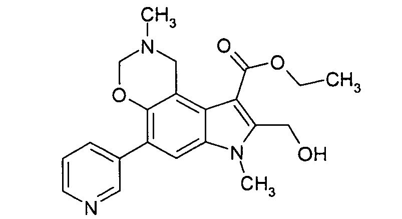 | 381.44 | 382 |
| **1.2.2(16)** | 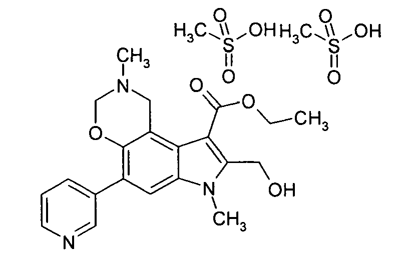 | 573.66 | 382 |
| **1.2.2(17)** | 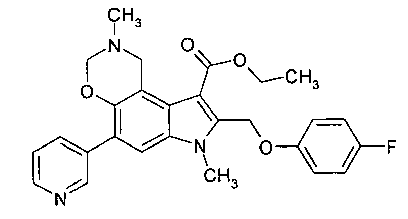 | 475,52 | 476 |
| Table 1. | | | |
| **1.2.2(18)** | 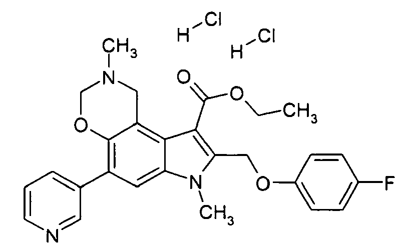 | 548,44 | 476 |
| **1.1.3(1)** | 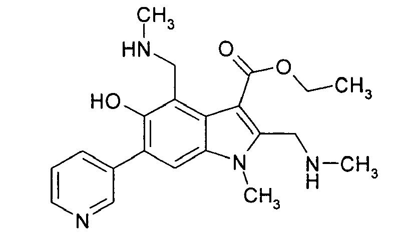 | 382,47 | 383 |
| **1.1.3(2)** | 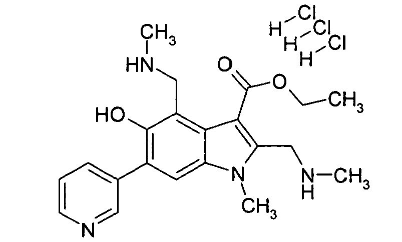 | 491,85 | 383 |
| **1.1.3(3)** | 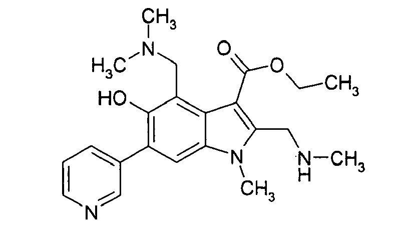 | 396,49 | 397 |
| **1.1.3(4)** | 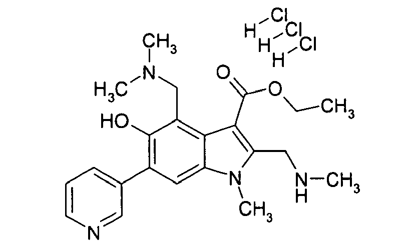 | 505,87 | 397 |
| **1.1.3(5)** | 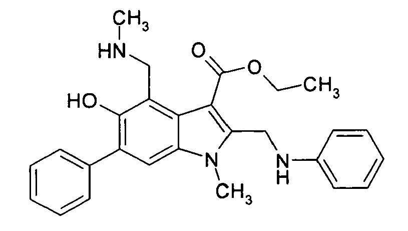 | 443,55 | 444 |
| Table 1. | | | |
| **1.1.3(6)** | 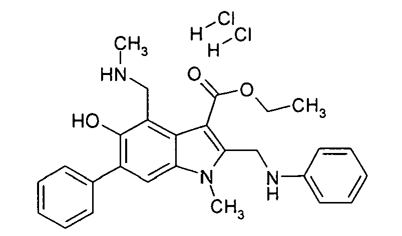 | 516.47 | 444 |
| **1.1.3(7)** | 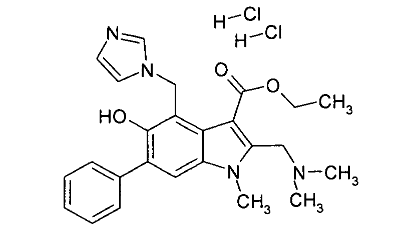 | 505.45 | 433 |
| **1.1.3(8)** | 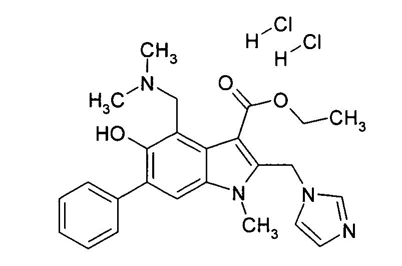 | 505.45 | 433 |
| **1.1.3(9)** | 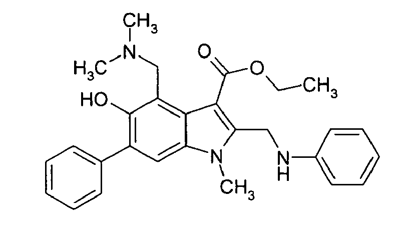 | 457,58 | 458 |
| **1.1.3(10)** | 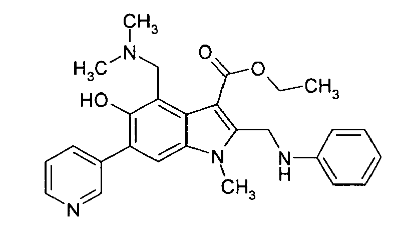 | 458,57 | 459 |
| **1.1.3(11)** | 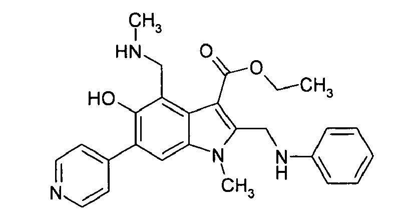 | 444,54 | 445 |
| Table 1. | | | |
| **1.1.3(12)** | 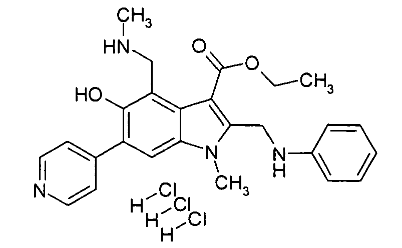 | 553,92 | 445 |
| **1.1.3(13)** | 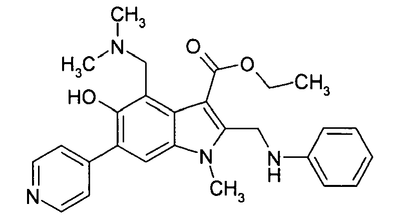 | 458,57 | 459 |
| **1.1.3(14)** | 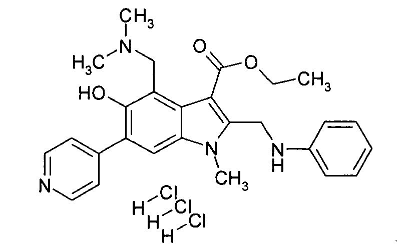 | 567,95 | 459 |
| **1.1.3(15)** | 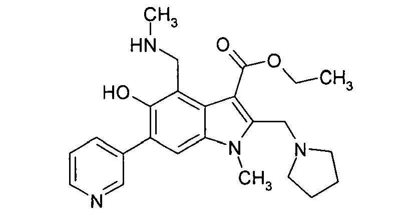 | 422,53 | 423 |
| **1.1.3(16)** | 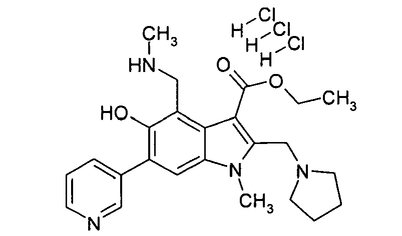 | 531,91 | 423 |
| **1.1.3(17)** | 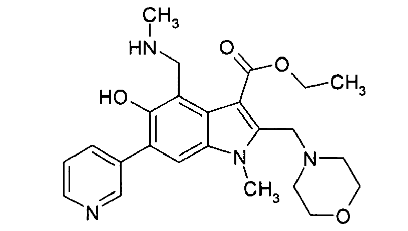 | 438,53 | 439 |
| Table 1. | | | |
| **1.1.3(18)** | 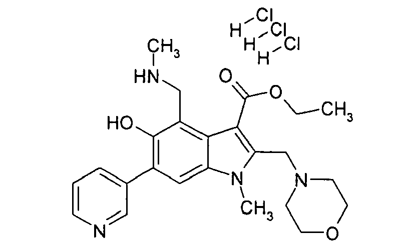 | 547,91 | 439 |
| **1.1.3(19)** | 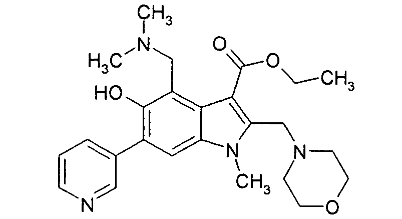 | 452,56 | 453 |
| **1.1.3(20)** | 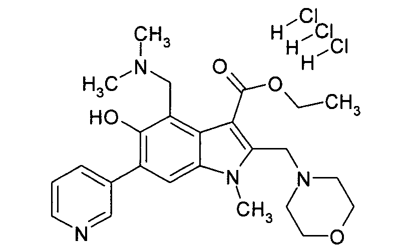 | 561,94 | 453 |
| **1.1.3(21)** | 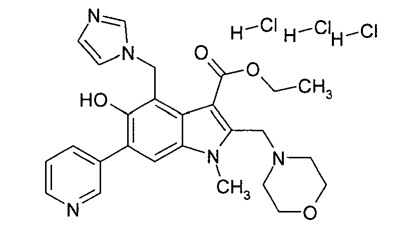 | 585,93 | 476 |
| **1.1.3(22)** | 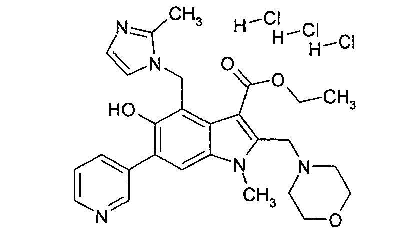 | 598,96 | 490 |
| **1.1.3(23)** | 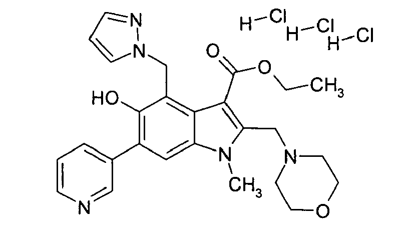 | 585,93 | 476 |
| Table 1. | | | |
| **1.1.3(24)** | 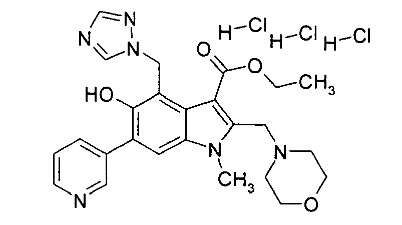 | 586,92 | 477 |
| **1.2.3(1)** | 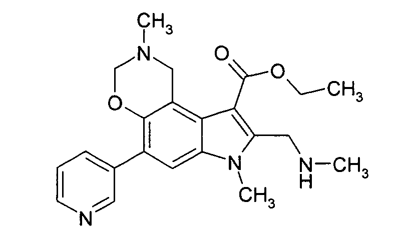 | 394,48 | 395 |
| **1.2.3(2)** | 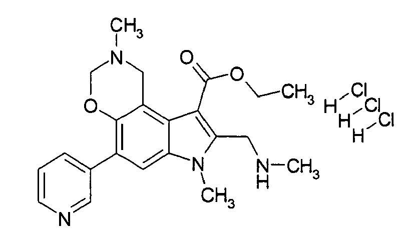 | 503,86 | 395 |
| **1.2.3(3)** | 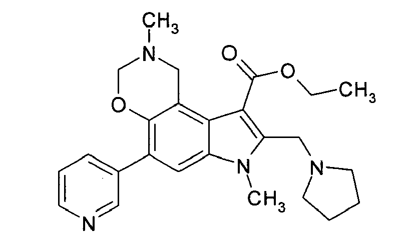 | 434,54 | 435 |
| **1.2.3(4)** | 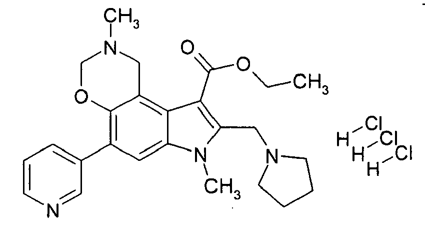 | 543,92 | 435 |
| **1.2.3(5)** | 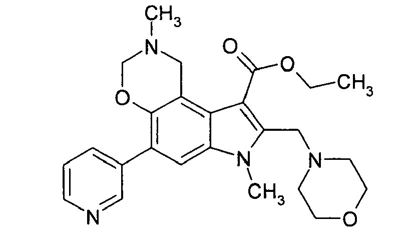 | 450,54 | 451 |
| Table 1. | | | |
| **1.2.3(6)** | 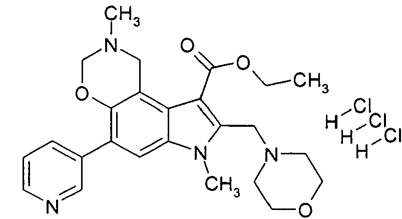 | 559,92 | 451 |
| **1.2.3(7)** | 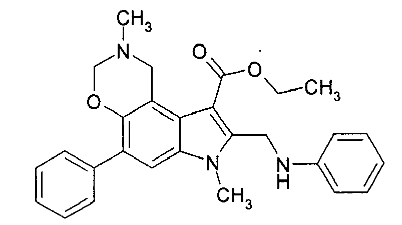 | 455,56 | 456 |
| **1.2.3(8)** | 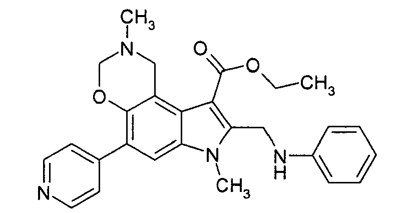 | 456,55 | 456 |
| **1.1.4(1)** | 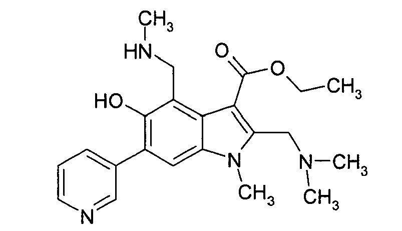 | 396,49 | 397 |
| **1.1.4(2)** | 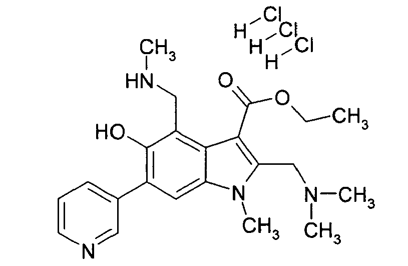 | 505,87 | 397 |
| **1.1.4(3)** | 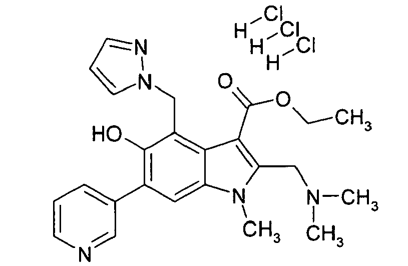 | 542.89 | 434 |
| Table 1. | | | |
| **1.1.4(4)** | 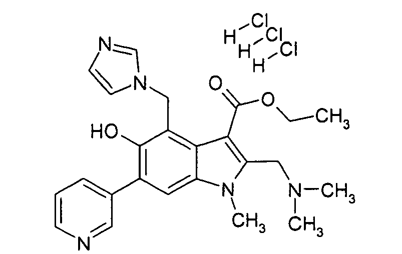 | 542.89 | 434 |
| **1.1.4(5)** | 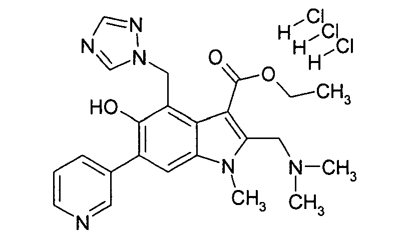 | 543,88 | 435 |
| **1.1.4(6)** | 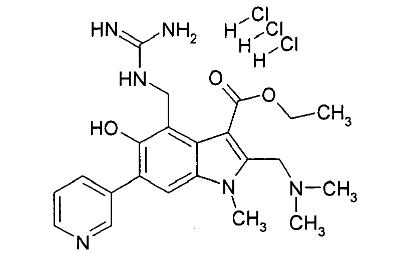 | 533,89 | 425 |
| **1.2.4(1)** | 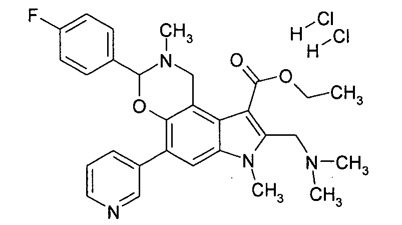 | 611,97 | 503 |
| **1.2.4(2)** | 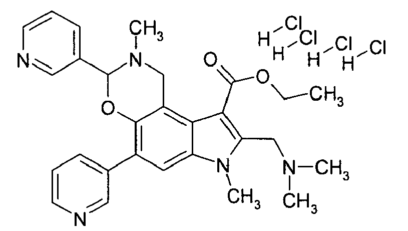 | 631,43 | 486 |
| **1.2.5(2)** | 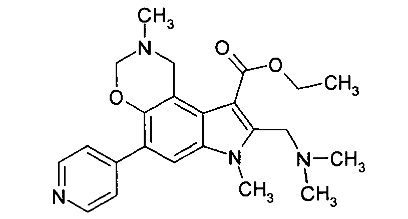 | 408,50 | 409 |
| Table 1. | | | |
| **1.2.5(2c)** | 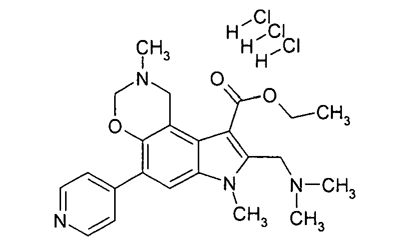 | 517,88 | 409 |
| **1.1.5(1)** | 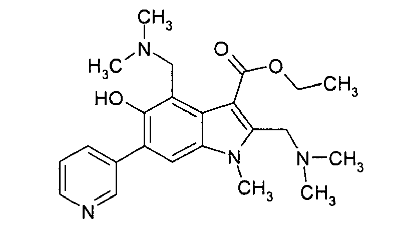 | 410,52 | 411 |
| **1.1.5(1c)** | 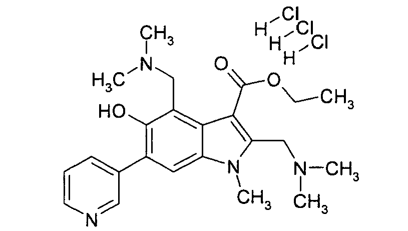 | 519,90 | 411 |
| **1.2.5(1)** | 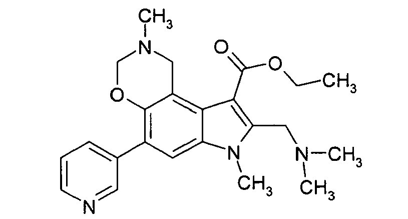 | 408,50 | 409 |
| **1.2.5(1c)** | 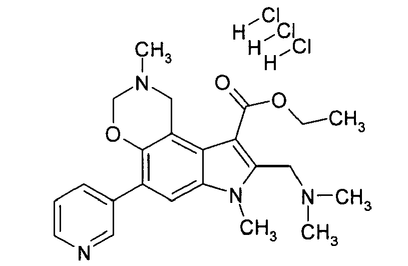 | 517,88 | 409 |
| **1.1.5(2)** | 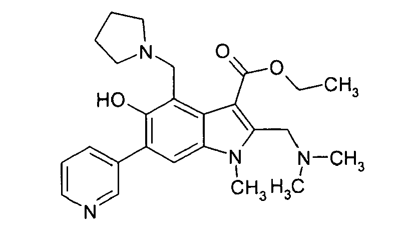 | 436,56 | 437 |
| Table 1. | | | |
| **1.1.5(2c)** | 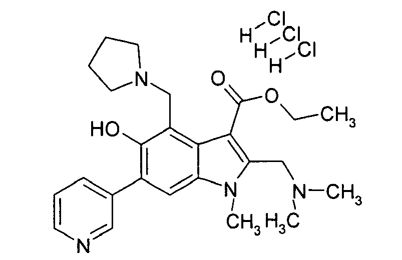 | 545,94 | 437 |
| **1.1.5(3)** | 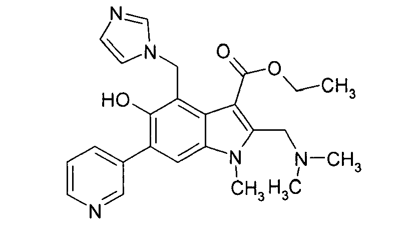 | 433,51 | 434 |
| **1.1.5(3c)** | 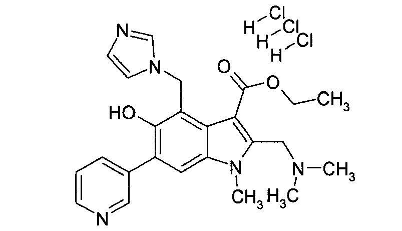 | 542,89 | 434 |
| **1.1.5(4)** | 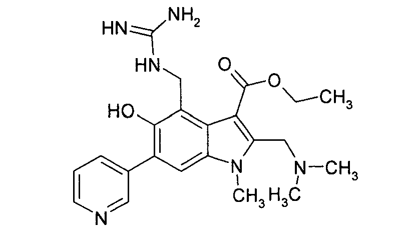 | 424,51 | 425 |
| **1.1.5(4c)** | 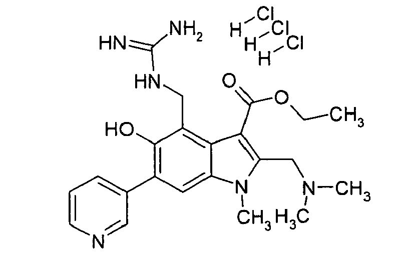 | 533,89 | 425 |
| **1.1.5(5)** | 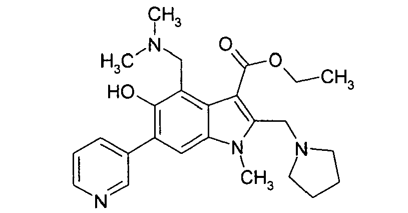 | 436,56 | 437 |
| Table 1. | | | |
| **1.1.5(5c)** | 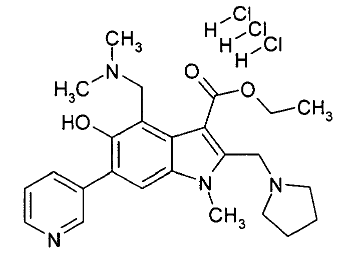 | 545,94 | 437 |

### Example 3. Test of compounds of general formula 1 for antiviral activity.

The test of compounds of general formula **1** for antiviral activity was conducted at a concentration of 10 mg/ml in relation to flu virus A in a culture of MDCK cells, with the virus diluted 10⁻³ by methods disclosed in prior art publications [Zotova, S.A; Komeeva, T.M; Shvedov, V.L; Fadeeva, N.L; Dineva, LA.; Fedyakina, I.T.; Kristova, M.L.; Nikolaeva, I.S.; Piters, V.V.; Guskova, T.A. Khim-Farm.Zh. 1995, 29(1), 51-53. PCT WO 2005/087729 A1, 2005**.].**

Inhibition of virus reproduction by some substituted 1H-indole-3-carboxylic acids of general formula **1** is shown in Table **2.**

**Table 2. Inhibition of Flu Virus A Reproduction by Substituted 1H-Indole-3-Carboxylic Acids of General Formula 1**

| **Nº** | **Formula** | **% of inhibition** |
|---|---|---|
| **1.1(11)** | 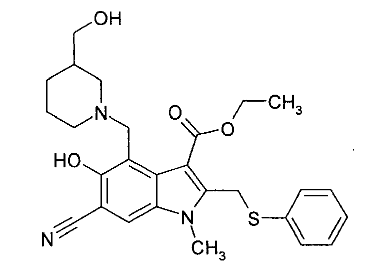 | <50 |
| **1.1(18)** | 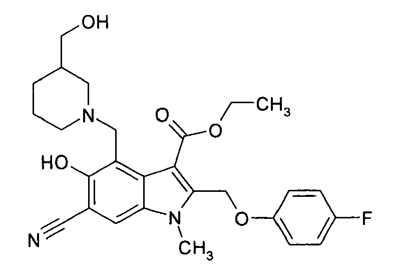 | <50 |
| Table 2. | | |
| **1.1.1(2)** | 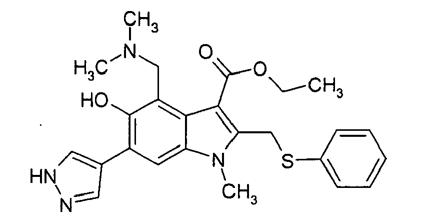 | 63 |
| **1.1.1(3)** | 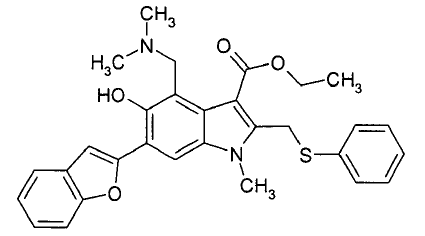 | 52 |
| **1.1.1(4)** | 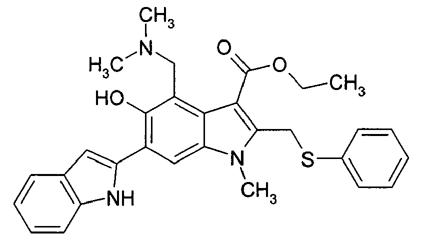 | 66 |
| **1.1.1(5)** | 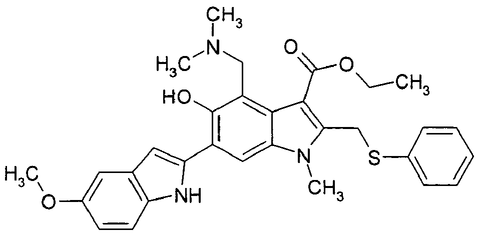 | 60 |
| **1.1.1(6)** | 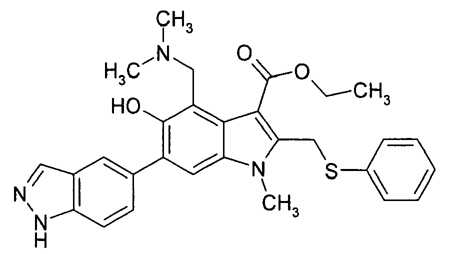 | 58 |
| **1.1.1(7)** | 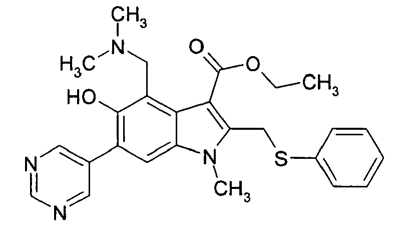 | 62 |
| Table 2. | | |
| **1.1.1(9)** | 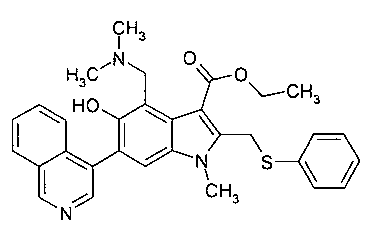 | 88 |
| **1.1.1(10)** | 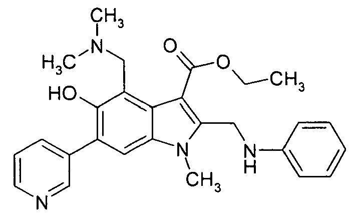 | 100 |
| **1.2(1)** | 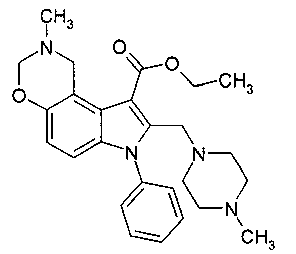 | <50 |
| **1.2(2)** | 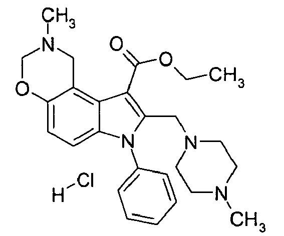 | <50 |
| **1.2(5)** | 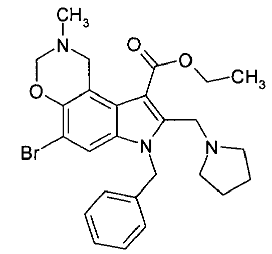 | 97 |
| **1.2(6)** | 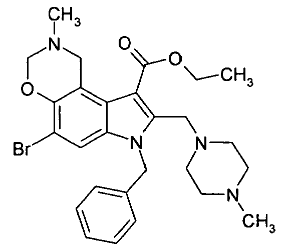 | 92 |
| Table 2. | | |
| **1.2(7)** | 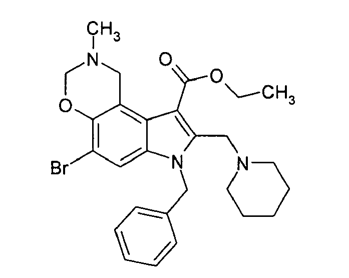 | 100 |
| **1.2(8)** | 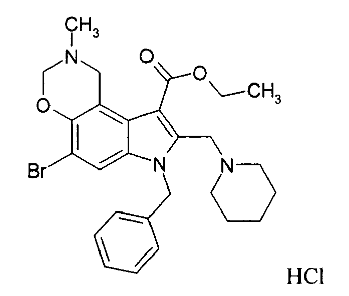 | 100 |
| **1.2(9)** | 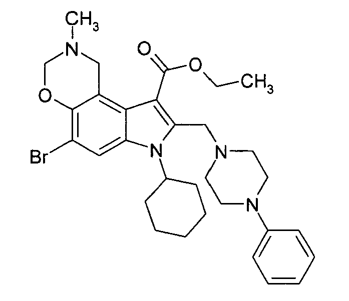 | 62 |
| **1.2(10)** | 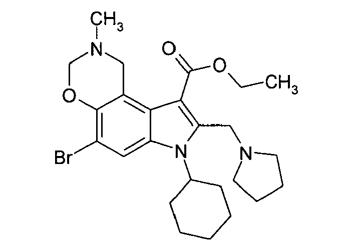 | 81 |
| **1.2(11)** | 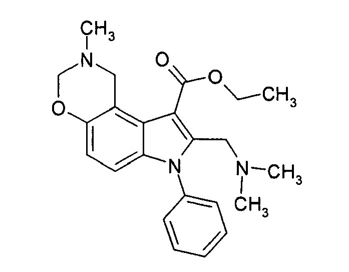 | 77 |
| Table 2. | | |
| **1.2(12)** | 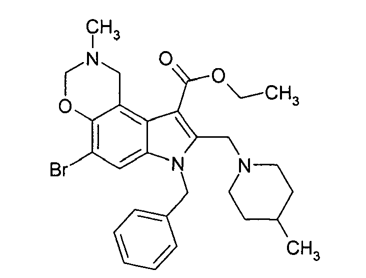 | 85 |
| **1.2(13)** | 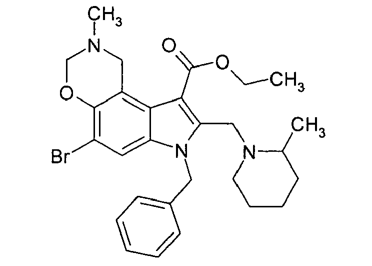 | 77 |
| **1.2(14)** | 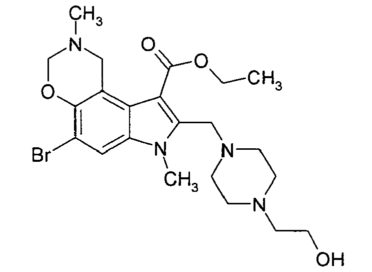 | 95 |
| **1.2(16)** | 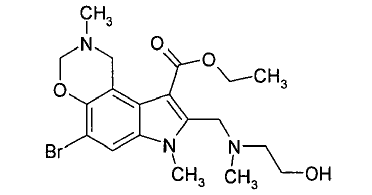 | 90 |
| **1.2(17)** | 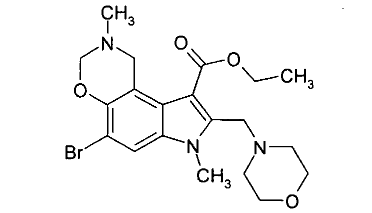 | 97 |
| **1.2(18)** | 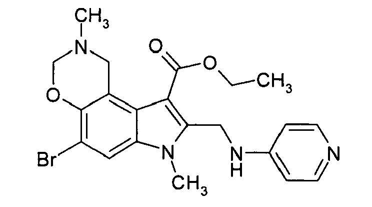 | 100 |
| Table 2. | | |
| **1.2(19)** | 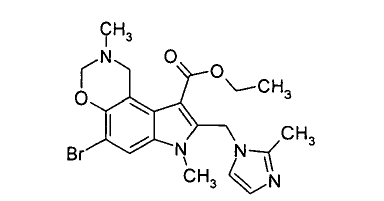 | 88 |
| **1.2(26)** | 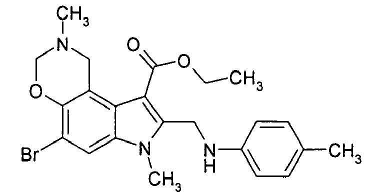 | 100 |
| **1.2(27)** | 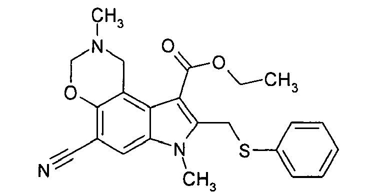 | <50 |
| **1.2(30)** | 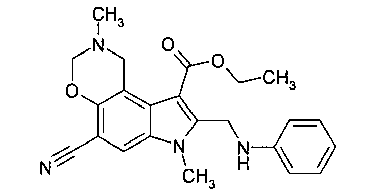 | <50 |
| **1.2(31)** | 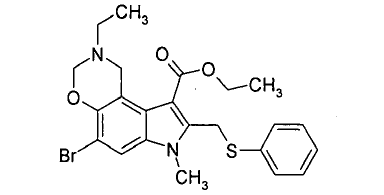 | 100 |
| **1.2(32)** | 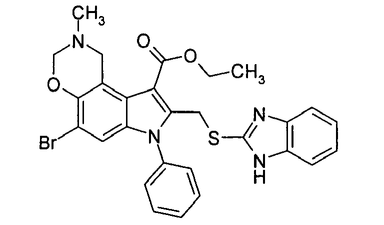 | 65 |
| **1.2(33)** | 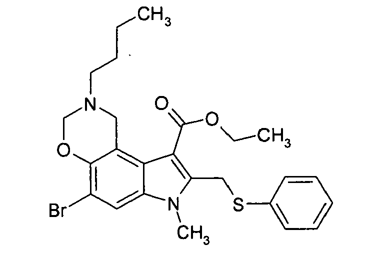 | 95 |
| Table 2. | | |
| **1.2(34)** | 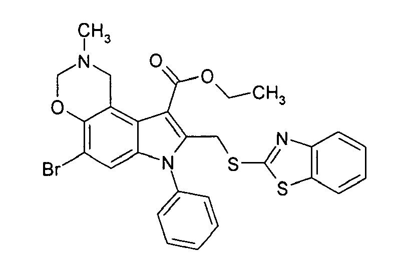 | <50 |
| **1.2(35)** | 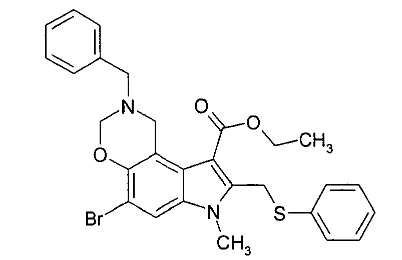 | 98 |
| **1.2(36)** | 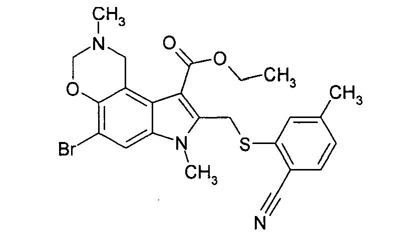 | <50 |
| **1.2(37)** | 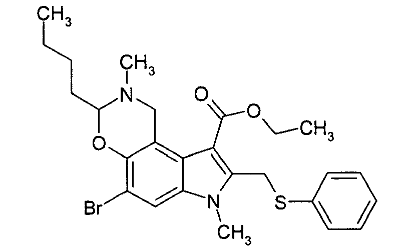 | 77 |
| **1.2(38)** | 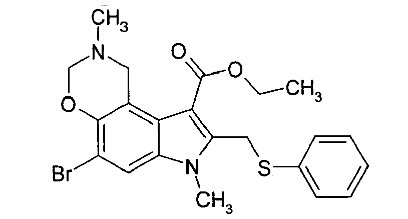 | 100 |
| **1.2(39)** | 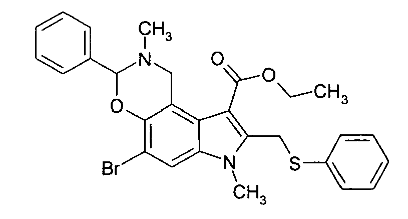 | 62 |
| Table 2. | | |
| **1.2(41)** | 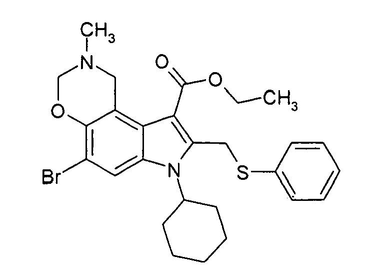 | 89 |
| **1.2(42)** | 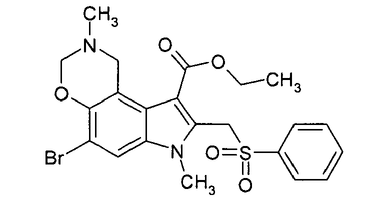 | 88 |
| **1.2(43)** | 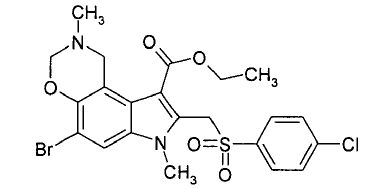 | 77 |
| **1.2(44)** | 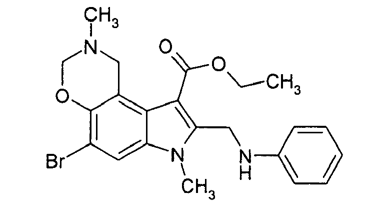 | 100 |
| **1.2(45)** | 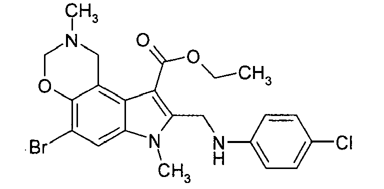 | 100 |
| **1.2(46)** | 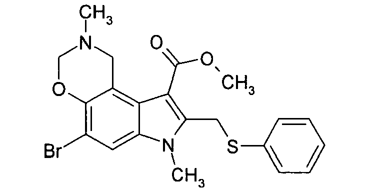 | 100 |
| **1.2(47)** | 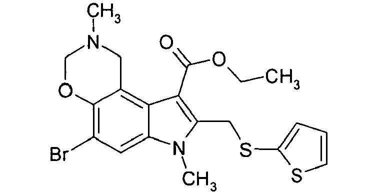 | 100 |
| Table 2. | | |
| **1.2(48)** | 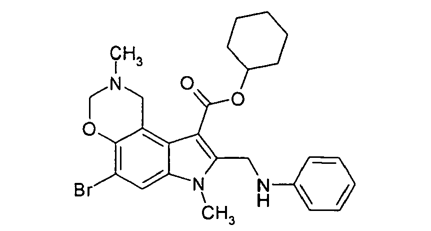 | 76 |
| **1.2(49)** | 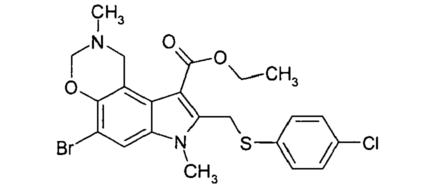 | 90 |
| **1.2.1(1)** | 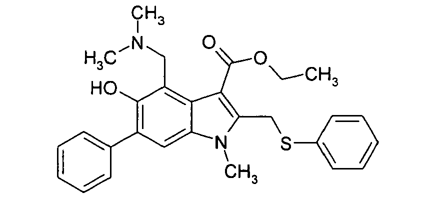 | 96 |
| **1.2.1(2)** | 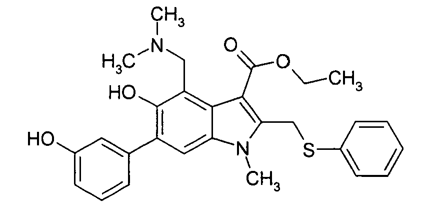 | 51 |
| **1.2.1(3)** | 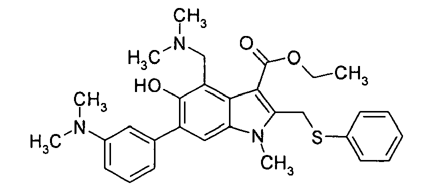 | 77 |
| **1.2.1(5)** | 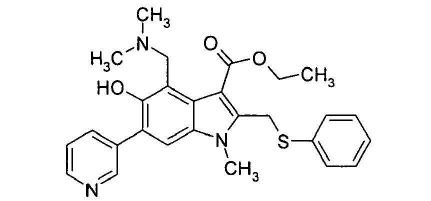 | 100 |
| **1.2.1(6)** | 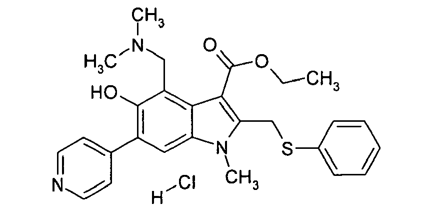 | 100 |
| Table 2. | | |
| **1.2.1(8)** | 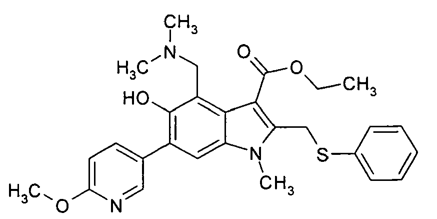 | 81 |
| **1.2.1(23)** | 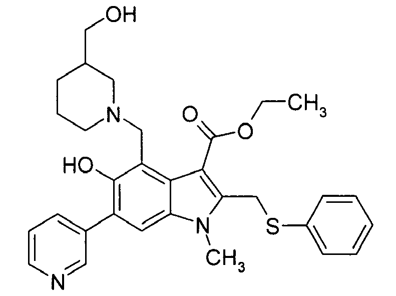 | 95 |
| **1.2.1(29)** | 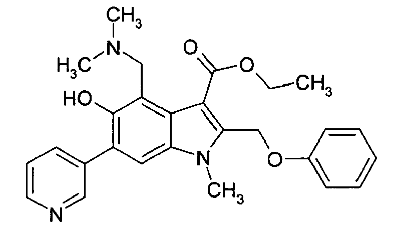 | 88 |
| **1.2.2(3)** | 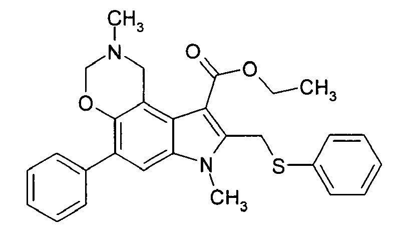 | 95 |
| **1.2.2(5)** | 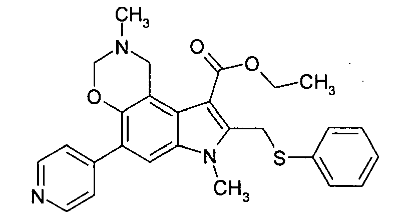 | 100 |
| **1.2.2(7)** | 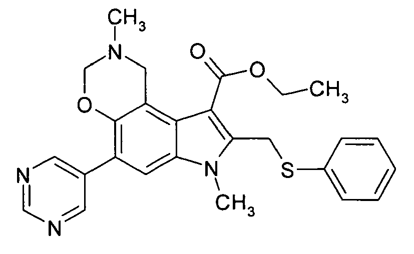 | 88 |
| Table 2. | | |
| **1.2.2(8)** | 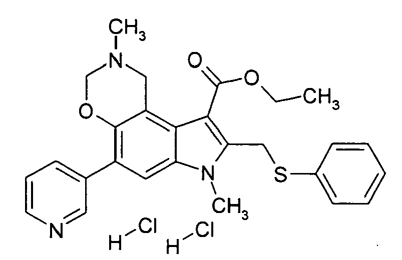 | 100 |
| **1.2.3(7)** | 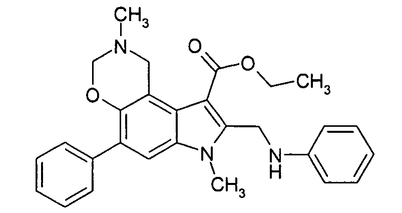 | 90 |
| **1.2.3(8)** | 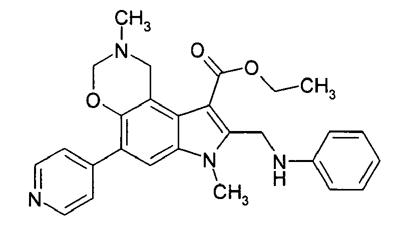 | 100 |

**Example 4.** Antiviral activity of a pharmaceutical composition containing one of the compounds of general formula **1** as an active substance was determined in relation to the viruses by human hepatocytes (Huh7.5.1) cloned expressly for this purpose and having a high sensitivity to infection by the hepatitis C virus [Ralf Bartenschlager* and Thomas Pietschmann. Proc Natl Acad Sci USA. 2005 July 12; 102 (28): 9739-9740]. The cells were cultivated in a modified Eagle environment Dulbecco (DMEM) containing 9% calf serum and 1% of replaceable amino acids. Cells were poured into each hole of a six-hole plate (200,000 cells per hole) and were left for 24 hours, whereupon the test compounds were added to them at different concentrations. The cells and test compounds were left for 24 hours, whereupon the hepatitis C virus strain JFH-1 (genotype 2a) was added to the cells, as described in [Zhong J, Gastaminza P, Cheng G, Kapadia S, Kato T, Burton DR, Wieland SF, Uprichard SL, Wakita T, Chisari FV: Robust hepatitis C virus infection in vitro. Proc Natl Acad Sci U S A 2005, 102:9294-9299], and the cells were incubated at 37°C for 72 hours. At the end of incubation, the cell medium was removed, and were lysogenized with a buffer containing 50 mM of tris-HCl, pH 7.2; 150 mM of NaCl, 0.1% SDS, 0.1% of Na deoxycholate, 1% of Triton X-100, and 17.4 g/ml PMSF, and the protein content was quantified using the BCA Protein Assay (Pierce Biotechnology, USA). Before the sample was applied to gel, the concentration of each sample was adjusted so that 10 µg of protein was added to each gel hole. The samples were mixed with an equal volume of a reducing buffer, heated at 95°C for 7 minutes, and subjected to electrophoresis in a 4-20% tris-glycine buffer (Invitrogen, USA). The separated proteins were transferred on to a 0.45µM nitrocellulose membrane (Pierce, USA), using a semidry transfer system. After the transfer, the membranes were blocked with a Superblock buffer and incubated with primary mouse antibodies specific to virus proteins NS5A (Biodesign International, Saco, ME.; 1:1000 dilution) and Core (Affinity Bioreagents, Golden, CO; 1:1000 dilution), and further left for one hour at room temperature. The membranes were then left with a secondary anti-mouse immunoglubulin G conjugated with horseradish peroxidase (IgG, Pierce, 1:10000 dilution). The resultant protein strips were visualized using the LumiGlo hemi-luminescent reagent (Cell Signaling, Danvers, MA) and membrane exposition with X-ray film. The strips were digitized with the use of the ImageJ program package [http://rsb.info.nih.gov/ij/].

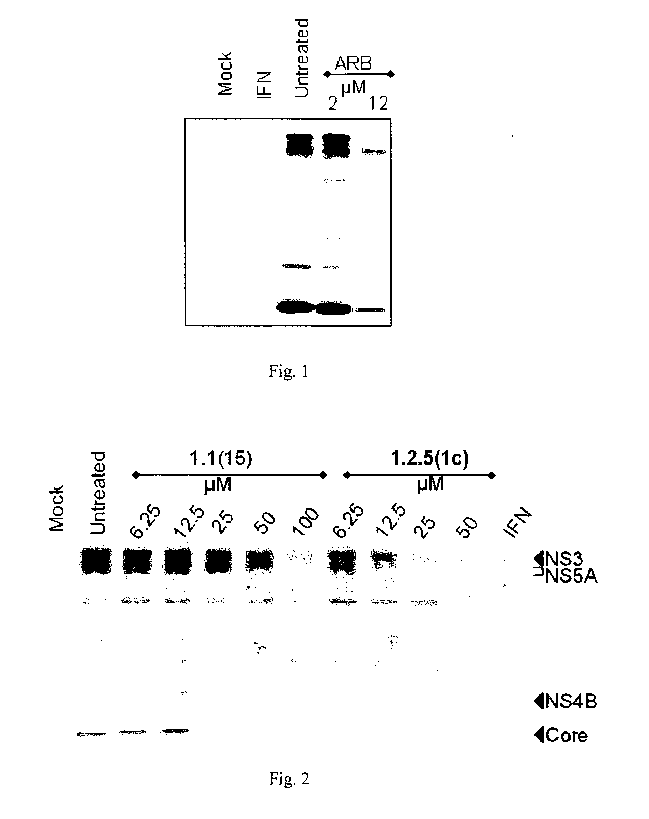

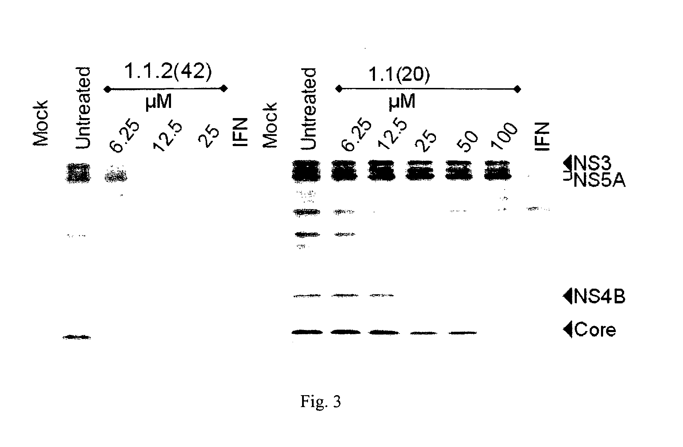

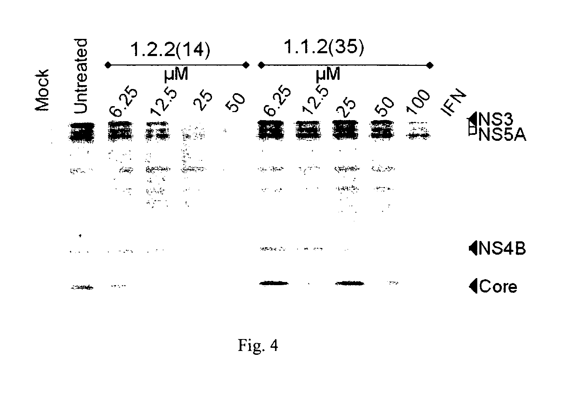

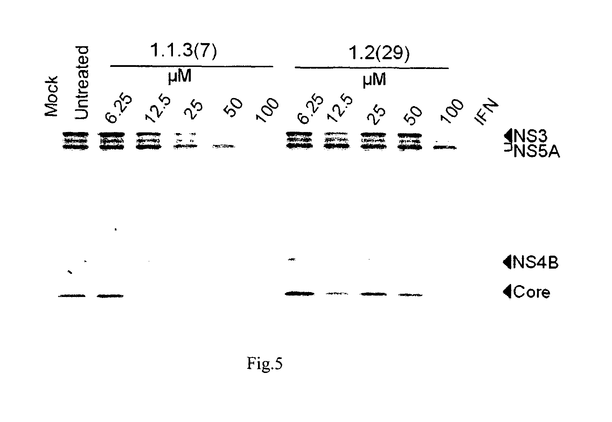

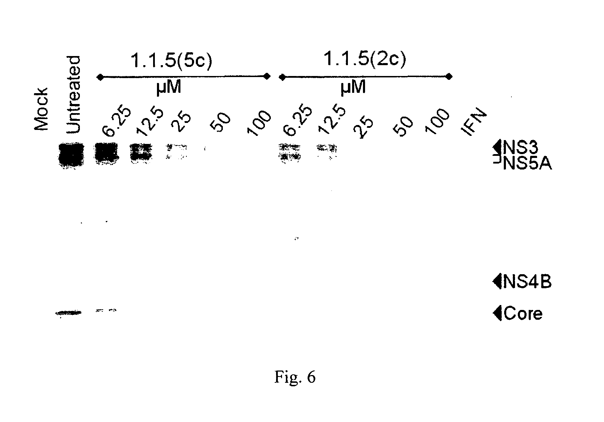

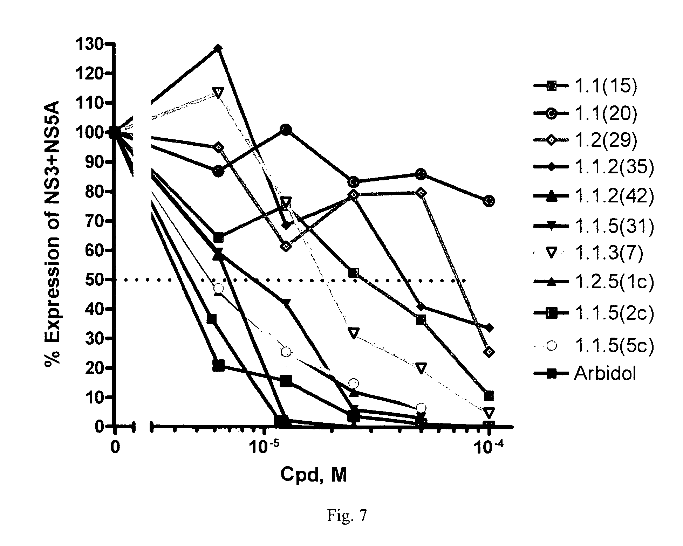

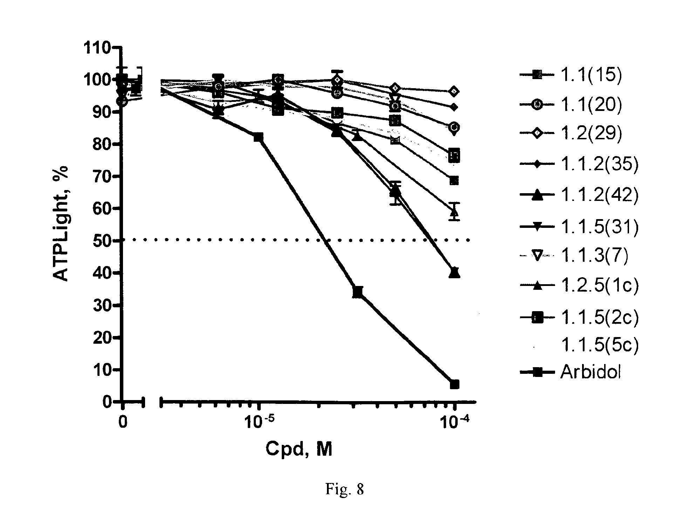

The results of the tests are shown in FIGs. 1 to 8 and in Table 3. Table 3 shows the values of antiviral activity (IC₅₀), toxicity (TC₅₀), and the therapeutic index (SI₅₀ = TC₅₀/IC₅₀) of some of the substances tested. The data shown confirm the high efficiency of the new antiviral substances.

**Table 3. Antiviral Activity (IC₅₀^{HCV}), Toxicity (TC₅₀), and Therapeutic Index (SI₅₀ = TC₅₀/IC₅₀) of Some of the Substances Tested**

| **Nº comp.** | **Formula** | **IC₅₀** | **TC₅₀** | **Therapeutic index SI₅₀ = TC ₅₀ / IC₅₀** |
|---|---|---|---|---|
| | | **uM** | | |
| **1.1(15)** | 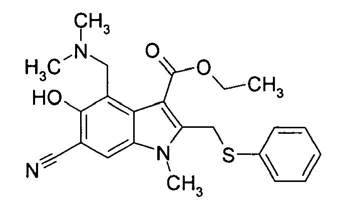 | 28 | >100 | >4 |
| **1.2(29)** | 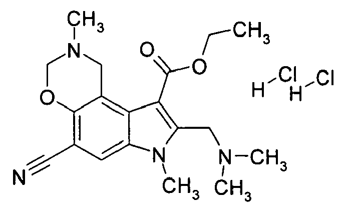 | >100 | >100 | - |
| Table 3. | | | | |
| **1.1.2(35)** | 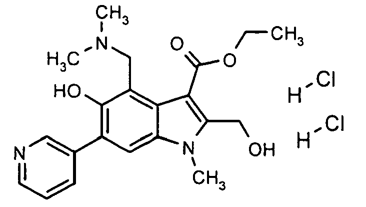 | 6 | >100 | >16 |
| **1.1.2(42)** | 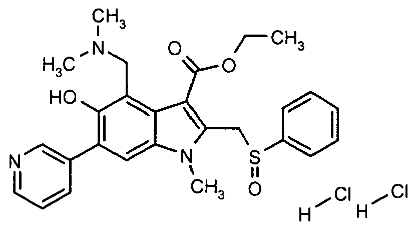 | 18 | >100 | >5 |
| **1.2.2(14)** | 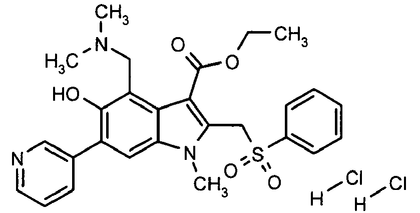 | 43 | >100 | >2 |
| **1.1.3(7)** | 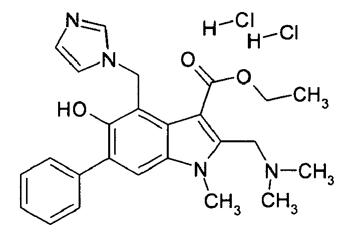 | 72 | >100 | >1,4 |
| **1.2.5(1c)** | 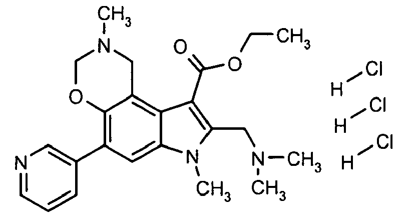 | 6 | 78 | 13 |
| **1.1.5(2c)** | 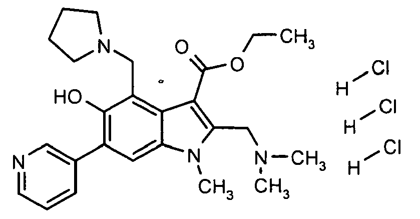 | 7 | 75 | 11 |
| Table 3. | | | | |
| **1.1.5(5c)** | 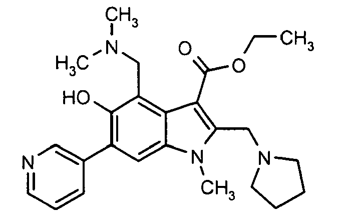 | <5 | >100 | >20 |

### Example 5. Preparation of a medicament in tablet form.

A mixture of 1,600 mg of starch, 1,600 mg of ground lactose, 400 mg of talc, and 1,000 mg of hydrochloride of 4-dimethylaminomethyl-5-hydroxy-1-methyl-2-(pyrrolidin-1-yl)-6-(pyridin-3-yl)-1H-indole-3-carboxylic acid ethyl ester **1.1.5(5c)** is prepared and compressed into a bar. The resultant bar is comminuted into granules and sifted through a sieve to collect granules 14-16 mesh. The granules thus obtained are shaped into tablets of suitable form weighing 560 mg each. Similarly, according to the invention, pharmaceutical compositions are produced in the form of tablets containing other substituted 1H-indole-3-carboxylic acids of general formula **1.**

### Example 6. Preparation of a medicament in capsule form.

Hydrochloride of 4-dimethylaminomethyl-5-hydroxy-1-methyl-2-(pyrrolidin-1-yl)-6-(pyridin-3-yl)-1H-indole-3-carboxylic acid ethyl ester **1.1.5(5c)** is carefully mixed with lactose powder at a ratio of 2:1. The resultant powdery mixture is packed into gelatin capsule of suitable size 300 mg to a capsule.

### Example 7. Preparation of a medicament in the form of injectable compositions for intramuscular, intraperitoneal or subcutaneous injections.

A solution comprising 500 mg of hydrochloride of 4-dimethylaminomethyl-5-hydroxy-1-methyl-2-(pyrrolidin-1-yl)-6-(pyridin-3-yl)-1H-indole-3-carboxylic acid ethyl ester **1.1.5(5c)** is mixed with 300 mg of chlorobutanol, 2 ml of propylene glycol, and 100 ml of injectable water. The resultant solution is filtered and placed into capsules 1 ml to a capsule, and the capsules are sealed and sterilized in an autoclave.

### Industrial applicability

The invention could be used in medicine, veterinary, biochemistry.

## Claims

1. Substituted indoles of general formula **1,** or racemates, optical isomers, pharmaceutically acceptable salts and/or hydrates thereof 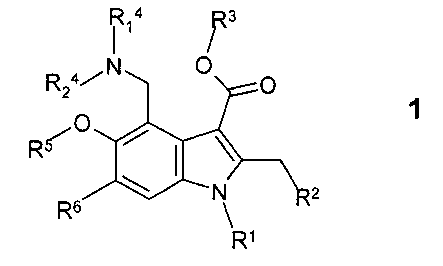 wherein: R¹, R₁⁴ and R₂⁴ independently of each other are an amino group substituent, selected from hydrogen, optionally substituted alkyl, optionally substituted cycloalkyl, optionally substituted aryl or optionally substituted heterocyclyl, or R₁⁴ and R₂⁴ together with the nitrogen atom to which they are attached via R₁⁴ and R₂⁴ form an optionally substituted azaheterocyclyl or guanidyl; R² is an alkyl substituent selected from hydrogen, optionally substituted mercapto group, optionally substituted amino group, optionally substituted hydroxy group, R³ is lower alkyl; R⁵ is hydrogen atom, or R⁵ together with the oxygen atom to which it is attached and R₂⁴ together with the nitrogen atom to which it is attached via R⁵ and R₂⁴ form an oxazine ring; R⁶ is a cyclic system substituent selected from hydrogen, halogen, cyano group, optionally substituted aryl or optionally substituted heterocyclyl,
with the **exception** of the compounds of the general formula **A** 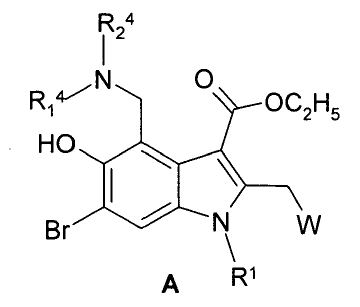 wherein: R¹ is alkyl or cycloalkyl; R₁⁴ and R₂⁴ independently of each other are an amino group substituent selected from hydrogen, optionally substituted alkyl, optionally substituted cycloalkyl, optionally substituted aryl or optionally substituted heterocyclyl, or R₁⁴ and R₂⁴ together with the nitrogen atom to which they are attached via R₁⁴ and R₂⁴ form an optionally substituted azaheterocyclyl; W is a substituted mercapto group.

2. Compounds as claimed in claim 1 representing 5-hydroxy-1H-indole-3-carboxylates of the general formula **1.1** and pyrrolo[3,2-*f*][1,3]benzoxazine-5-carboxylates of the general formula **1.2,** or racemates, optical isomers, pharmaceutically acceptable salts and/or hydrates thereof 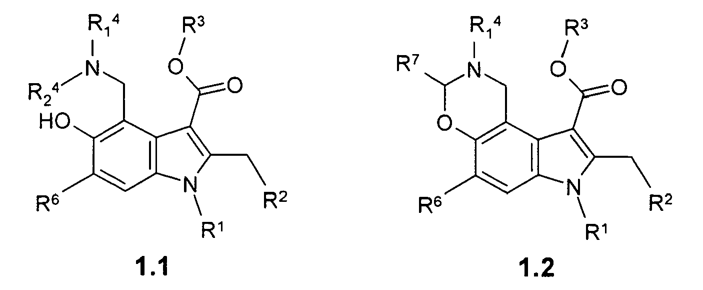 wherein: R¹, R², R³, R₁⁴, R₂⁴ and R⁶ have the above meanings; R⁷ is a cyclic system substituent selected from hydrogen, optionally substituted alkyl, optionally substituted cycloalkyl, optionally substituted aryl or optionally substituted heterocyclyl.

3. Compounds as claimed in claim 2 representing ethyl 4-(aminomethyl)-6-aryl(or heterocyclyl)-5-hydroxy-1H-indole-3-carboxylates of the general formula **1.1.1** and ethyl 9-aryl(or heterocyclyl)-1,2,3,7-tetrahydropyrrolo[3,2-f][1,3]benzoxazine-5-carboxylates of the general formula **1.2.1,** or racemates, optical isomers, pharmaceutically acceptable salts and/or hydrates thereof 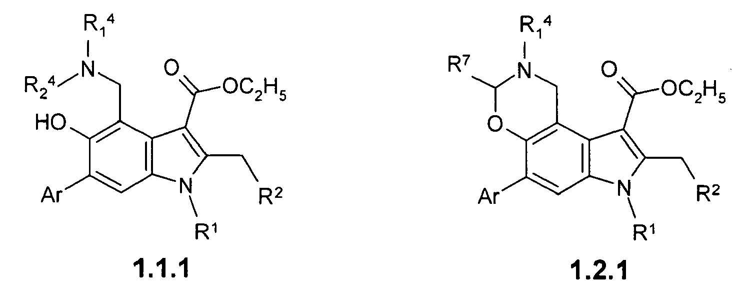 wherein: R¹, R², R₁⁴, R₂⁴ and R⁷ have the above meanings; Ar is aryl or 5-6-membered heterocyclyl, comprising, at least, one heteroatom selected from N, O or S.

4. Compounds as claimed in claim 3 representing ethyl 4-(aminomethyl)-6-aryl(or pyridyl)-5-hydroxy-1-methylindole-3-carboxylates of the general formula **1.1.2** and ethyl 9-aryl(or pyridyl)-7-methyl-1,2,3,7-tetrahydropyrrolo[3,2-*f*][1,3]benzoxazine-5-carboxylates of the general formula **1.2.2,** or racemates, optical isomers, pharmaceutically acceptable salts and/or hydrates thereof 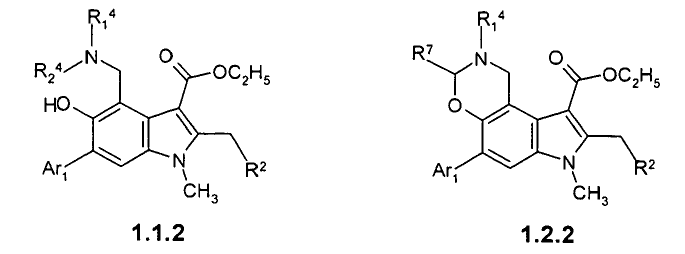 wherein: R², R₁⁴, R₂⁴ and R⁷ have the above meanings; Ar₁ is aryl or pyridyl.

5. Compounds as claimed in claim 4 representing ethyl 6-aryl(or pyridyl)-2,4-bis(aminomethyl)-5-hydroxy-1-methylindole-3-carboxylates of the general formula **1.1.3** and ethyl 6-(aminomethyl)-9-aryl(or pyridyl)-7-methyl-1,2,3,7-tetrahydropyrrolo[3,2-*f*][1,3]benzoxazine-5-carboxylates of the general formula **1.2.3,** or racemates, optical isomers, pharmaceutically acceptable salts and/or hydrates thereof 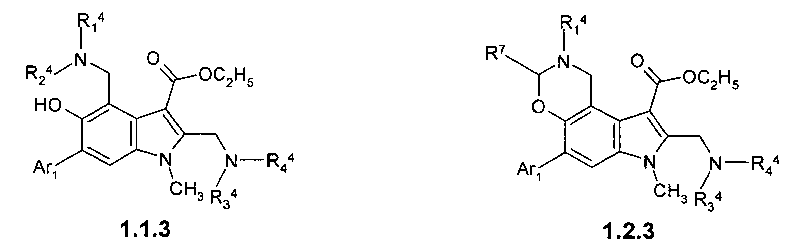 wherein: R₁⁴, R₂⁴, R⁷ and Ar₁ have the above meanings; R₃⁴ and R₄⁴ independently of each other are an amino group substituent selected from hydrogen, optionally substituted alkyl, optionally substituted cycloalkyl, optionally substituted aryl or optionally substituted heterocyclyl, or R₃⁴ and R₄⁴ together with the nitrogen atom to which they are attached via R₃⁴ and R₄⁴ form an optionally substituted azaheterocyclyl or guanidyl.

6. Compounds as claimed in claim 5 representing ethyl 4-(aminomethyl)-6-aryl(or pyridyl)-5-hydroxy-2-((dimethylamino)methyl)-1-methylindole-3-carboxylates of the general formula **1.1.4** and ethyl 9-aryl(or pyridyl)-6-((dimethylamino)methyl)-7-methyl-1,2,3,7-tetrahydropyrrolo[3,2-*f*][1,3]benzoxazine-5-carboxylates of the general formula **1.2.4,** or racemates, optical isomers, pharmaceutically acceptable salts and/or hydrates thereof 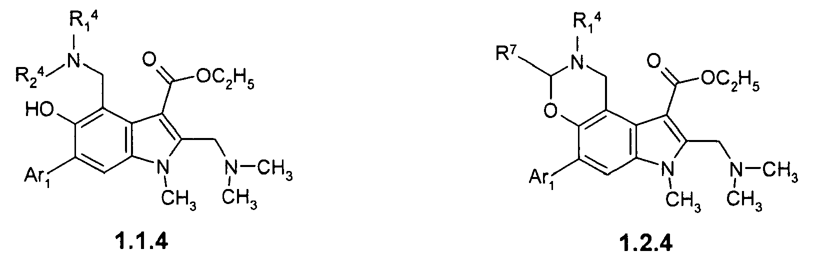 wherein: R₁⁴, R₂⁴, R⁷ and Ar₁ have the above meanings.

7. Compounds as claimed in claim 6 representing ethyl 6-aryl(or pyridyl)-2,4-bis((dimethylamino)methyl)-5-hydroxy-1-methylindole-3-carboxylates of the general formula **1.1.5** and ethyl 9-aryl(or pyridyl)-3,7-dimethyl-6-((dimethylamino)methyl)-1,2,3,7-tetrahydropyrrolo[3,2-*f*][1,3]benzoxazine-5-carboxylates of the general formula **1.2.5,** or racemates, optical isomers, pharmaceutically acceptable salts and/or hydrates thereof 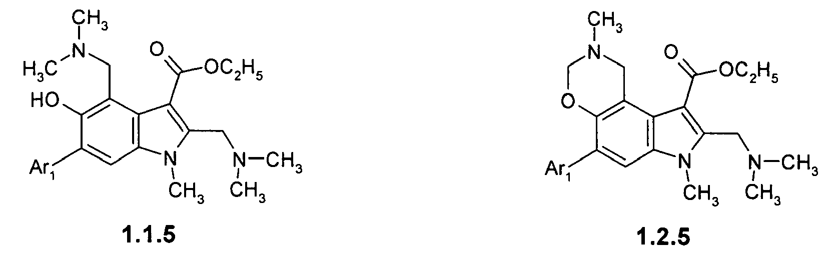 wherein Ar₁ is as defined above.

8. Compounds as claimed in any of claims 1-7 representing ethyl 2,4-bis((dimethylamino)methyl)-5-hydroxy-1-methyl-6-(pyridin-3-yl)-1H-indole-3-carboxylate of the formula **1.1.5(1)** and ethyl 3,7-dimethyl-6-((dimethylamino)methyl)-9-(pyridin-3-yl)-1,2,3,7-tetrahydropyrrolo[3,2-*f*][1,3]benzoxazine-5-carboxylate of the formula **1.2.5(1),** or racemates, optical isomers, pharmaceutically acceptable salts and/or hydrates thereof

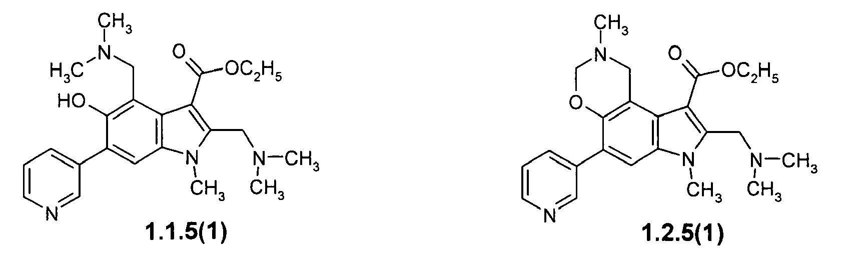

9. Compounds as claimed in any of claims 1-7 representing ethyl 2-((dimethylamino)methyl)-5-hydroxy-1-methyl-6-(pyridin-3-yl)-4-((pyrrolidin-1-yl)methyl)-indole-3-carboxylate of the formula **1.1.5(2),** ethyl 2-((dimethylamino)methyl)-5-hydroxy-4-((imidazol-1-yl)methyl)-1-methyl-6-(pyridin-3-yl)-indole-3-carboxylate of the formula **1.1.5(3),** ethyl 2-((dimethylamino)methyl)-5-hydroxy-4-(guanidylmethyl)-1-methyl-6-(pyridin-3-yl)-indole-3-carboxylate of the formula **1.1.5(4)** and ethyl 4-((dimethylamino)methyl)-5-hydroxy-1-methyl-6-(pyridin-3-yl)-2-((pyrrolidin-1-yl)methyl)-indole-3-carboxylate of the formula **1.1.5(5),** or racemates, optical isomers, pharmaceutically acceptable salts and/or hydrates thereof

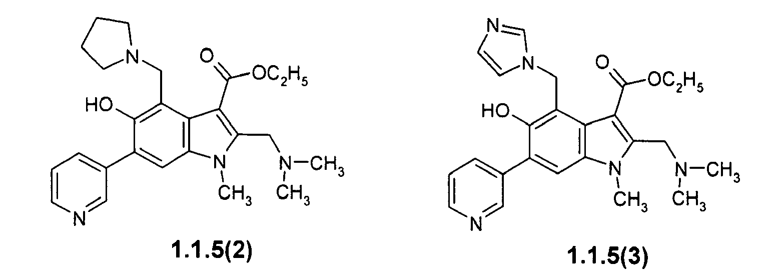

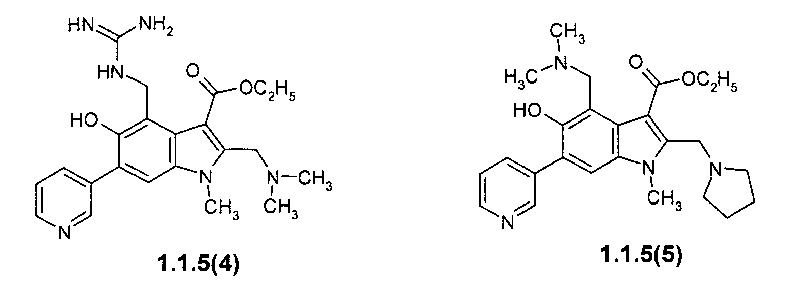

10. A method for the preparation of substituted 4-(aminomethyl)-5-hydroxy-1H-indole-3-carboxylates of the general formula **1.1** as claimed in claim 2 by interaction of substituted 5-hydroxy-1H-indole-3-carboxylates of the general formula **2** with formaldehyde or paraformaldehyde and amines of the general formula **3** 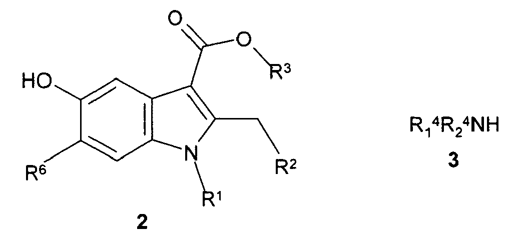 wherein: R¹, R², R³, R₁⁴, R₂⁴ and R⁶ have the above meanings.

11. A method for the preparation of 1,2,3,7-tetrahydropyrrolo[3,2-*f*][1,3]benzoxazine-5-carboxylic acid esters of the general formula **1.2** as claimed in claim 2 by interaction of 5-hydroxy-1H-indole-3-carboxylates of the general formula **2** with aldehydes of the general formula **4** and primary amines of the general formula **5** 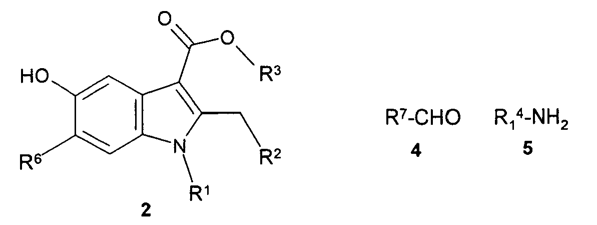 wherein: R¹, R², R³, R₁⁴, R⁶ and R⁷ have the above meanings.

12. A method for the preparation of 1,2,3,7-tetrahydropyrrolo[3,2-f][1,3]benzoxazine-5-carboxyates of the general formula **1.2** as claimed in claim 2 by interaction of 4-(aminomethyl)-5-hydroxy-1H-indole-3-carboxyates of the general formula **1.1** where R₂⁴ = H with aldehydes of the general formula **4** 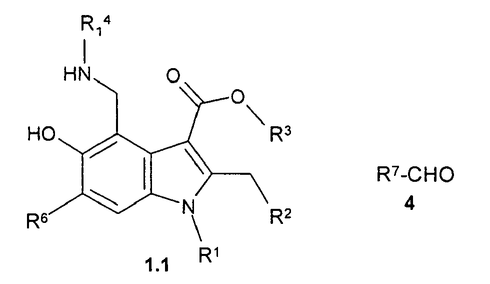 wherein: R¹, R², R³, R₁⁴, R⁶ and R⁷ have the above meanings.

13. Use of substituted indoles as claimed in any of claims 1-9 as active ingredients of pharmaceutical compositions having antiviral activity.

14. Pharmaceutical composition having antiviral activity comprising as an active ingredient at least one substituted indole as claimed in any of claims 1-9.

15. Pharmaceutical composition having antiviral activity with respect to viruses of infectious hepatitis (HCV, HBV), comprising as an active ingredient at least one substituted indole as claimed in any of claims 1-9.

16. Pharmaceutical composition having antiviral activity with respect to viruses of atypical pneumonia (SARS) and avian influenza, comprising as an active ingredient at least one substituted indole as claimed in any of claims 1-9.

17. Pharmaceutical composition having antiviral activity with respect to human immunodeficiency virus (HIV), comprising as an active ingredient at least one substituted indole as claimed in any of claims 1-9.

18. A method for the preparation of a pharmaceutical composition as claimed in any of claims 14-17 by mixing an active ingredient with an exicipient and/or a solvent, **characterized in that** as active ingredient at least one substituted indole as claimed in any of claims 1-9 is used.

19. A medicament in the form of tablets, capsules or injections placed in pharmaceutically acceptable packing for prophylaxis and treatment of virus diseases, prepared on the basis of a pharmaceutical composition as claimed in any of claims 14-17.

20. A medicament in the form of tablets, capsules or injections placed in pharmaceutically acceptable packing, for prophylaxis and treatment of virus diseases caused by viruses of infectious hepatitis (HCV, HBV), prepared on the basis of a pharmaceutical composition as claimed in any of claims 14-17.

21. A medicament in the form of tablets, capsules or injections placed in pharmaceutically acceptable packing, for prophylaxis and treatment of virus diseases induced by viruses of atypical pneumonia (SARS) and avian influenza, prepared on the basis of a pharmaceutical composition as claimed in any of claims 14-17.

22. A medicament in the form of tablets, capsules or injections placed in pharmaceutically acceptable packing, for prophylaxis and treatment of virus diseases caused by human immunodeficiency viruses (HIV), prepared on the basis of a pharmaceutical composition as claimed in any of claims 14-17.

23. A therapeutic kit for prophylaxis and treatment of virus diseases, comprising a medicament as claimed in claim 19.

24. A therapeutic kit for prophylaxis and treatment of virus diseases caused by viruses of infectious hepatitis (HCV, HBV), comprising a medicament as claimed in claim 20.

25. A therapeutic kit for prophylaxis and treatment of virus diseases caused by viruses of atypical pneumonia (SARS) and avian influenza comprising a medicament as claimed in claim 21.

26. A therapeutic kit for prophylaxis and treatment of virus diseases caused by human immunodeficiency viruses (HIV), comprising a medicament as claimed in claim 22.

27. A method for prophylaxis and treatment of virus diseases of humans and warm-blooded animals by administration of a medicament as claimed in claim 19 or a therapeutic kit as claimed in claim 23 to human or warm-blooded animal.

28. A method for prophylaxis and treatment of virus diseases of humans and warm-blooded animals caused by viruses of infectious hepatitis (HCV, HBV) by administration of a medicament as claimed in claim 20 or a therapeutic kit as claimed in claim 24 to human or warm-blooded animal.

29. A method for prophylaxis and treatment of virus diseases of humans and warm-blooded animals caused by viruses of atypical pneumonia (SARS) or avian influenza by administration of a medicament as claimed in claim 21 or a therapeutic kit as claimed in claim 25 to human or warm-blooded animal.

30. A method for prophylaxis and treatment of virus diseases of humans and warm-blooded animals caused by human immunodeficiency viruses (HIV) by administration of a medicament as claimed in claim 22 or a therapeutic kit as claimed in claim 26 to human or warm-blooded animal.
